# EUROPEAN PATENT APPLICATION

(11) **EP 4 835 080 A1**
(43) Date of publication of application: **04.11.2026**
(21) Application number: 24911359.8
(22) Date of filing: 26.12.2024
(51) Int. Cl.: C12N 15/13, C12N 15/63, C12N 15/79, C12N 15/85, C12N 15/81, C07K 16/28, C07K 16/30, C07K 16/46, A61K 39/395, A61K 47/68, A61P 35/00

(54) **MULTI-SPECIFIC ANTIBODY TARGETING HER3 AND TROP2 AND CONJUGATE THEREOF**

(30) Priority: 27.12.2023 CN 202311825615
(71) Applicant: Jiangsu Alphamab Biopharmaceuticals Co., Ltd., Jiangsu 215000 (CN)
(72) Inventor: XU, Ting, Suzhou, Jiangsu 215000 (CN); WANG, Ling, Suzhou, Jiangsu 215000 (CN); CHEN, Feier, Suzhou, Jiangsu 215000 (CN); LIU, Yang, Suzhou, Jiangsu 215000 (CN); LI, Fan, Suzhou, Jiangsu 215000 (CN); SHAN, Chuankun, Suzhou, Jiangsu 215000 (CN); DOU, Yaqi, Suzhou, Jiangsu 215000 (CN); PENG, Jianjian, Suzhou, Jiangsu 215000 (CN)
(74) Representative: Latscha Schöllhorn Partner AG
(86) International application number: PCT/CN2024/142837
(87) International publication number: WO 2025/140438

(57) **Abstract**

The present application relates to an HER3 single-domain antibody, a multispecific antigen-binding protein comprising the single-domain antibody, and a protein-drug conjugate. The present application further discloses antitumor use of the protein-drug conjugate.

## Description

### TECHNICAL FIELD

The present application relates to the field of biopharmaceuticals and particularly to a multispecific antibody targeting HER3 and TROP2 and a conjugate thereof.

### BACKGROUND

Cancer has become the second leading health threat worldwide. Cytotoxic drug-based chemotherapy has always been the primary means of cancer treatment. These cytotoxic drugs include DNA base analogs (e.g., 5-fluorouracil and 8-azaguanine), DNA interaction agents (e.g., cisplatin and actinomycin D), antimetabolites (e.g., aminopterin and methotrexate), microtubule inhibitors (e.g., paclitaxel and vincristine derivatives), etc. Most chemotherapeutic drugs have a relatively low therapeutic index, and their serious side effects primarily result from off-target toxicity. To solve this problem, scientists have been exploring cancer treatment methods with higher therapeutic windows.

The concept of "magic bullet" was first proposed by Paul Ehrlich at the beginning of the 20th century. He hypothesized that some compounds could enter target tumor cells directly. Theoretically, these compounds can effectively kill cancer cells while being harmless to normal cells. A possible approach is to find markers that can distinguish tumor cells from normal cells, for example, HER2 molecules on the surface of breast cancer cells and CD20 molecules on the surface of B-cell lymphoma. Since the advent of hybridoma technology in 1975, more and more monoclonal antibodies against these tumor antigens have been developed. Although monoclonal antibody drugs have relatively good targeting capability, their killing effects on tumor cells are far inferior to that of chemotherapy. Thus, a new concept, antibody-drug conjugates (ADCs), is proposed. In this concept, an antibody and a cytotoxic substance are bridged by a linker, with the aim of improving the therapeutic window for tumors.

Human trophoblast cell surface antigen 2 (TROP2) is a single-pass transmembrane surface glycoprotein belonging to the GA733 gene family. It is mainly expressed in epithelial cells and regulates intracellular calcium levels. There is evidence that TROP2 can affect intracellular signaling pathways and is responsible for cell growth, proliferation, and transformation. During embryonic development, the TROP2 protein plays an important role in placentation, embryo implantation, stem cell proliferation, and organ development. There is evidence that TROP2 expression is up-regulated in many tumor cells, and that the up-regulation can promote tumor growth, proliferation, and invasion. TROP2 is widely expressed in solid tumors. A study shows that TROP2 is overexpressed in 100% of squamous cell carcinoma cases (n = 72) and 93.9% of adenocarcinoma cases (n = 181). Its expression is involved in the signaling pathways of tumor proliferation, migration, invasion, and metastasis. Thus, high Trop-2 expression is a negative prognostic factor that affects the overall survival of patients with various solid tumors, including breast cancer. In addition, given that the Trop-2 expression rate in breast cancer can reach 78%, while that in HR+/HER2-breast cancer can reach up to 78.5%, and that in TNBC can even reach up to 95%, TROP2 has undoubtedly become the "hottest target" in breast cancer after HER2.

Human epidermal growth factor receptor 3 (HER3), also known as ErbB3, is a cell surface receptor tyrosine kinase belonging to the ErbB family, which includes EGFR (ErbB1), HER2/neu (ErbB2), HER3 (ErbB3), and HER4 (ErbB4). This member of the ERBB family was discovered over three decades ago in 1989; however, no HER3-targeting drug has been approved to date. HER3 overexpression is common in various types of cancer. Unlike other molecules in the HER family, HER3 lacks intracellular kinase activity, but it can phosphorylate tyrosine kinases by forming heterodimers with other receptors, such as HER2. After ligand-induced dimerized receptor formation, it can intracellularly signal through PI3K/AKT signals and MAPK/ERK signals, playing a key role in cell survival and proliferation and ultimately leading to tumor progression. Recent studies have shown that HER3 overexpression may be associated with poor prognoses in many solid tumors, including breast cancer, gastric cancer, ovarian cancer, and melanoma.

At present, neither a multispecific antibody or antigen-binding fragment that targets HER3 and TROP2 simultaneously nor an ADC drug that targets these two targets simultaneously has been found in the prior art.

### SUMMARY

In a first aspect, the present disclosure relates to a human epidermal growth factor receptor 3 (HER3)-binding protein comprising at least one immunoglobulin single variable domain, such as a VHH derived from an animal of the family Camelidae.

In a second aspect, the present disclosure relates to a multispecific antigen-binding protein comprising an antibody or antigen-binding fragment that binds to trophoblast cell surface antigen 2 (TROP2) and at least one immunoglobulin single variable domain that binds to human epidermal growth factor receptor 3 (HER3).

In a third aspect, the present disclosure further relates to an antibody-drug conjugate consisting of a multispecific antigen-binding protein that binds to HER3 and TROP2 and a cytotoxin, and an anti-tumor effect thereof.

The specific features of the invention to which the present application relates are shown in the appended claims. The characteristics and advantages of the invention to which the present application relates can be better understood with reference to the exemplary embodiments and drawings described in detail below.

### BRIEF DESCRIPTION OF THE DRAWINGS

The drawings are briefly described below:
FIG. 1 shows the affinity of iBT11 for HER3 (ELISA method), wherein □ represents iBT11-Chis, and ∘ represents iBT1-Chis.
FIG. 2 shows a comparison of the MS spectra of the conjugation product, antibody A-ADC, and the naked antibody, antibody A.
FIG. 3 shows the overall reaction scheme for preparing antibody A-ADC from antibody A.
FIG. 4 shows the killing effect of antibody A-ADC on BxPC-3 cells.
FIG. 5 shows the killing effect of antibody A-ADC on A431 cells.
FIG. 6 shows the killing effect of antibody A-ADC on CAPAN-2 cells.
FIG. 7 shows the killing effect of antibody A-ADC on HCC827 cells.
FIG. 8 shows the proliferation inhibition rates of antibody A-ADC against BxPC-3 and 293T-GFP cells.
FIG. 9 shows tumor volume change curves in an HCC827 CDX model under the action of different doses of antibody A-ADC.
FIG. 10 shows tumor volume change curves in an NCI-N87 CDX model under the action of different doses of antibody A-ADC.
FIG. 11 shows tumor volume change curves in an osimertinib-resistant human lung cancer PDX model under the action of different doses of antibody A-ADC.
FIG. 12 shows tumor volume change curves in an HCC827 CDX model under the action of antibody A-ADC and different doses of a positive control drug. The arrow marks on the horizontal axis (time) indicate the specific timing of four doses.

### DETAILED DESCRIPTION

### Definitions of Terms

Unless otherwise indicated or defined, all terms used herein have the ordinary meanings in the art that would be understood by those skilled in the art. Refer to, for example, standard handbooks, such as Sambrook et al., "Molecular Cloning: A Laboratory Manual" (2nd Edition), Vol. 1-3, Cold Spring Harbor Laboratory Press (1989); Lewin, "Genes IV", Oxford University Press, New York (1990); and Roitt et al., "Immunology" (2nd Edition), Gower Medical Publishing, London, New York (1989), and the general prior art cited herein; in addition, unless otherwise specified, all methods, steps, techniques, and procedures that are not specifically described can be, and have been, performed in a manner that itself is known to those skilled in the art. Also refer to, for example, standard handbooks, the general prior art described above, and the other references cited therein.

The terms "complete antibody", "full-length antibody", and "whole antibody" are used interchangeably herein and generally refer to an immunoglobulin molecule consisting of two identical pairs of polypeptide chains (each pair has one "light" (L) chain and one "heavy" (H) chain). Antibody light chains can be classified as κ and λ light chains. Heavy chains can be classified as µ, δ, γ, α, or ε, and the isotypes of antibodies are defined as IgM, IgD, IgG, IgA, and IgE, respectively. Within a light chain and a heavy chain, the variable regions and the constant regions can be linked by a "J" region of about 12 or more amino acids. The heavy chain can further comprise a "D" region of about 3 or more amino acids. The heavy chain may consist of a heavy chain variable region (VH) and a heavy chain constant region (CH). The heavy chain constant region may consist of 3 domains (CH1, CH2, and CH3). The light chain may consist of a light chain variable region (VL) and a light chain constant region (CL). The light chain constant region may consist of one domain: CL. The constant regions of an antibody can mediate the binding of immunoglobulins to host tissues or factors, including various cells of the immune system (e.g., effector cells) and the first component of the classical complement system (C1q). The VH and VL regions can also be subdivided into regions with high variability (known as complementarity determining regions (CDRs)) interspersed with relatively conserved regions known as framework regions (FRs). For example, the VH and VL may each comprise or consist of 3 CDRs and 4 FRs arranged from the amino-terminus to the carboxy-terminus in the following order: FR1, CDR1, FR2, CDR2, FR3, CDR3, FR4. The variable regions (VH and VL) of each heavy/light chain pair form an antibody binding site.

The term "antibody fragment" or "antigen-binding fragment" refers to a molecule different from a full-length antibody, the molecule comprising a portion of the full-length antibody and retaining the ability to specifically bind to an antigen (i.e., to bind to the same antigen as the full-length antibody from which the portion or fragment is derived). Examples of antibody fragments include, but are not limited to, Fv, Fab, Fab', Fab'-SH, and F(ab')₂; diabodies; linear antibodies; single-chain antibodies (e.g., scFvs); single-domain antibodies; bivalent antibodies or fragments thereof; and camelid antibodies (heavy-chain antibodies). The term also encompasses fragments of bispecific antibodies or multispecific antibodies, and/or bispecific antibodies or multispecific antibodies formed from antibody fragments.

"Complementarity determining regions" or "CDR regions" or "CDRs" are regions in an antibody variable domain that are highly variable in sequence and form structurally defined loops ("hypervariable loops") and/or comprise antigen contact residues ("antigen contact points"). CDRs are primarily responsible for binding to antigenic epitopes. The CDRs of heavy and light chains are generally referred to as CDR1, CDR2, and CDR3, and are sequentially numbered from the N-terminus. The CDRs located in a heavy chain variable domain of an antibody are referred to as HCDR1, HCDR2, and HCDR3, and the CDRs located in a light chain variable domain of an antibody are referred to as LCDR1, LCDR2, and LCDR3. In the amino acid sequence of a given light or heavy chain variable region, the exact amino acid sequence boundaries of the CDRs can be determined using any one or a combination of many well-known antibody CDR assignment systems, including, e.g., Chothia, which is based on the three-dimensional structures of antibodies and the topology of CDR loops (Chothia et al., (1989) Nature 342: 877-883; Al-Lazikani et al., "Standard conformations for the canonical structures of immunoglobulins", Journal of Molecular Biology, 273: 927-948 (1997)), Kabat, which is based on antibody sequence variability (Kabat et al., Sequences of Proteins of Immunological Interest, 4th Edition, U.S. Department of Health and Human Services, National Institutes of Health (1987)), AbM (University of Bath), Contact (University College London), International ImMunoGeneTics database (IMGT) (imgt.cines.fr/ on the World Wide Web), and the North CDR definition, which is based on an affinity propagation clustering using a large number of crystal structures (North et al., "A New Clustering of Antibody CDR Loop Conformations", Journal of Molecular Biology, 406, 228-256 (2011)).

In the present disclosure, the regional ranges of CDRs defined using the Kabat, AbM, or IMGT scheme are as follows:

| CDR | Kabat scheme | AbM scheme | IMGT scheme | Chothia scheme |
|---|---|---|---|---|
| LCDR1 (Kabat and Chothia numbering systems) | L24-L34 | L24-L34 | L27-L32 | L26-L32 |
| LCDR2 (Kabat and Chothia numbering systems) | L50-L56 | L50-L56 | L50-L52 | L50-L52 |
| LCDR3 (Kabat and Chothia numbering systems) | L89-L97 | L89-L97 | L89-L96 | L91-L96 |
| HCDR1 (Kabat numbering system) | H31-H35B | H26-H35B | H26-H35B | H26-H32 |
| HCDR1 (Chothia numbering system) | H31-H35 | H26-H35 | H26-H35 | H26-H32 |
| HCDR2 (Kabat and Chothia numbering systems) | H50-H65 | H50-H58 | H51-H57 | H53-H55 |
| HCDR3 (Kabat and Chothia numbering systems) | H95-H102 | H95-H102 | H93-H102 | H96-H101 |

Unless otherwise specified, the term "CDR" or "CDR sequence" as used in the present disclosure encompasses CDR sequences determined by any one of the schemes described above.

Herein, when "Kabat CDR" is referred to, it refers to a CDR determined based on the Kabat scheme. Similarly, "Chothia CDR" refers to a CDR determined based on the Chothia scheme, "Abm CDR" refers to a CDR determined based on the Abm scheme, and "IMGT CDR" refers to a CDR determined based on the IMGT scheme.

The term "antibody" is not limited by any particular antibody production method. For example, it includes, in particular, recombinant antibodies, monoclonal antibodies, and polyclonal antibodies. Antibodies may be antibodies of different isotypes, e.g., IgG (e.g., the IgG1, IgG2, IgG3, or IgG4 subtype), IgA1, IgA2, IgD, IgE, or IgM antibodies.

As used herein, the term "antigen-binding protein", "binding protein", or "binding molecule" includes molecules comprising at least one antigen-binding site, wherein the site specifically binds to a target molecule. The antigen-binding protein may be an antibody, e.g., a full-length antibody, or an antigen-binding fragment of the antibody, or a chimeric antigen receptor (CAR), or any other polypeptide having an affinity higher than usual, e.g., a scaffold protein, a cyclic peptide, a soluble receptor, a receptor-antibody (Rab) protein, or a fragment of these polypeptides. The target molecule may be any molecule. Herein, the target molecule may particularly be HER3 or TROP2, particularly a conformational epitope of HER3 or TROP2.

As used herein, the term "multispecific antigen-binding protein" is used in the broadest sense to refer to a binding protein capable of binding (preferably specifically binding) to two or more antigens. Without being bound by theory, in general, the multispecific antigen-binding protein comprises a plurality of structures that are relatively independent and provide binding specificities for the plurality of antigens, respectively; for example, each of the plurality of structures independently comprises one or more binding sites for its respective target antigen; for example, at least one of the plurality of structures is an antibody or antibody fragment. In certain aspects, at least one binding specificity is provided by an antibody. In certain aspects, at least one binding specificity is provided by a full-length antibody. In certain aspects, at least one binding specificity is provided by a VHH domain. In certain aspects, a multispecific binding protein is a bispecific antibody.

In the present application, the term "bispecific antibody" generally refers to an antibody capable of binding to two antigens or antigenic epitopes. In some embodiments of the present disclosure, the bispecific antibody may comprise a light chain and a heavy chain of an antibody capable of specifically binding to a first antigen or antigenic epitope, and a light chain and a heavy chain of an antibody capable of specifically binding to a second antigen or antigenic epitope. In some embodiments of the present disclosure, the bispecific antibody may comprise a VHH domain capable of specifically binding to a first antigen or antigenic epitope, and a light chain and a heavy chain of an antibody capable of specifically binding to a second antigen or antigenic epitope.

The term "epitope" or "antigenic epitope" generally refers to a site on an antigen to which an immunoglobulin or antibody specifically binds. "Epitope" is also referred to in the art as "antigenic determinant". Epitopes or antigenic determinants usually consist of chemically active surface groups of molecules such as amino acids or carbohydrates or sugar side chains and usually have specific three-dimensional structural characteristics and specific charge characteristics. For example, an epitope usually comprises at least 3, 4, 5, 6, 7, 8, 9, 10, 11, 12, 13, 14, or 15 contiguous or non-contiguous amino acids in a unique spatial conformation, and it may be "linear" or "conformational". See, e.g., Epitope Mapping Protocols in Methods in Molecular Biology, Vol. 66, G. E. Morris, Ed. (1996). In a linear epitope, all points of interaction between a protein and an interacting molecule (e.g., an antibody) are present linearly along the primary amino acid sequence of the protein. In a conformational epitope, the points of interaction are present across amino acid residues on the protein that are separated from one another.

The term "specificity" refers to the number of different types of antigens or epitopes to which a particular antigen-binding molecule or antigen-binding protein can bind. The specificity of an antigen-binding protein can be determined based on its affinity and/or avidity. Affinity, expressed as the dissociation equilibrium constant (KD) of an antigen and an antigen-binding protein, measures the binding strength between an epitope and an antigen-binding site on the antigen-binding protein: the smaller the KD value, the stronger the binding strength between the epitope and the antigen-binding protein (or, the affinity can also be expressed as the association constant (KA), which is 1/KD). As will be appreciated by those skilled in the art, affinity can be determined in a known manner depending on the specific antigen of interest. Avidity measures the binding strength between an antigen-binding protein (e.g., an immunoglobulin, an antibody, an immunoglobulin single variable domain, or a polypeptide comprising same) and a related antigen. Avidity is related to both the affinity between antigen-binding sites on its antigen-binding protein and the number of related binding sites present on the antigen-binding protein.

As used in the present application, the term "domain" (of a polypeptide or protein) refers to a folded protein structure that is capable of maintaining its tertiary structure independently of the rest of the protein. In general, a domain is responsible for a single functional property of a protein and, in many cases, may be added, removed, or transferred to other proteins without compromising the functionality of the rest of the protein and/or the domain.

As used in the present application, the term "immunoglobulin domain" refers to a globular region of an antibody chain (e.g., a chain of a conventional 4-chain antibody or a chain of a heavy-chain antibody) or a polypeptide consisting essentially of such globular regions. An immunoglobulin domain is characterized in that it maintains the immunoglobulin folding characteristics of an antibody molecule.

As used in the present application, the term "immunoglobulin variable domain" refers to an immunoglobulin domain consisting essentially of: four "framework regions" referred to in the art and hereinafter as "framework region 1" or "FR1", "framework region 2" or "FR2", "framework region 3" or "FR3", and "framework region 4" or "FR4", and three "complementarity determining regions" or "CDRs" referred to in the art and hereinafter as "complementarity determining region 1" or "CDR1", "complementarity determining region 2" or "CDR2", and "complementarity determining region 3" or "CDR3" that space the framework regions apart. Thus, the general structure or sequence of an immunoglobulin variable domain can be represented as follows: FR1-CDR1-FR2-CDR2-FR3-CDR3-FR4. An immunoglobulin variable domain imparts specificity for an antigen to an antibody due to the antigen-binding site it has. The variable domains of the heavy and light chains of natural antibodies generally have similar structures.

As used in the present application, the term "immunoglobulin single variable domain" refers to an immunoglobulin variable domain that is capable of specifically binding to an antigenic epitope without pairing with other immunoglobulin variable domains. A single heavy chain variable (VH) or light chain variable (VL) domain may be sufficient to provide antigen-binding specificity. An example of the immunoglobulin single variable domain in the context of the present application is a "domain antibody", such as an immunoglobulin single variable domain VH or VL (VH domain or VL domain). Another example of the immunoglobulin single variable domain is a "VHH domain" (or "VHH" for short) of the family Camelidae as defined below, sometimes also known as a "single-domain antibody" or "nanobody".

A "VHH domain", also known as a single-domain antibody, a VHH, a VHH domain, a VHH antibody fragment, and a VHH antibody, is the variable domain of an antigen-binding immunoglobulin known as a "heavy-chain antibody" (Hamers-Casterman C, Atarhouch T, Muyldermans S, Robinson G, Hamers C, Songa EB, Bendahman N, Hamers R.: "Naturally occurring antibodies devoid of light chains"; Nature 363,446-448 (1993)). The term "VHH domain" is used to distinguish the variable domain from the heavy chain variable domain (which is referred to in the present application as a "VH domain") present in a conventional 4-chain antibody. The VHH domain specifically binds to an epitope without requiring additional antigen-binding domains (as opposed to the VH or VL domain in a conventional 4-chain antibody where epitope recognition involves both the VL and VH domains). A VHH domain is a small, stable, and efficient antigen recognition unit formed from a single immunoglobulin domain. A VHH is, for example, derived from an animal of the family Camelidae, such as an alpaca, or is a humanized form thereof or a sequence-optimized form thereof (e.g., an affinity-matured form to increase the binding affinity). In some embodiments, the VHH is a monovalent monospecific polypeptide molecule consisting of, or consisting essentially of, a single heavy chain variable region (e.g., the heavy chain variable region of a heavy-chain antibody). Preferably, in one embodiment, the present disclosure provides an immunoglobulin single variable domain comprising a humanized VHH. Preferably, in one embodiment, the present disclosure provides an antibody comprising a humanized VHH. Preferably, in one embodiment, the present disclosure provides a multispecific antigen-binding protein (e.g., multispecific antibody) comprising a humanized VHH.

In the context of the present application, the terms "VHH domain", "VHH", "VHH domain", "VHH antibody fragment", and "VHH antibody" are used interchangeably.

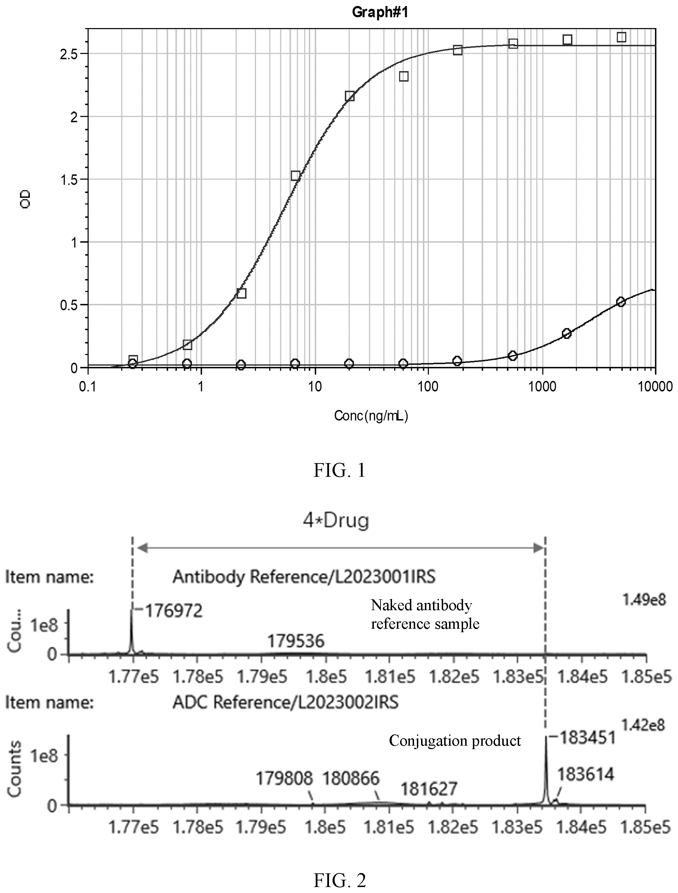

For example, as shown in Riechmann and Muyldermans, J. Immunol. Methods 231, 25-38 (1999) (see FIG. 2 therein), the amino acid residues used in VHH domains of the family Camelidae can be numbered according to the general numbering method for VH domains given by Kabat et al. (Kabat et al., Sequences of Proteins of Immunological Interest, 5th Ed. Public Health Service, National Institutes of Health, Bethesda, Md. (1991)).

Alternative methods for numbering the amino acid residues of VH domains are known in the art and can also be similarly applied to VHH domains. For example, Chothia CDRs refer to the positions of structural loops (Chothia and Lesk, J. Mol. Biol. 196: 901-917 (1987)). AbM CDRs represent a compromise between Kabat hypervariable regions and Chothia structure loops, and are used in Oxford Molecular's AbM antibody modeling software. "Contact" CDRs are based on the analysis of available complex crystal structures.

Single-domain antibodies or VHHs may also be contained in larger polypeptides/proteins. Examples of polypeptides/proteins comprising the VHH of the present disclosure include, but are not limited to, a heavy-chain antibody (HcAb).

The "heavy-chain antibody" described in the present disclosure refers to an antibody having no light chain, which may comprise, for example, from the N-segment to the C-segment, VH-Fc or VH-CH2-CH3 or VH-hinge region-CH2-CH3, or may comprise VH-CH1-CH2-CH3. The heavy-chain antibody of the present disclosure may also encompass a homodimer, e.g., a heavy-chain dimer antibody having no light chain. The heavy-chain antibody may comprise a VH from a standard antibody or a VH from a single-domain antibody. For example, the VH in the heavy-chain antibody may be a VHH. For example, the heavy-chain antibody may be a heavy-chain antibody having framework regions and/or a heavy chain constant region derived from animals of the family Camelidae (llamas and camels, particularly alpacas), a humanized form thereof or a sequence-optimized form thereof (affinity-matured form), or a fragment thereof (e.g., a fragment comprising at least a portion of the constant region). The heavy-chain antibody may also encompass an antibody formed by fusing a heavy chain variable region or a VHH to an Fc region (e.g., a human IgG Fc region, e.g., a human IgG1 or IgG4 Fc region).

The term "Fc region" is used herein to define a C-terminal region of an immunoglobulin heavy chain that comprises at least a portion of the constant region. The term includes Fc regions of natural sequences and variant Fc regions. A natural immunoglobulin "Fc domain" comprises two or three constant domains: a CH2 domain, a CH3 domain, and an optional CH4 domain. For example, in natural antibodies, an immunoglobulin Fc domain comprises the second and the third constant domains (the CH2 domain and the CH3 domain) derived from two heavy chains of IgG, IgA, and IgD antibodies, or comprises the second, the third, and the fourth constant domains (the CH2 domain, the CH3 domain, and the CH4 domain) derived from two heavy chains of IgM and IgE antibodies. Unless otherwise specified herein, the amino acid residues in an Fc region or heavy chain constant region are numbered according to the EU numbering system (also known as the EU index) described in, for example, Kabat et al., Sequences of Proteins of Immunological Interes, 5th Edition, Public Health Service, National Institutes of Health, Bethesda, MD, 1991. Herein, the term "Fc region" does not comprise the heavy chain variable region VH and the light chain variable region VL, as well as the heavy chain constant region CH1 and the light chain constant region CL, of an immunoglobulin, but in some cases, it may comprise the hinge region at the N-terminus of the heavy chain constant region. In some embodiments, the Fc region of the present disclosure is from IgG1, IgG2, IgG3, or IgG4. In some embodiments, the Fc region of the present disclosure comprises the amino acid sequence set forth in SEQ ID NO: 2 or 3, or an amino acid sequence having at least 85%, 90%, 91%, 92%, 93%, 94%, 95%, 96%, 97%, 98%, or 99% identity to the amino acid sequence.

The term "protein-drug conjugate" generally refers to a binding protein (e.g., an antibody or an antigen-binding fragment thereof) linked to one or more chemical drugs, such as an antibody-drug conjugate (ADC). The chemical drugs may be any therapeutic agents and/or cytotoxic agents. The antibody-drug conjugate may have anywhere from 1 to 16 drugs conjugated to the antibody; for example, it may include drug-loaded species of 2, 4, 6, or 8. In the present disclosure, the drugs may include mitotic inhibitors, anti-tumor antibiotics, immunomodulators, vectors for gene therapy, alkylating agents, anti-angiogenic agents, anti-metabolites, boron-containing agents, chemoprotective agents, hormones, anti-hormone agents, corticosteroids, photoactive therapeutic agents, oligonucleotides, radionuclide agents, topoisomerase inhibitors, tyrosine kinase inhibitors, and/or radiosensitizers, etc.

In the present application, the term "drug/antibody ratio" or "DAR" generally refers to the number of drugs linked to the antibody (or protein) of the ADC. The DAR of an ADC may range from 1 to 8, or may also be higher (e.g., 10). The range of the DAR can depend on the number of linking sites on the antibody. In the present application, the DAR may be the number of drugs loaded onto an individual antibody. The DAR may also be an average or mean DAR of a group of ADCs.

The term "linker" refers to a structural fragment that links a drug (e.g., a small-molecule drug) and an antibody moiety. It will be appreciated that the linker has a functional group that can form a bond with a functional group of an antibody or an antigen-binding fragment thereof before being linked to the antibody or the antigen-binding fragment thereof.

In the present application, the term "HER3" generally refers to human epidermal growth factor receptor 3 (SwissProt P21860). Human HER3, also known as ErbB-3, ERBB3, c-erbB-3, c-erbB3, or receptor tyrosine protein kinase erbB-3, is a member of the epidermal growth factor receptor (EGFR) family of receptor tyrosine kinases. This family also includes HER1 (also known as EGFR), HER2, and HER4. HER3 is a transmembrane receptor similar to EGFR, consisting of an extracellular ligand-binding domain (ECD), a dimerization domain within the ECD, a transmembrane domain, an intracellular protein tyrosine kinase domain (TKD), and a C-terminal phosphorylation domain. HER3 is capable of binding to its ligands. Although it cannot directly transmit signals into cells via protein phosphorylation, it can indirectly activate signaling pathways by forming heterodimers with other HER family members with kinase activity. Dimer formation between HER family members expands the signaling potential of HER3 and is not only a means of signal diversification but also a means of signal amplification. The HER3 may include any variants, truncated forms, fragments, isoforms, and species homologs of HER3 that are naturally expressed by cells, including tumor cells, or are expressed by cells transfected with the HER3 gene or cDNA.

As used herein, the term "alkyl" refers to a saturated aliphatic hydrocarbon group that is a linear or branched group containing 1 to 20 carbon atoms, preferably an alkyl group containing 1 to 12 carbon atoms, more preferably an alkyl group containing 1 to 10 carbon atoms, and most preferably an alkyl group containing 1 to 6 carbon atoms.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, and more preferably 3 to 10 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl groups include spiro-ring, fused-ring, and bridged-ring cycloalkyl groups.

The term "cycloalkylene" refers to a saturated or partially unsaturated monocyclic or polycyclic hydrocarbon group, which is a residue derived by removing two hydrogen atoms from the same carbon atom or two different carbon atoms of the parental alkane.

The term "heterocycloalkyl" refers to a saturated or partially unsaturated monocyclic or polycyclic hydrocarbon substituent containing 3 to 20 ring atoms, wherein one or more of the ring atoms are heteroatoms selected from nitrogen, oxygen, and S(O)ₘ (where m is an integer from 0 to 2), but a ring moiety of -O-O-, -O-S-, or -S-S- is excluded, and the other ring atoms are carbon atoms.

The term "heterocycloalkylene" refers to a saturated or partially unsaturated monocyclic or polycyclic hydrocarbon group containing 3 to 20 ring atoms, wherein one or more of the ring atoms are heteroatoms selected from nitrogen, oxygen, and S(O)ₘ (where m is an integer from 0 to 2), but a ring moiety of -O-O-, -O-S-, or -S-S- is excluded, and the other ring atoms are carbon atoms. The group is a residue derived by removing two hydrogen atoms from the same carbon atom or two different carbon atoms of the parental alkane on this basis.

The term "alkoxy" refers to -O-(alkyl) and -O-(unsubstituted cycloalkyl), wherein the alkyl or cycloalkyl is defined as described above.

The term "aryl" refers to a 6- to 14-membered, preferably 6- to 10-membered, all-carbon monocyclic or fused polycyclic (i.e., rings that share a pair of adjacent carbon atoms) group having a conjugated π-electron system, such as phenyl and naphthyl, preferably phenyl. The aryl ring may be fused to a heteroaryl, heterocyclyl, or cycloalkyl ring, wherein the ring attached to the parental structure is the aryl ring. Aryl may be substituted or unsubstituted, and when it is substituted, the substituent is preferably one or more groups independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "arylene" refers to a 6- to 14-membered all-carbon monocyclic or fused polycyclic ring having a conjugated π-electron system, which is a residue derived by removing two hydrogen atoms from two different carbon atoms of the parental aromatic ring.

The term "heteroaryl" refers to a heteroaromatic system containing 1 to 4 heteroatoms and 5 to 14 ring atoms, wherein the heteroatoms are selected from oxygen, sulfur, and nitrogen. Heteroaryl is preferably 5- to 10-membered, and is more preferably 5-membered or 6-membered, such as furanyl, thienyl, pyridinyl, pyrrolyl, N-alkylpyrrolyl, pyrimidinyl, pyrazinyl, imidazolyl, and tetrazolyl. The heteroaryl ring may be fused to an aryl, heterocyclyl, or cycloalkyl ring, wherein the ring attached to the parental structure is the heteroaryl ring. Heteroaryl may be optionally substituted or unsubstituted, and when it is substituted, the substituent is preferably one or more groups independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heteroarylene" refers to a heteroaromatic polycyclic ring containing 1 to 4 heteroatoms and 5 to 14 ring atoms, which is a residue derived by removing two hydrogen atoms from two different carbon atoms of the parental aromatic ring.

The term "optional" or "optionally" means that the subsequently described event or circumstance occurs or does not occur, and the description includes instances where the event or circumstance occurs and instances where it does not. For example, when a group or structure is "optionally substituted", the group or structure may be substituted or unsubstituted.

The term "pharmaceutically acceptable salt" refers to salts that retain the biological effects and properties of the conjugates of the present disclosure and are not biologically or otherwise undesired. The conjugates of the present disclosure may be present in pharmaceutically acceptable salt forms thereof, including acid addition salts and base addition salts. In the present disclosure, pharmaceutically acceptable non-toxic acid addition salts refer to salts formed from the conjugates of the present disclosure with organic or inorganic acids, wherein the organic or inorganic acids include, but are not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propanoic acid, benzoic acid, p-toluenesulfonic acid, malic acid, etc. Pharmaceutically acceptable non-toxic base addition salts refer to salts formed from the conjugates of the present disclosure with organic or inorganic bases, including but not limited to alkali metal salts, such as lithium, sodium, or potassium salts; alkaline earth metal salts, such as calcium or magnesium salts; and organic base salts, such as ammonium salts formed with N group-containing organic bases.

Where there is no contradiction according to the context, "pharmaceutically acceptable" and "pharmaceutical" are used interchangeably herein.

As used herein, the term "and/or" refers to any one of the options or two or more of the options.

As used herein, the term "comprise", "contain", or "include" refers to including the stated elements, integers, or steps, but not excluding any other elements, integers, or steps. As used herein, the term "comprise", "contain", or "include" also encompasses combinations of the stated elements, integers, or steps, unless otherwise indicated.

The term "administer" generally refers to a method of giving a subject (e.g., a patient) a certain dose of a compound or pharmaceutical composition. Administration may be performed by any suitable means, including parenteral, intrapulmonary, and intranasal, as well as (if topical treatment is desired) intralesional administration. Parenteral infusion includes, for example, intramuscular, intravenous, intraarterial, intraperitoneal, or subcutaneous administration.

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value. Unless otherwise specified, all numerical values mentioned in the present application are seen to be modified by "about". If there is a question or there is no common understanding for the margin of error of a particular value or parameter acknowledged in the art, "about" means +5% of that value or parameter.

The term "effective amount" refers to an amount or dose of the antibody or fragment or composition or combination of the present disclosure that produces the expected effects in a patient in need of treatment or prevention after being administered to the patient in a single dose or multiple doses. "Therapeutically effective amount" and "prophylactically effective amount" may be included according to the expected effects.

"Therapeutically effective amount" refers to an amount effective, at dosages and for periods of time necessary, to achieve the desired therapeutic result. A therapeutically effective amount is also one in which any toxic or detrimental effect of the antibody or antibody fragment or composition or combination is outweighed by the therapeutically beneficial effects. "Prophylactically effective amount" refers to an amount effective, at dosages and for periods of time necessary, to achieve the desired prophylactic result. In general, as a prophylactic dose is used in a subject prior to or at an earlier stage of a disease, the prophylactically effective amount will be less than the therapeutically effective amount.

"Individual" or "subject" includes mammals. Mammals include, but are not limited to, domestic animals (e.g., cows, sheep, cats, dogs, and horses), primates (e.g., human and non-human primates such as monkeys), rabbits, and rodents (e.g., mice and rats). In some embodiments, the individual or subject is a human.

The terms "cancer" and "cancerous" refer to or describe physiological conditions in mammals that are typically characterized by unregulated cell growth. Cancer may be at an early, intermediate, or advanced stage or be metastatic cancer.

The term "tumor" refers to all neoplastic cell growth and proliferation, whether malignant or benign, and all precancerous and cancerous cells and tissues. "Tumor" encompasses solid tumors and hematological tumors, as well as metastatic lesions. The terms "cancer", "cancerous", and "tumor" are not mutually exclusive when referred to herein.

The term "pharmaceutical auxiliary material" refers to diluents, adjuvants (e.g., Freund's adjuvants (complete and incomplete)), excipients, carriers, stabilizers, or the like, which are administered together with an active substance.

The term "pharmaceutical composition" refers to a composition that is present in a form that allows the biological activity of an active ingredient contained therein to be effective and does not contain additional ingredients that have unacceptable toxicity to a subject to which the composition is administered.

As used herein, "treat" refers to slowing, interrupting, arresting, alleviating, stopping, reducing, or reversing the progression or severity of an existing symptom, condition, disorder, or disease.

### Detailed Description of the Invention

### Human Epidermal Growth Factor Receptor 3 (HER3)-Binding Protein

In one aspect, the present application provides a single-domain antibody against human epidermal growth factor receptor 3 (HER3), comprising a CDR1, a CDR2, and a CDR3 from the VHH set forth in SEQ ID NO: 1.

In some embodiments, the CDR1, the CDR2, and the CDR3 are defined according to the following scheme: Kabat, AbM, Chothia, or IMGT.

In some embodiments, the single-domain antibody is of the family Camelidae, humanized, or chimeric.

In some embodiments, the CDR1, the CDR2, and the CDR3 from the VHH set forth in SEQ ID NO: 1 are selected from any one of the following groups: SEQ ID NOs: 8-10 (Kabat scheme), SEQ ID NOs: 11-13 (AbM scheme), SEQ ID NOs: 14-16 (Chothia scheme), and SEQ ID NOs: 17-19 (IMGT scheme).

In some embodiments, the single-domain antibody comprises or consists of: (1) the amino acid sequence set forth in SEQ ID NO: 1; (2) an amino acid sequence having at least 85%, 90%, 91%, 92%, 93%, 94%, 95%, 96%, 97%, 98%, or 99% identity to SEQ ID NO: 1; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 1.

In some embodiments, the single-domain antibody is humanized.

In some embodiments, the single-domain antibody comprises the amino acid sequence set forth in any one of SEQ ID NOs: 2-7.

In some embodiments, the single-domain antibody consists of the amino acid sequence set forth in any one of SEQ ID NOs: 2-7.

In another aspect, the present application provides a human epidermal growth factor receptor 3 (HER3)-binding protein comprising at least one immunoglobulin single variable domain, wherein the immunoglobulin single variable domain comprises a CDR1, a CDR2, and a CDR3 from the VHH set forth in SEQ ID NO: 1.

The "at least one immunoglobulin single variable domain" may be one or more immunoglobulin single variable domains. Unless otherwise specified, the amino acid sequences of the immunoglobulin single variable domains may be identical or different. In some embodiments, "more" may be 2, 3, 4, or 5, or more.

In some embodiments, the CDR1, the CDR2, and the CDR3 are defined according to the following scheme: Kabat, AbM, Chothia, or IMGT.

In some embodiments, the immunoglobulin single variable domain is of the family Camelidae, humanized, or chimeric.

In some embodiments, the CDR1, the CDR2, and the CDR3 from the VHH set forth in SEQ ID NO: 1 are selected from any one of the following groups: SEQ ID NOs: 8-10 (Kabat scheme), SEQ ID NOs: 11-13 (AbM scheme), SEQ ID NOs: 14-16 (Chothia scheme), and SEQ ID NOs: 17-19 (IMGT scheme).

In some embodiments, the at least one immunoglobulin single variable domain comprises or consists of one or more VHHs, wherein at least one VHH is set forth in SEQ ID NO: 1.

In some embodiments, the at least one immunoglobulin single variable domain comprises one or more VHHs represented by: (1) the amino acid sequence set forth in SEQ ID NO: 1; (2) an amino acid sequence having at least 85%, 90%, 91%, 92%, 93%, 94%, 95%, 96%, 97%, 98%, or 99% identity to SEQ ID NO: 1; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 1.

In some embodiments, the at least one immunoglobulin single variable domain consists of one or more VHHs represented by: (1) the amino acid sequence set forth in SEQ ID NO: 1; (2) an amino acid sequence having at least 85%, 90%, 91%, 92%, 93%, 94%, 95%, 96%, 97%, 98%, or 99% identity to SEQ ID NO: 1; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 1.

In some embodiments, the immunoglobulin single variable domain is a humanized VHH, and the humanized VHH comprises an amino acid sequence having at least 80%, preferably at least 90%, more preferably at least 95%, and even more preferably at least 99% sequence identity to SEQ ID NO: 1. In some embodiments, the amino acid sequence of the humanized VHH comprises one or more amino acid substitutions, preferably conservative amino acid substitutions, compared to SEQ ID NO: 1. For example, the amino acid sequence of the humanized immunoglobulin single variable domain comprises 1, 2, 3, 4, 5, 6, 7, 8, 9, or 10 conservative amino acid substitutions compared to SEQ ID NO: 1.

In some embodiments, the at least one immunoglobulin single variable domain may comprise one or more aforesaid humanized VHHs.

In some embodiments, the at least one immunoglobulin single variable domain comprises one or more of the amino acid sequences set forth in SEQ ID NOs: 2-7.

In some embodiments, the at least one immunoglobulin single variable domain comprises one or more of the amino acid sequences set forth in SEQ ID NOs: 1-7.

In some embodiments, the at least one immunoglobulin single variable domain consists of one or more amino acid sequences set forth in SEQ ID NOs: 1-7.

In some other embodiments, the at least one immunoglobulin single variable domain comprises an amino acid sequence selected from the amino acid sequences set forth in SEQ ID NOs: 1-7. For example, the at least one immunoglobulin single variable domain comprises one or more VHHs represented by: (1) the amino acid sequence set forth in SEQ ID NO: 1; (2) an amino acid sequence having at least 85%, 90%, 91%, 92%, 93%, 94%, 95%, 96%, 97%, 98%, or 99% identity to SEQ ID NO: 1; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 1; the at least one immunoglobulin single variable domain comprises one or more VHHs represented by: (1) the amino acid sequence set forth in SEQ ID NO: 2; (2) an amino acid sequence having at least 85%, 90%, 91%, 92%, 93%, 94%, 95%, 96%, 97%, 98%, or 99% identity to SEQ ID NO: 2; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 2; the at least one immunoglobulin single variable domain comprises one or more VHHs represented by: (1) the amino acid sequence set forth in SEQ ID NO: 3; (2) an amino acid sequence having at least 85%, 90%, 91%, 92%, 93%, 94%, 95%, 96%, 97%, 98%, or 99% identity to SEQ ID NO: 3; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 3; the at least one immunoglobulin single variable domain comprises one or more VHHs represented by: (1) the amino acid sequence set forth in SEQ ID NO: 4; (2) an amino acid sequence having at least 85%, 90%, 91%, 92%, 93%, 94%, 95%, 96%, 97%, 98%, or 99% identity to SEQ ID NO: 4; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 4; the at least one immunoglobulin single variable domain comprises one or more VHHs represented by: (1) the amino acid sequence set forth in SEQ ID NO: 5; (2) an amino acid sequence having at least 85%, 90%, 91%, 92%, 93%, 94%, 95%, 96%, 97%, 98%, or 99% identity to SEQ ID NO: 5; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 5; the at least one immunoglobulin single variable domain comprises one or more VHHs represented by: (1) the amino acid sequence set forth in SEQ ID NO: 6; (2) an amino acid sequence having at least 85%, 90%, 91%, 92%, 93%, 94%, 95%, 96%, 97%, 98%, or 99% identity to SEQ ID NO: 6; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 6; or the at least one immunoglobulin single variable domain comprises one or more (1) VHHs set forth in SEQ ID NO: 7; (2) amino acid sequences having at least 85%, 90%, 91%, 92%, 93%, 94%, 95%, 96%, 97%, 98%, or 99% identity to SEQ ID NO: 7; or (3) amino acid sequences comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 7.

For example, in some embodiments, the at least one immunoglobulin single variable domain consists of one or more VHHs set forth in SEQ ID NO: 1; the at least one immunoglobulin single variable domain consists of one or more VHHs set forth in SEQ ID NO: 2; the at least one immunoglobulin single variable domain consists of one or more VHHs set forth in SEQ ID NO: 3; the at least one immunoglobulin single variable domain consists of one or more VHHs set forth in SEQ ID NO: 4; the at least one immunoglobulin single variable domain consists of one or more VHHs set forth in SEQ ID NO: 5; the at least one immunoglobulin single variable domain consists of one or more VHHs set forth in SEQ ID NO: 6; or the at least one immunoglobulin single variable domain consists of one or more VHHs set forth in SEQ ID NO: 7.

In some embodiments, the HER3-binding protein comprises one aforesaid immunoglobulin single variable domain.

In some embodiments, the HER3-binding protein comprises 2, 3, 4, or 5 or more aforesaid immunoglobulin single variable domains.

In some embodiments, the HER3-binding protein of the present application comprises an immunoglobulin Fc region in addition to the at least one immunoglobulin single variable domain. The inclusion of the immunoglobulin Fc region in the HER3-binding protein of the present disclosure may cause the binding molecule to form a dimer. Fc regions that can be used in the present disclosure may be from different subtypes of immunoglobulins, for example, IgG (e.g., the IgG1, IgG2, IgG3, or IgG4 subtype), IgA1, IgA2, IgD, IgE, or IgM.

In some embodiments, a mutation may be introduced into a wild-type Fc sequence to alter an Fc-mediated related activity. The mutation includes, but is not limited to: a) a mutation that alters Fc-mediated CDC activity; b) a mutation that alters Fc-mediated ADCC activity; or c) a mutation that alters FcRn-mediated *in vivo* half-life. Such mutations are described in Leonard G Presta, Current Opinion in Immunology 2008, 20: 460-470; Esohe E. Idusogie et al., J Immunol 2000, 164: 4178-4184; RAPHAEL A. CLYNES et al., Nature Medicine, 2000, Volume 6, Number 4: 443-446; and Paul R. Hinton et al., J Immunol, 2006, 176: 346-356. For example, 1, 2, 3, 4, 5, 6, 7, 8, 9, or 10 amino acids of the CH2 region may be mutated to increase or remove Fc-mediated ADCC or CDC activity or to enhance or reduce FcRn affinity. In addition, 1, 2, 3, 4, 5, 6, 7, 8, 9, or 10 amino acids of the hinge region may be mutated to increase the stability of the protein.

The immunoglobulin Fc region is preferably a human immunoglobulin Fc region, for example, a human IgG1, IgG2, IgG3, or IgG4 Fc region. In some specific embodiments, the amino acid sequence of the immunoglobulin Fc region is set forth in SEQ ID NO: 20.

In some specific embodiments, in the HER3-binding protein of the present application, the immunoglobulin Fc region (e.g., human IgG1 Fc region) is linked directly or linked indirectly by a linker to the C-terminus of the immunoglobulin single variable domain (e.g., VHH).

In some embodiments, the HER3-binding protein of the present application has at least one of the following characteristics:
(a) having specific binding to human HER3;
(b) binding to human HER3 with a K_{D} value of less than 1 × 10⁻⁷ M, preferably less than 1 × 10⁻⁸ M;
(c) specifically binding to human HER3 with an EC₅₀ value of less than 200 ng/mL, preferably less than 100 ng/mL, and more preferably less than 50 ng/mL;
(d) being capable of blocking the formation of a heterodimer from HER3 and HER2;
(e) being capable of blocking the binding of HER3 to a ligand therefor (e.g., NRG1 or NRG1b1), e.g., blocking the binding of HER3 to the ligand therefor with an IC₅₀ value of no greater than 200 ng/mL, preferably no greater than 150 ng/mL, and more preferably no greater than 120 ng/mL; and
(f) being capable of inhibiting the proliferation of tumor cells.

### Fusion Protein

In another aspect, the present application further relates to a fusion protein comprising the HER3-binding protein or the single-domain antibody against HER3 described in the present disclosure.

In some specific embodiments, the fusion protein is a multispecific antigen-binding protein, e.g., a bispecific antigen-binding protein, e.g., a bispecific antibody. In some more specific embodiments, the multispecific antigen-binding protein further comprises a second binding specificity and optionally more binding specificities.

In some embodiments, the fusion protein comprises, in addition to the HER3-binding protein or the single-domain antibody against HER3, one or more additional biologically active proteins that may be any proteins having biological, therapeutic, prophylactic, or diagnostic significance or function and, when administered to a subject, mediate biological activity that can prevent or alleviate a disease, disorder, or condition. Specifically, the biologically active proteins may be agonists, antagonists, modulators, ligands, cytokines, enzymes, or hormones, particularly biologically active proteins that can be used for preventing and/or treating tumors, especially solid tumors and/or non-solid tumors.

### Multispecific Antigen-Binding Protein

In another aspect, the present application further relates to a multispecific antigen-binding protein comprising a structural moiety that specifically binds to HER3 (HER3-binding region), e.g., an HER3-binding protein, preferably the HER3-binding protein described in the present application.

In some embodiments, the multispecific antigen-binding protein comprises, in addition to the HER3-binding protein, one or more additional antigen-binding regions that provide different antigen-binding specificities, i.e., binding to different antigens or different epitopes of the same antigen compared to the HER3-binding protein.

In some embodiments, the multispecific antigen-binding protein comprises a trophoblast cell surface antigen 2 (TROP2)-binding region and a human epidermal growth factor receptor 3 (HER3)-binding region. In some more specific embodiments, the trophoblast cell surface antigen 2 (TROP2)-binding region and the human epidermal growth factor receptor 3 (HER3)-binding region are both antibodies or antibody fragments (e.g., immunoglobulin single variable domains); that is, the binding specificities to TROP2 and HER3 are both provided by antibodies or antibody fragments; that is, the multispecific antigen-binding protein is an anti-TROP2×HER3 bispecific antibody.

In some embodiments, the multispecific antigen-binding protein comprises a trophoblast cell surface antigen 2 (TROP2)-binding protein, e.g., an antibody or an antigen-binding fragment thereof that binds to TROP2, and the aforementioned at least one immunoglobulin single variable domain that binds to human epidermal growth factor receptor 3 (HER3);
specifically, the antibody or the antigen-binding fragment thereof that binds to TROP2 comprises a heavy chain variable region and a light chain variable region, wherein the heavy chain variable region comprises a VH CDR1, a VH CDR2, and a VH CDR3 selected from any one of the following groups: SEQ ID NOs: 22-24 (Kabat scheme), SEQ ID NOs: 25-27 (AbM scheme), SEQ ID NOs: 28-30 (Chothia scheme), and SEQ ID NOs: 31-33 (IMGT scheme), and
the light chain variable region comprises a VL CDR1, a VL CDR2, and a VL CDR3 selected from any one of the following groups: SEQ ID NOs: 35-37 (Kabat scheme), SEQ ID NOs: 38-40 (AbM scheme), SEQ ID NOs: 41-43 (Chothia scheme), and SEQ ID NOs: 44-46 (IMGT scheme).

In some embodiments, in the multispecific antigen-binding protein, the immunoglobulin single variable domain that binds to HER3 comprises a VHH CDR1, a VHH CDR2, and a VHH CDR3 selected from any one of the following groups: SEQ ID NOs: 8-10 (Kabat scheme), SEQ ID NOs: 11-13 (AbM scheme), SEQ ID NOs: 14-16 (Chothia scheme), and SEQ ID NOs: 17-19 (IMGT scheme).

In some embodiments, the heavy chain variable region of the antibody or the antigen-binding fragment thereof that binds to TROP2 comprises or consists of an amino acid sequence represented by: (1) the amino acid sequence set forth in SEQ ID NO: 21; (2) an amino acid sequence having at least 85%, 90%, 91%, 92%, 93%, 94%, 95%, 96%, 97%, 98%, or 99% identity to SEQ ID NO: 21; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 21. In some embodiments, the heavy chain variable region of the antibody or the antigen-binding fragment thereof that binds to TROP2 comprises or consists of the amino acid sequence set forth in SEQ ID NO: 21.

In some embodiments, the light chain variable region of the antibody or the antigen-binding fragment thereof that binds to TROP2 comprises or consists of an amino acid sequence represented by: (1) the amino acid sequence set forth in SEQ ID NO: 34; (2) an amino acid sequence having at least 85%, 90%, 91%, 92%, 93%, 94%, 95%, 96%, 97%, 98%, or 99% identity to SEQ ID NO: 34; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 34. In some embodiments, the light chain variable region of the antibody or the antigen-binding fragment thereof that binds to TROP2 comprises or consists of the amino acid sequence set forth in SEQ ID NO: 34.

In some embodiments, the antibody or the antigen-binding fragment thereof that binds to TROP2 comprises a heavy chain that further comprises a human IgG1 constant region or a variant thereof; for example, the heavy chain comprises an amino acid sequence represented by: (1) the amino acid sequence set forth in SEQ ID NO: 47; (2) an amino acid sequence having at least 85%, 90%, 91%, 92%, 93%, 94%, 95%, 96%, 97%, 98%, or 99% identity to SEQ ID NO: 47; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 47.

In some embodiments, the antibody or the antigen-binding fragment thereof that binds to TROP2 comprises a light chain that further comprises a human Igκ constant region or a variant thereof; for example, the light chain comprises an amino acid sequence represented by: (1) the amino acid sequence set forth in SEQ ID NO: 48; (2) an amino acid sequence having at least 85%, 90%, 91%, 92%, 93%, 94%, 95%, 96%, 97%, 98%, or 99% identity to SEQ ID NO: 48; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 48.

In some specific embodiments, the antibody that binds to TROP2 comprises a heavy chain that comprises an amino acid sequence represented by: (1) the amino acid sequence set forth in SEQ ID NO: 49; (2) an amino acid sequence having at least 85%, 90%, 91%, 92%, 93%, 94%, 95%, 96%, 97%, 98%, or 99% identity to SEQ ID NO: 49; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 49. In some specific embodiments, the antibody that binds to TROP2 comprises a heavy chain that comprises the amino acid sequence set forth in SEQ ID NO: 49.

In some specific embodiments, the antibody that binds to TROP2 comprises a light chain that comprises an amino acid sequence represented by: (1) the amino acid sequence set forth in SEQ ID NO: 50; (2) an amino acid sequence having at least 85%, 90%, 91%, 92%, 93%, 94%, 95%, 96%, 97%, 98%, or 99% identity to SEQ ID NO: 50; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 50. In some specific embodiments, the antibody that binds to TROP2 comprises a light chain that comprises the amino acid sequence set forth in SEQ ID NO: 50.

As an example, the antibody that binds to TROP2 may have the same structure as sacituzumab (hRS7), which is published in WHO Drug Information Proposed INN List 115 (2016).

In some embodiments, in the multispecific antigen-binding protein, the at least one immunoglobulin single variable domain that binds to HER3 comprises an amino acid sequence represented by: (1) the amino acid sequence set forth in one of SEQ ID NOs: 1-7; (2) an amino acid sequence having at least 85%, 90%, 91%, 92%, 93%, 94%, 95%, 96%, 97%, 98%, or 99% identity to one of SEQ ID NOs: 1-7; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to one of SEQ ID NOs: 1-7.

In some embodiments, the at least one immunoglobulin single variable domain that binds to HER3 comprises one or more amino acid sequences represented by: (1) the amino acid sequence set forth in SEQ ID NO: 1; (2) an amino acid sequence having at least 85%, 90%, 91%, 92%, 93%, 94%, 95%, 96%, 97%, 98%, or 99% identity to SEQ ID NO: 1; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 1. In some embodiments, the at least one immunoglobulin single variable domain that binds to HER3 comprises one or more amino acid sequences represented by: (1) the amino acid sequence set forth in SEQ ID NO: 7; (2) an amino acid sequence having at least 85%, 90%, 91%, 92%, 93%, 94%, 95%, 96%, 97%, 98%, or 99% identity to SEQ ID NO: 7; or (3) an amino acid sequence comprising one or more amino acid substitutions, preferably conservative amino acid substitutions, e.g., 1, 2, 3, 4, 5, 6, 7, 8, 9, or 10 conservative amino acid substitutions, compared to SEQ ID NO: 7.

In some embodiments, the multispecific antigen-binding protein comprises one aforesaid immunoglobulin single variable domain; in some other embodiments, the multispecific antigen-binding protein comprises two aforesaid immunoglobulin single variable domains.

In some embodiments, in the multispecific antigen-binding protein, the at least one immunoglobulin single variable domain that binds to HER3 is linked directly or linked indirectly by a linker to the antibody or the antigen-binding fragment that binds to TROP2.

In some embodiments, the linker is a polypeptide; for example, the linker may be a non-functional amino acid sequence that is 1-20 or more amino acids in length and has no secondary or higher-order structure. For example, the linker is a flexible linker, e.g., one or more of (GGG)n, (GGGS)n, (GGGA)n, (GGGAA)n, (GGGGS)n, and (GGGGGS)n, wherein n is an integer selected from 1 to 30, an integer selected from 1 to 20, an integer selected from 1 to 10, an integer selected from 1 to 9, an integer selected from 1 to 8, an integer selected from 1 to 7, an integer selected from 1 to 6, an integer selected from 1 to 5, an integer selected from 1 to 4, or an integer selected from 1 to 3. In some embodiments, the linker is GGGGGS, GGGGS, GS, GAP, (GGGGS)×3, GAPGGGGGS, or the like.

In some embodiments, the C-terminus of the at least one immunoglobulin single variable domain that binds to HER3 is linked to the N-terminus of a heavy chain of the antibody or the antigen-binding fragment that binds to TROP2.

In some embodiments, the N-terminus of the at least one immunoglobulin single variable domain that binds to HER3 is linked to the C-terminus of a heavy chain of the antibody or the antigen-binding fragment that binds to TROP2.

In some embodiments, the C-terminus of the at least one immunoglobulin single variable domain that binds to HER3 is linked to the N-terminus of a light chain of the antibody or the antigen-binding fragment that binds to TROP2.

In some embodiments, the N-terminus of the at least one immunoglobulin single variable domain that binds to HER3 is linked to the C-terminus of a light chain of the antibody or the antigen-binding fragment that binds to TROP2.

In some specific embodiments, the multispecific antigen-binding protein comprises a first polypeptide set forth in SEQ ID NO: 51 and a second polypeptide set forth in SEQ ID NO: 50.

In some specific embodiments, the multispecific antigen-binding protein comprises more than one first polypeptide set forth in SEQ ID NO: 51 and more than one second polypeptide set forth in SEQ ID NO: 50. In some embodiments, the multispecific antigen-binding protein forms a homodimer through a disulfide bond in a hinge region of the first polypeptide.

In some embodiments, the multispecific antigen-binding protein described in the present application can (a) bind to HER3 and TROP2 simultaneously (ELISA method), e.g., with an EC₅₀ value of <100 nM, preferably <50 nM; (b) bind to cells expressing HER3 or TROP2; and/or (c) bridge cells expressing TROP2 and HER3.

### Protein-Drug Conjugate

In another aspect, the present application further relates to a protein-drug conjugate having the structure of formula I:

P-(L1-spl-L2-sp2-D)n (I),

wherein protein P comprises the multispecific antigen-binding protein described in the present application, D is a substance having biological activity, L1 is a linker for linking to P, sp1 is a first spacing unit, L2 is a cleavable linker, sp2 is a second spacing unit and is linked to D, and n = 1-20.

In some embodiments, L1 is selected from: 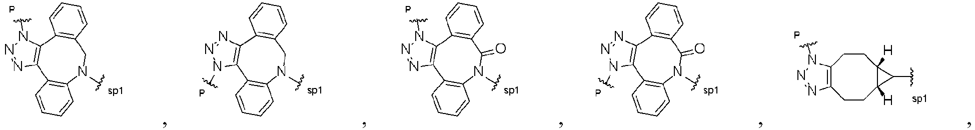 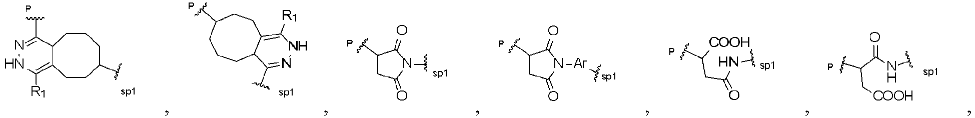 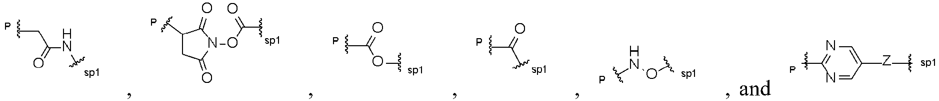 (the side linked to the protein is denoted by P, and the side linked to the first spacing unit is denoted by sp 1), wherein Ar represents C₆₋₁₀ arylene optionally substituted with halogen or C₁₋₆ alkyl; R₁ is selected from hydrogen, halogen, and C₁₋₆ alkyl; Z is selected from a direct bond, C₂₋₆ alkynylene, C₂₋₆ alkenylene, C₆₋₁₀ arylene, 5-10 membered heteroarylene, amido, sulfonamido, imino, and CF₂.

In some embodiments, L1 is selected from 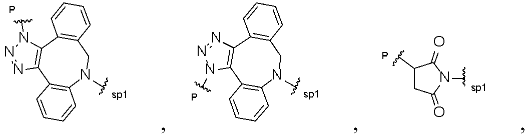 and

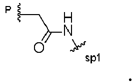

In some embodiments, the first spacing unit sp1 has the structure represented by formula II: 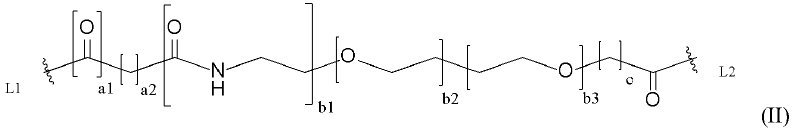 (the side linked to L1 is denoted by L1, and the side linked to L2 is denoted by L2), wherein a1 = 0 or 1, a2 = an integer from 0 to 6, b1 = 0 or 1, b2 = an integer from 0 to 16, b3 = an integer from 0 to 16, and c = an integer from 0 to 6, and at least one of b2 and b3 is 0.

In some embodiments, in the structure of sp1, a1 = 1; in some other embodiments, a1 = 0.

In some embodiments, a2 = 0, 1, 2, 3, 4, 5, or 6.

In some embodiments, b1 = 0 or 1.

In some embodiments, b2 = 0, 1, 2, 3, 4, 5, 6, 7, 8, 9, 10, 11, 12, 13, 14, 15, or 16.

In some embodiments, b3 = 0, 1, 2, 3, 4, 5, 6, 7, 8, 9, 10, 11, 12, 13, 14, 15, or 16.

In some embodiments, c = 0, 1, 2, 3, 4, 5, or 6.

The above optional embodiments of a1, a2, b1, b2, b3, and c can be combined arbitrarily, provided that at least one of b2 and b3 is 0.

In some specific embodiments, the structure of sp1 is selected from the following groups:
(1) a1 = 0, a2 = 2, 3, 4, 5, or 6, b1 = 0, b2 = 0, b3 = 0, and c = 0;
(2) a1 = 0, a2 = 0, b1 = 0, b2 = 0, b3 = 0, and c = 2, 3, 4, 5, or 6;
(3) a1 = 1, a2 = 2, 3, 4, 5, or 6, b1 = 1, b2 = 2, 3, 4, 5, 6, 7, or 8, b3 = 0, and c = 0;
(4) a1 = 0, a2 = 2, 3, 4, 5, or 6, b1 = 1, b2 = 2, 3, 4, 5, 6, 7, or 8, b3 = 0, and c = 0; and
(5) a1 = 1, a2 = 0, b1 = 0, b2 = 0, b3 = 2, 3, 4, 5, 6, 7, or 8, and c = 2, 3, 4, 5, or 6.

In some embodiments, the structure of sp1 is specifically selected from the following groups:
(1.1) a1 = 0, a2 = 2, b1 = 0, b2 = 0, b3 = 0, and c = 0;
(1.2) a1 = 0, a2 = 3, b1 = 0, b2 = 0, b3 = 0, and c = 0;
(1.3) a1 = 0, a2 = 4, b1 = 0, b2 = 0, b3 = 0, and c = 0;
(1.4) a1 = 0, a2 = 5, b1 = 0, b2 = 0, b3 = 0, and c = 0;
(1.5) a1 = 0, a2 = 6, b1 = 0, b2 = 0, b3 = 0, and c = 0;
(2.1) a1 = 0, a2 = 0, b1 = 0, b2 = 0, b3 = 0, and c = 2;
(2.2) a1 = 0, a2 = 0, b1 = 0, b2 = 0, b3 = 0, and c = 3;
(2.3) a1 = 0, a2 = 0, b1 = 0, b2 = 0, b3 = 0, and c = 4;
(2.4) a1 = 0, a2 = 0, b1 = 0, b2 = 0, b3 = 0, and c = 5;
(2.5) a1 = 0, a2 = 0, b1 = 0, b2 = 0, b3 = 0, and c = 6;
(3.1) a1 = 1, a2 = 2, b1 = 1, b2 = 2, b3 = 0, and c = 0;
(3.2) a1 = 1, a2 = 2, b1 = 1, b2 = 3, b3 = 0, and c = 0;
(3.3) a1 = 1, a2 = 2, b1 = 1, b2 = 4, b3 = 0, and c = 0;
(3.4) a1 = 1, a2 = 2, b1 = 1, b2 = 5, b3 = 0, and c = 0;
(3.5) a1 = 1, a2 = 2, b1 = 1, b2 = 6, b3 = 0, and c = 0;
(3.6) a1 = 1, a2 = 2, b1 = 1, b2 = 7, b3 = 0, and c = 0;
(3.7) a1 = 1, a2 = 2, b1 = 1, b2 = 8, b3 = 0, and c = 0;
(3.8) a1 = 1, a2 = 3, b1 = 1, b2 = 2, b3 = 0, and c = 0;
(3.9) a1 = 1, a2 = 3, b1 = 1, b2 = 3, b3 = 0, and c = 0;
(3.10) a1 = 1, a2 = 3, b1 = 1, b2 = 4, b3 = 0, and c = 0;
(3.11) a1 = 1, a2 = 3, b1 = 1, b2 = 5, b3 = 0, and c = 0;
(3.12) a1 = 1, a2 = 3, b1 = 1, b2 = 6, b3 = 0, and c = 0;
(3.13) a1 = 1, a2 = 3, b1 = 1, b2 = 7, b3 = 0, and c = 0;
(3.14) a1 = 1, a2 = 3, b1 = 1, b2 = 8, b3 = 0, and c = 0;
(3.15) a1 = 1, a2 = 4, b1 = 1, b2 = 2, b3 = 0, and c = 0;
(3.16) a1 = 1, a2 = 4, b1 = 1, b2 = 3, b3 = 0, and c = 0;
(3.17) a1 = 1, a2 = 4, b1 = 1, b2 = 4, b3 = 0, and c = 0;
(3.18) a1 = 1, a2 = 4, b1 = 1, b2 = 5, b3 = 0, and c = 0;
(3.19) a1 = 1, a2 = 4, b1 = 1, b2 = 6, b3 = 0, and c = 0;
(3.20) a1 = 1, a2 = 4, b1 = 1, b2 = 7, b3 = 0, and c = 0;
(3.21) a1 = 1, a2 = 4, b1 = 1, b2 = 8, b3 = 0, and c = 0;
(4.1) a1 = 0, a2 = 2, b1 = 1, b2 = 2, b3 = 0, and c = 0;
(4.2) a1 = 0, a2 = 2, b1 = 1, b2 = 3, b3 = 0, and c = 0;
(4.3) a1 = 0, a2 = 2, b1 = 1, b2 = 4, b3 = 0, and c = 0;
(4.4) a1 = 0, a2 = 2, b1 = 1, b2 = 5, b3 = 0, and c = 0;
(4.5) a1 = 0, a2 = 2, b1 = 1, b2 = 6, b3 = 0, and c = 0;
(4.6) a1 = 0, a2 = 2, b1 = 1, b2 = 7, b3 = 0, and c = 0;
(4.7) a1 = 0, a2 = 2, b1 = 1, b2 = 8, b3 = 0, and c = 0;
(4.8) a1 = 0, a2 = 3, b1 = 1, b2 = 2, b3 = 0, and c = 0;
(4.9) a1 = 0, a2 = 3, b1 = 1, b2 = 3, b3 = 0, and c = 0;
(4.10) a1 = 0, a2 = 3, b1 = 1, b2 = 4, b3 = 0, and c = 0;
(4.11) a1 = 0, a2 = 3, b1 = 1, b2 = 5, b3 = 0, and c = 0;
(4.12) a1 = 0, a2 = 3, b1 = 1, b2 = 6, b3 = 0, and c = 0;
(4.13) a1 = 0, a2 = 3, b1 = 1, b2 = 7, b3 = 0, and c = 0;
(4.14) a1 = 0, a2 = 3, b1 = 1, b2 = 8, b3 = 0, and c = 0;
(4.15) a1 = 0, a2 = 4, b1 = 1, b2 = 2, b3 = 0, and c = 0;
(4.16) a1 = 0, a2 = 4, b1 = 1, b2 = 3, b3 = 0, and c = 0;
(4.17) a1 = 0, a2 = 4, b1 = 1, b2 = 4, b3 = 0, and c = 0;
(4.18) a1 = 0, a2 = 4, b1 = 1, b2 = 5, b3 = 0, and c = 0;
(4.19) a1 = 0, a2 = 4, b1 = 1, b2 = 6, b3 = 0, and c = 0;
(4.20) a1 = 0, a2 = 4, b1 = 1, b2 = 7, b3 = 0, and c = 0;
(4.21) a1 = 0, a2 = 4, b1 = 1, b2 = 8, b3 = 0, and c = 0;
(5.1) a1 = 1, a2 = 0, b1 = 0, b2 = 0, b3 = 2, and c = 2;
(5.2) a1 = 1, a2 = 0, b1 = 0, b2 = 0, b3 = 3, and c = 2;
(5.3) a1 = 1, a2 = 0, b1 = 0, b2 = 0, b3 = 4, and c = 2;
(5.4) a1 = 1, a2 = 0, b1 = 0, b2 = 0, b3 = 5, and c = 2;
(5.5) a1 = 1, a2 = 0, b1 = 0, b2 = 0, b3 = 6, and c = 2;
(5.6) a1 = 1, a2 = 0, b1 = 0, b2 = 0, b3 = 7, and c = 2; and
(5.7) a1 = 1, a2 = 0, b1 = 0, b2 = 0, b3 = 8, and c = 2.

In some embodiments, the cleavable linker L2 is a dipeptide, tripeptide, or tetrapeptide residue.

In some embodiments, L2 is selected from the following dipeptide residues: -Phe-Lys-, -Val-Ala-, -Val-Lys-, -Val-Cit-, -Ala-Lys-, -Phe-Cit-, -Leu-Cit-, -Ile-Cit-, -Phe-Arg-, -Trp-Cit-, -Gly-Gly-, -Ala-Ala-, -Gly-Val-, and -Gly-Glu-; the left side of the dipeptide residue is linked to sp1, and the right side thereof is linked to sp2. Preferably, L2 is selected from -Val-Ala-, -Val-Lys-, and -Val-Cit-.

In some embodiments, L2 is selected from the following tripeptide residues: -Glu-Val-Ala-, -Glu-Val-Cit-, -αGlu-Val-Ala-, -αGlu-Val-Cit-, -Val-Lys-Gly, and -Val-Cit-Gly-; the left side of the tripeptide residue is linked to sp1, and the right side thereof is linked to sp2.

In some embodiments, L2 is selected from the following tetrapeptide residues: -Gly-Gly-Phe-Gly- and -Gly-Phe-Gly-Gly-; the left side of the tetrapeptide residue is linked to sp1, and the right side thereof is linked to sp2.

In some embodiments, the second spacing unit sp2 is absent, or sp2 is selected from: 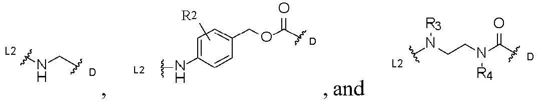 (the side linked to L2 is denoted by L2, and the side linked to the substance having biological activity D is denoted by D), wherein:
R₂ is independently selected from hydrogen, C₁₋₆ alkyl, hydroxy, amino, halogen, nitro, cyano, 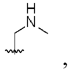 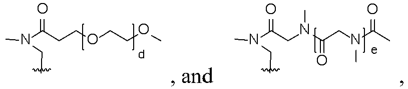 d is an integer from 1 to 20, and e is an integer from 1 to 20; R₃ and
R₄ are each independently selected from hydrogen and C₁₋₆ alkyl;
the alkyl may be optionally substituted with hydroxy, amino, halogen, nitro, and cyano.

In some embodiments, sp2 is

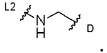

In some embodiments, sp2 is

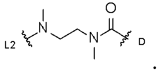

In some embodiments, sp2 is selected from 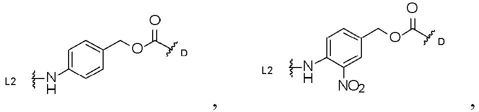 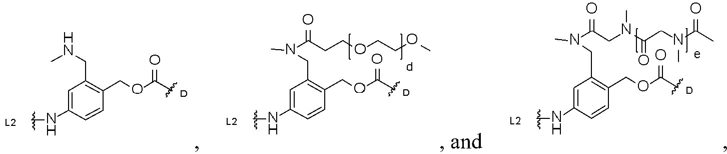 wherein d is an integer from 1 to 10, and e is an integer from 1 to 10.

In some embodiments, -L1-sp 1-L2-sp2- is selected from the following structures: 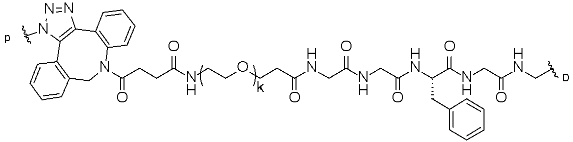 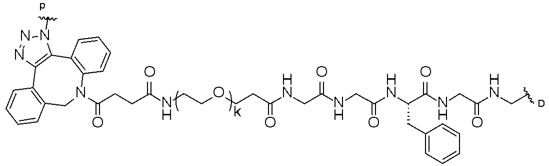 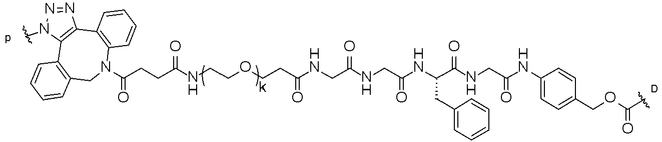 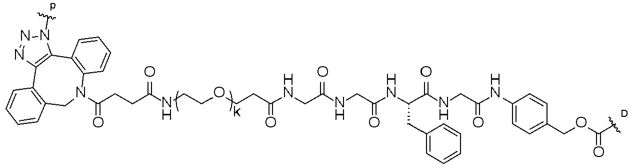 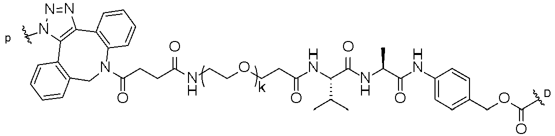 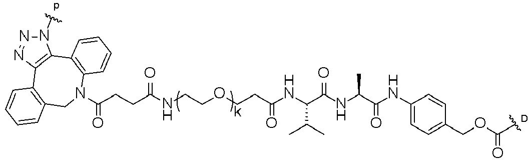 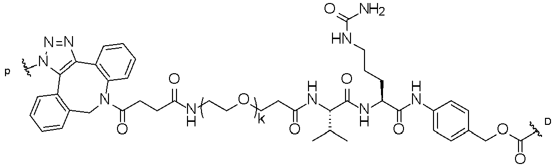 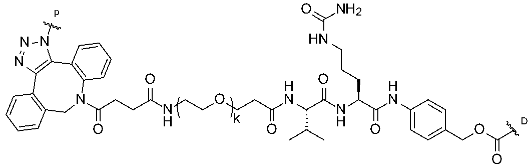 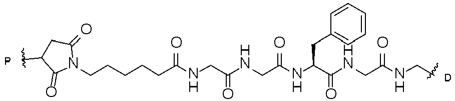 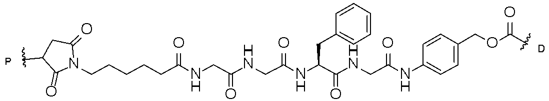 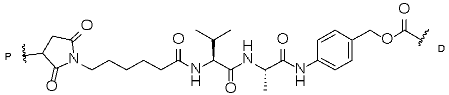 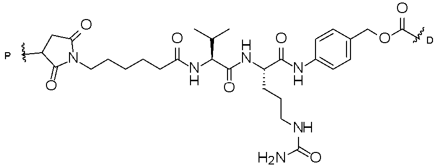 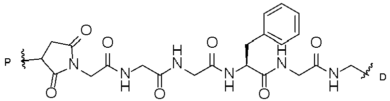 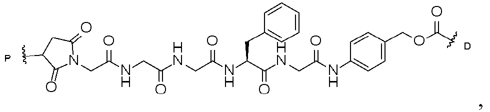 and 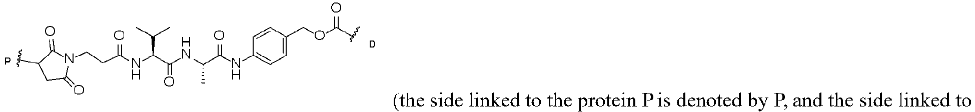 the substance having biological activity D is denoted by D), wherein k is an integer from 1 to 20.

In some embodiments, k is an integer from 2 to 16; for example, k = 2, 3, 4, 5, 6, 7, 8, 9, 10, 11, 12, 13, 14, 15, or 16.

In some embodiments, -L1-sp 1-L2-sp2- is further selected from the following structures: 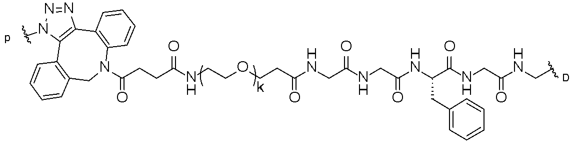 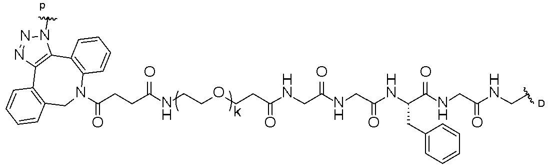 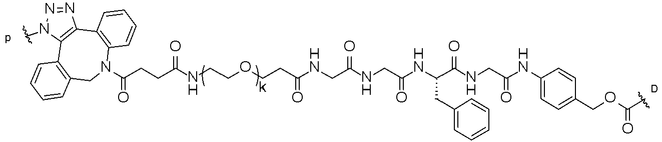 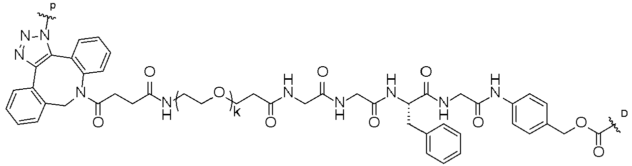 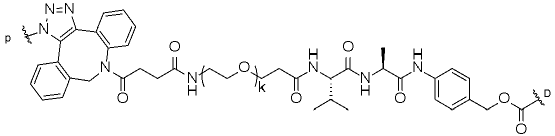 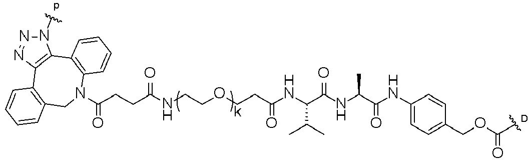 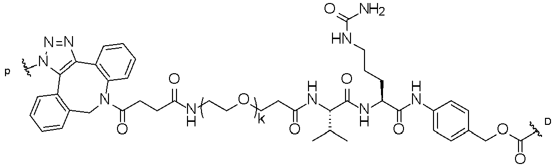 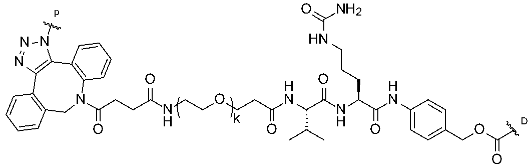 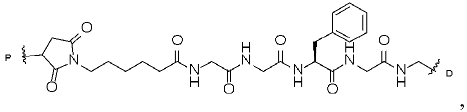 and 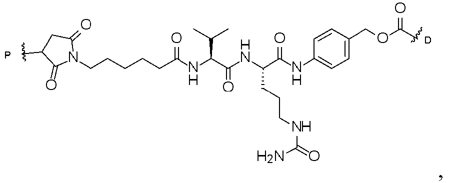 wherein k is as defined above.

In some embodiments, the substance having biological activity D may be selected from a cytotoxin, a protein kinase inhibitor, an immune agonist, a glucocorticoid, an oligonucleotide, a radioisotope, a polypeptide, and any combination thereof.

In some embodiments, D is a cytotoxin selected from a DNA alkylating agent, a DNA deconstructing agent, a topoisomerase I inhibitor, a topoisomerase II inhibitor, a microtubule inhibitor, a ribosome inhibitor, and any combination thereof.

In some specific embodiments, D is selected from an auristatin derivative, a maitansine derivative, an eribulin derivative, a tubulysin derivative, a pyrrolobenzodiazepine (PDB) derivative, a duocarmycin derivative, a calicheamicin derivative, PNU-159682 and a derivative thereof, a camptothecin derivative, an amatoxin derivative, and any combination thereof.

In some embodiments, D is selected from a camptothecin derivative, which refers to a compound having the same 5-membered fused parental core structure as camptothecin of natural origin and having substitution modifications at positions 7, 9, 10, and 11 thereof, which has equivalent or greater inhibitory activity against topoisomerase I than natural camptothecin.

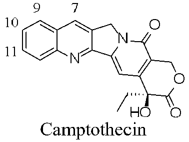

An optional camptothecin derivative may be derived from the entirety of the prior art, for example, the patent application documents WO2014057687, WO2020063676, CN111689980A, WO2022068878, WO2022068878, WO2020259258, WO2020219287, WO2022121981, WO2021173773, WO2019195665, WO2021067861, WO2022170971, WO2020200880, WO2021148501, WO2023109965, and WO2022015656, which are incorporated in the present application in their entirety.

In some embodiments, D has the structure represented by formula III: 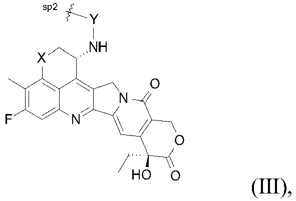
wherein X is selected from CH₂, NH, O, S, and SO₂;
Y is absent, or Y has the structure represented by 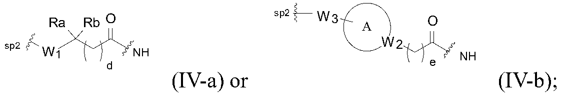
W₁ and W₃ are each independently selected from O, S, and NH, and W₂ is selected from C, CH, and N;
Rₐ and R_{b} are each independently selected from hydrogen, deuterium, halogen, C₁₋₆ alkyl, C₁₋₆ alkoxy, C₃₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
ring A is selected from the following groups: 5-10 membered cycloalkylene, 5-10 membered heterocycloalkylene, 6-10 membered arylene, and 5-10 membered heteroarylene;
the alkyl, alkoxy, cycloalkyl, heterocycloalkyl, cycloalkylene, heterocycloalkylene, arylene, and heteroarylene are each independently, optionally, and further substituted with a group selected from deuterium, halogen, hydroxy, amino, cyano, carbonyl, and nitro;
d and e are each independently selected from integers from 0 to 5.

In some embodiments, D has the structure represented by formula III-a: 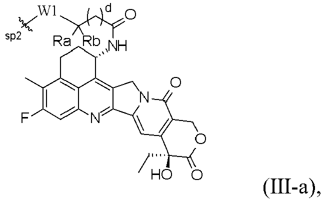
wherein W₁ is O or NH;
Rₐ and R_{b} are each independently selected from hydrogen, deuterium, halogen, C₁₋₆ alkyl, C₁₋₆ alkoxy, C₃₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
the alkyl, alkoxy, cycloalkyl, and heterocycloalkyl are each independently, optionally, and further substituted with a group selected from deuterium, halogen, hydroxy, amino, cyano, and nitro;
d is an integer from 0 to 5.

In some embodiments, W₁ is O.

In some embodiments, d is 0, 1, or 2.

In some embodiments, Rₐ is selected from hydrogen, deuterium, halogen, C₁₋₆ alkyl, C₁₋₆ alkoxy, C₃₋₆ cycloalkyl, 3-6 membered heterocycloalkyl, hydroxy, and amino, and R_{b} is hydrogen. The alkyl, alkoxy, cycloalkyl, and heterocycloalkyl are each independently, optionally, and further substituted with a group selected from deuterium, halogen, hydroxy, amino, cyano, and nitro.

In some embodiments, Rₐ and R_{b}, together with the carbon atom to which they are attached, form 3-6 membered cycloalkyl or 3-6 membered heterocycloalkyl. The cycloalkyl and heterocycloalkyl are each independently, optionally, and further substituted with a group selected from deuterium, halogen, hydroxy, amino, cyano, and nitro.

In some embodiments, D may be selected from the following groups:

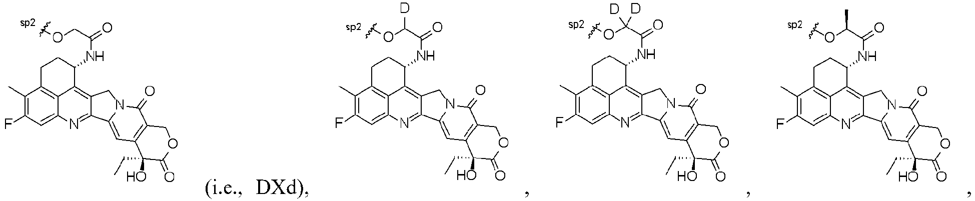

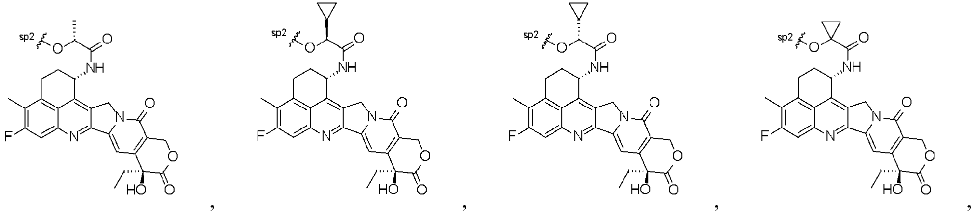

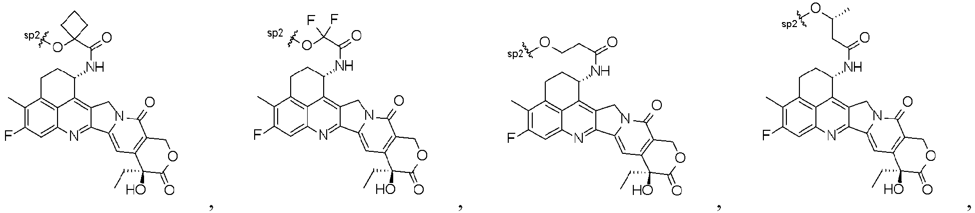

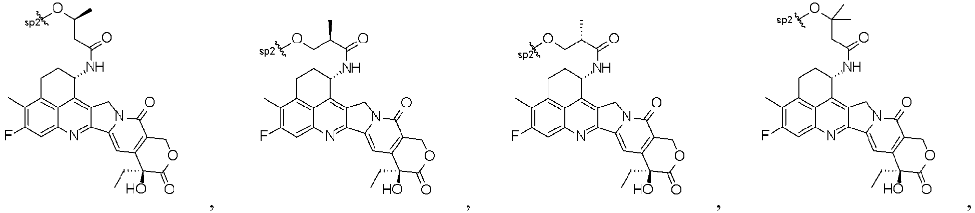

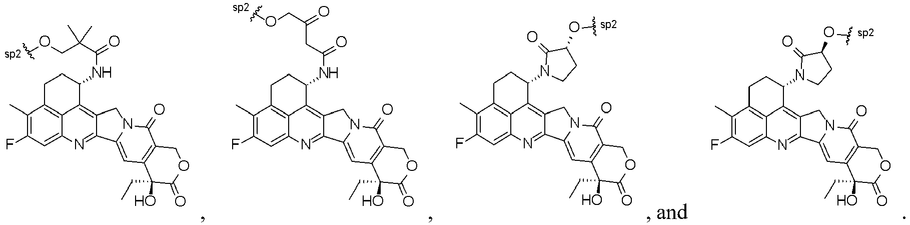

In some other embodiments, D has the structure represented by formula III-b: 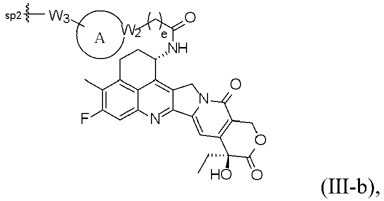
wherein W₃ is O or NH, and W₂ is C, CH, or N;
ring A is selected from the following groups: 5-10 membered cycloalkylene, 5-10 membered heterocycloalkylene, 6-10 membered arylene, and 5-10 membered heteroarylene;
the cycloalkylene, heterocycloalkylene, arylene, and heteroarylene are each independently, optionally, and further substituted with a group selected from deuterium, halogen, hydroxy, amino, cyano, carbonyl, and nitro;
e is an integer from 0 to 5.

In some embodiments, W₃ is O; in some other embodiments, W₂ is CH.

In some embodiments, e is 0, 1, or 2.

In some embodiments, ring A is 5-10 membered cycloalkylene. The cycloalkylene may be further substituted with a group selected from deuterium, halogen, hydroxy, amino, cyano, carbonyl, and nitro.

In some embodiments, D may be selected from the following groups:

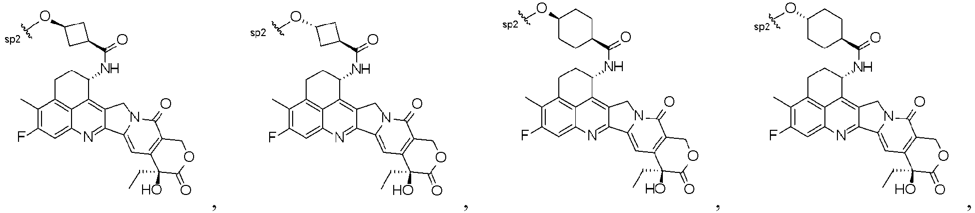

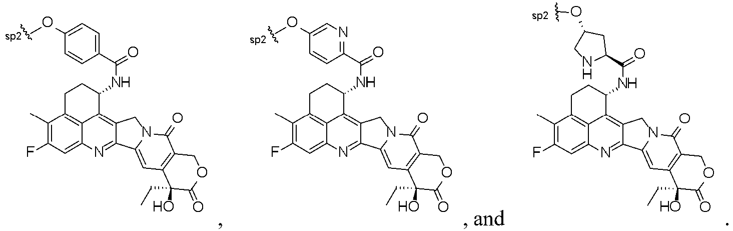

In some embodiments, D, as a camptothecin derivative, may also be selected from the following structures:

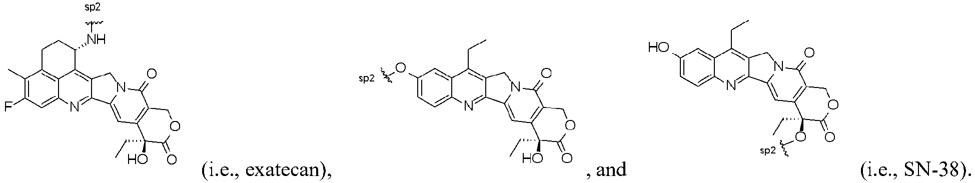

In some specific embodiments, -L1-sp1-L2-sp2-D is selected from the following structures: 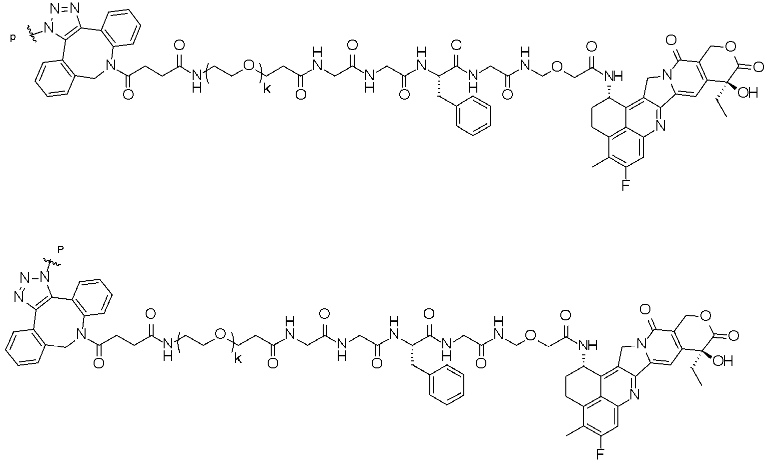 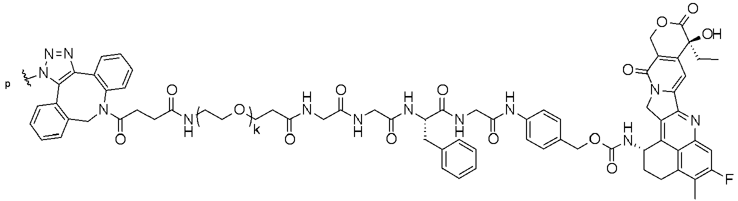 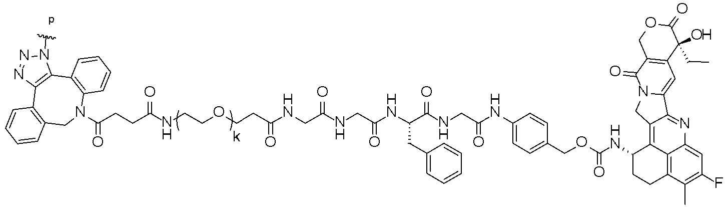 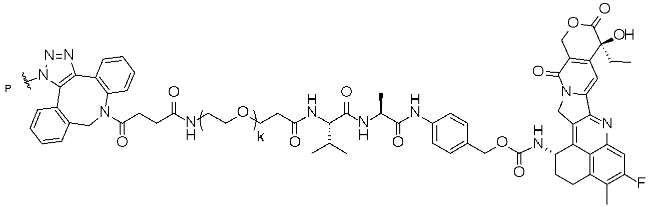 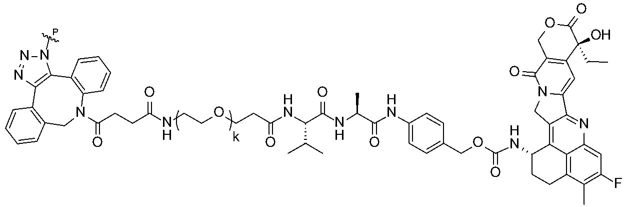 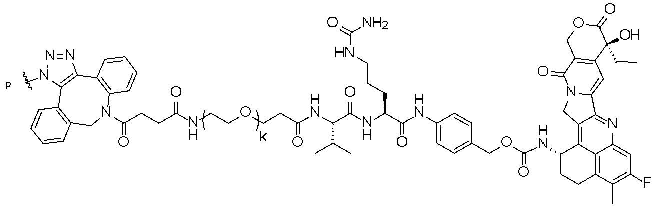 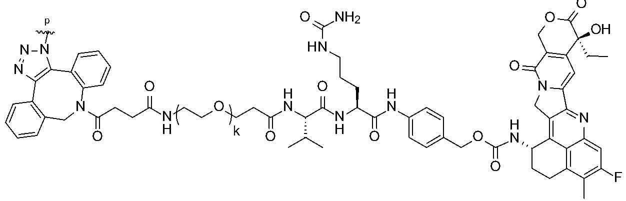 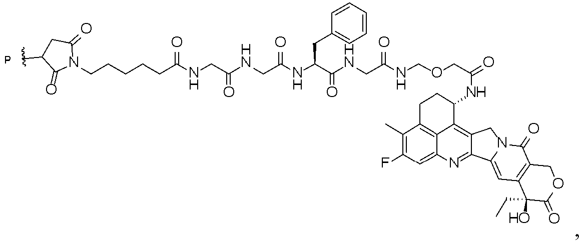 and 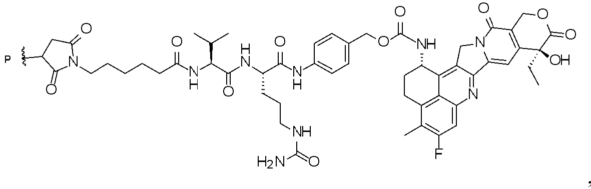 , wherein k is an integer from 1 to 20.

In some embodiments, k is an integer from 2 to 16; for example, k = 2, 3, 4, 5, 6, 7, 8, 9, 10, 11, 12, 13, 14, 15, or 16.

In some embodiments, n = 1-15; for example, n = 1-10, n = 1-8, n = 2-6, n = 2-5, or n = 2.5-3.5.

In some embodiments, in formula I, the linking unit L is linked to protein P by sulfhydryl; preferably, the sulfhydryl is derived from an antibody; more preferably, the sulfhydryl is obtained by reducing a disulfide bond between heavy chains and/or a disulfide bond between a heavy chain and a light chain.

In some other embodiments, the linking unit L is linked to protein P by an oligosaccharide; preferably, the oligosaccharide is derived from a natural glycan chain of an antibody.

In some embodiments, the oligosaccharide is derived from an N-glycan chain of an antibody.

In some embodiments, the oligosaccharide consists of 2-15 monosaccharides; preferably, the oligosaccharide consists of 2-10 monosaccharides.

In some embodiments, the oligosaccharide has the structure represented by formula V-a or formula V-b: 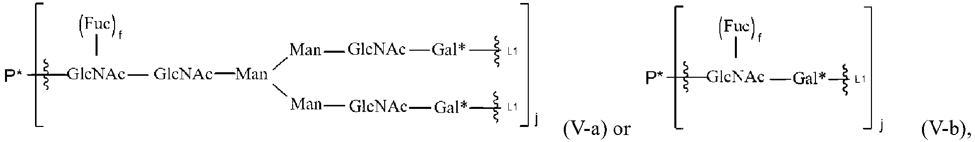 wherein P* binds to HER3 and TROP2; for example, P* comprises the antigen-binding protein described in the present application; GlcNAc is N-acetylglucosamine, Fuc is fucose, Man is mannose, f is 0 or 1, and j is 1 to 20;
Gal* is a modified galactose selected from the following structures: 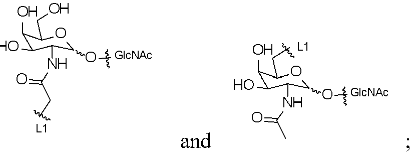 the oligosaccharide is linked to P* by the core GlcNAc.

In some embodiments, the modified galactose is linked to GlcNAc by a β-1,4-glycosidic bond.

In some embodiments, the oligosaccharide is linked to the Fc fragment of P*, preferably to the CH2 domain of the Fc fragment, and more preferably to Asn297 (numbered according to the EU index of Kabat) of the Fc fragment.

In some specific embodiments, the protein-drug conjugate described in the present application has the structure represented by formula VI: 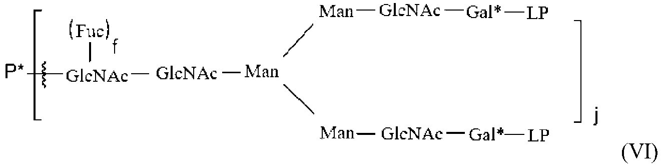 wherein P* binds to HER3 and TROP2; for example, P* comprises the antigen-binding protein described in the present application; GlcNAc is N-acetylglucosamine, Fuc is fucose, Man is mannose, f is 0 or 1, and j is 1 to 20;
Gal* is a modified galactose selected from the following structures: 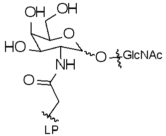 and

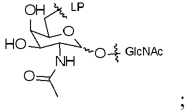

LP is selected from one of the following structures (a) to (f): 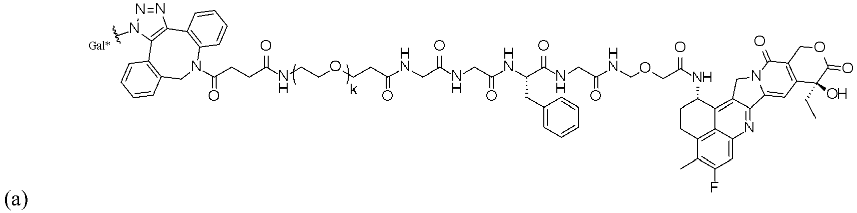 and/or 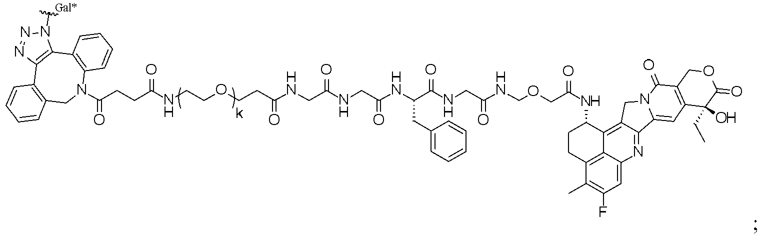 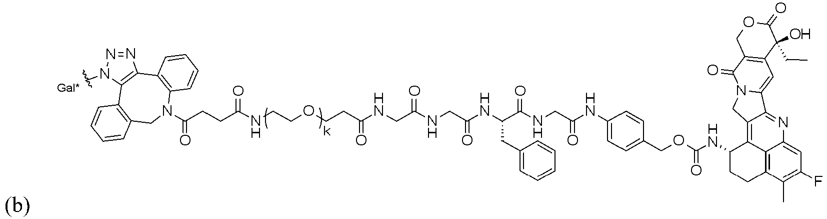 and/or 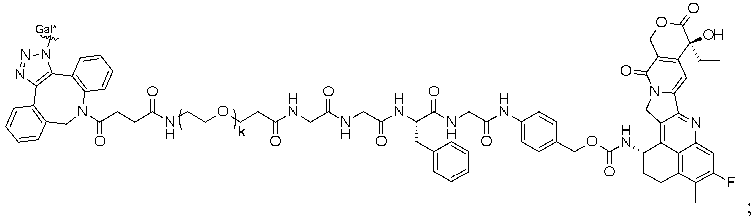 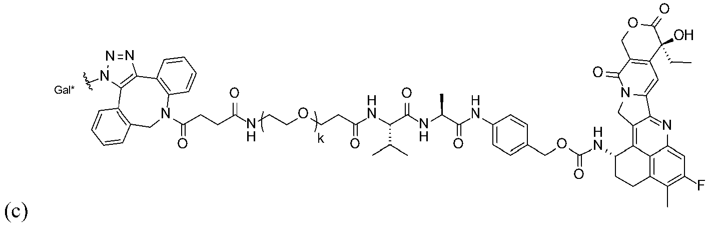 and/or 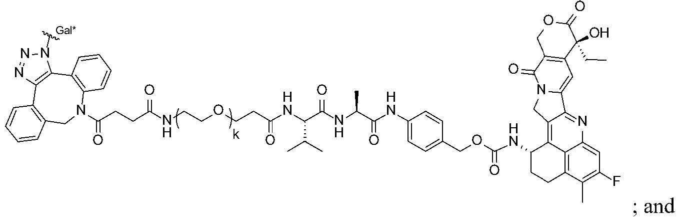 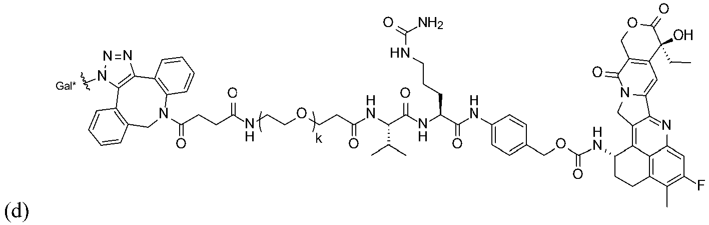 and/or 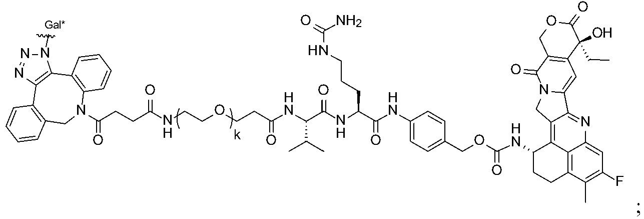 k is an integer from 1 to 20, e.g., 2, 3, 4, 5, 6, 7, 8, 9, or 10;
the core GlcNAc is directly linked to P*.

In some embodiments, the conjugate has an overall DAR value of 1 to 8.

In some embodiments, j = 1-10; for example, j = 1-8, j = 1.2-6, j = 1.5-3, or j = 1.5-2.5.

In some embodiments, the antigen-binding protein comprises a first polypeptide set forth in SEQ ID NO: 51 and a second polypeptide set forth in SEQ ID NO: 50.

In some embodiments, in the protein-drug conjugate, the oligosaccharide is linked to the Fc fragment of P*, preferably to the CH2 domain of the Fc fragment, and more preferably to Asn297 (numbered according to the EU index of Kabat) of the Fc fragment.

As described above, the oligosaccharide may be derived from a natural glycan chain of an antibody. Specifically, a preparation method for a precursor of the oligosaccharide (or referred to herein as a protein derivative) comprises the following step: reacting an antibody whose N-glycoform is mainly G0F/G0 with UDP-GalNAz or a salt thereof, or with other UDP-GalNAc azido derivatives, in the presence of a catalyst to give the precursor of the aforementioned oligosaccharide.

UDP-GalNAz has the following structure:

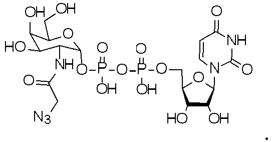

Methods for obtaining antibodies of the G0F/G0 glycoform are well-known in the art. For example, antibodies expressed by eukaryotic cells are post-translationally modified, and the glycan can be converted to the G0F/G0 form by treatment with galactosidase, which removes any terminal galactose residue and leaves the terminal N-acetylglucosamine residues.

In some other embodiments, the starting glycan chain of the G0F/G0 glycoform described in the present disclosure may also be obtained by expression using a B4GALT1 gene knockout cell line and purification. One example of knocking out the B4GALT1 gene from an expression cell line is by homologous recombination technology, and other examples of knocking out the B4GALT1 gene include the use of zinc-finger nucleases (ZFNs) or transcription activator-like effector nucleases (TALENs). Specific methods are described in reports such as Nature Biotechnology, volume 33, pages 842-844 (2015). Int. J. Mol. Sci. 2015, 16(10), 23849-23866.

In some embodiments, the aforementioned catalyst is a galactosyltransferase or a functional variant or fragment thereof.

In some embodiments, the catalyst is β-1,4-galactosyltransferase or a functional variant or fragment thereof.

In some embodiments, the catalyst is bovine β-1,4-galactosyltransferase or human β-1,4-galactosyltransferase or a functional variant or fragment thereof.

In some embodiments, the catalyst is human β-(1,4)-GalT1 having mutation Y285L or bovine β-(1,4)-GalT1 having mutation Y289L.

In some embodiments, the catalyst is the β-1,4-acetylgalactosyltransferase disclosed in Patent Application WO2016170186.

In some embodiments, the catalyst comprises the sequence set forth in any one of SEQ ID NOs: 52-54.

### Protein Derivative

In another aspect, the present application further relates to a protein derivative having the structure represented by formula VII: 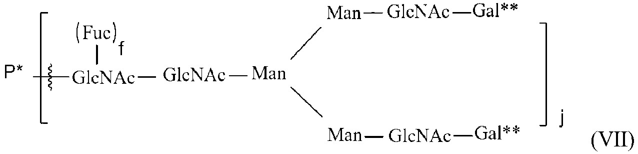 wherein P* binds to HER3 and TROP2 and comprises the antigen-binding protein described in the present application, GlcNAc is N-acetylglucosamine, Fuc is fucose, Man is mannose, f is 0 or 1, and j is 1 to 20;
Gal** is a modified galactose selected from the following structures: 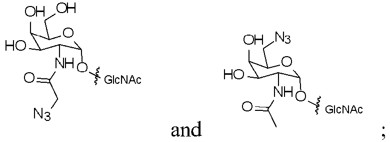 the oligosaccharide is linked to P* by the core GlcNAc.

The protein derivative may be obtained by reacting an antigen-binding protein whose N-glycoform is mainly G0F with UDP-GalNAz or a salt thereof, or with other UDP-GalNAc azido derivatives, in the presence of a catalyst.

### Nucleic Acid of Present Disclosure and Vector and Host Cell Comprising Same

In one aspect, the present disclosure provides a nucleic acid encoding any of the above binding protein, single variable domain, VHH, fusion protein, antibody, and multispecific antigen-binding protein or any fragment (e.g., antigen/target-binding fragment) thereof. The present disclosure also encompasses a nucleic acid that hybridizes with the nucleic acid described above under stringent conditions, a nucleic acid having one or more substitutions (e.g., conservative substitutions), deletions, or insertions compared to the nucleic acid described above, or a nucleic acid sequence having at least 80%, at least 85%, at least 90%, 91%, 92%, 93%, 94%, 95%, 96%, 97%, 98%, 99%, or more identity compared to the nucleic acid described above.

For example, the nucleic acid of the present disclosure comprises a nucleic acid encoding an amino acid sequence selected from any one of SEQ ID NOs: 1-51, or a nucleic acid encoding an amino acid sequence having at least 85%, 90%, 91%, 92%, 93%, 94%, 95%, 96%, 97%, 98%, or 99% identity to an amino acid sequence selected from any one of SEQ ID NOs: 1-51.

As will be appreciated by those skilled in the art, the amino acid sequence of each binding protein, single variable domain, VHH, fusion protein, antibody, or multispecific antigen-binding protein or any fragment thereof may be encoded by a variety of nucleic acid sequences due to codon degeneracy. Nucleic acid sequences encoding the molecules of the present disclosure may be produced using methods well-known in the art, e.g., by *de novo* solid-phase DNA synthesis, or by PCR amplification.

In one aspect, the present disclosure provides a nucleic acid encoding any of the above binding protein, single variable domain, VHH, fusion protein, antibody, and multispecific antigen-binding protein or any peptide chain/fragment thereof. When expressed from a suitable expression vector, the polypeptide encoded by the nucleic acid is capable of showing the ability to bind to human HER3 and/or TROP2.

In one embodiment, nucleic acids encoding chains of the binding protein, the single variable domain, the VHH, the fusion protein, the antibody, and the multispecific antigen-binding protein of the present disclosure may be in the same vector or different vectors. In yet another embodiment, nucleic acids encoding chains of the binding protein, the single variable domain, the VHH, the fusion protein, the antibody, and the multispecific antigen-binding protein of the present disclosure may be introduced into the same host cell or different host cells for expression. Thus, in some embodiments, a method for producing the binding protein, the single variable domain, the VHH, the fusion protein, the antibody, or the multispecific antigen-binding protein of the present disclosure comprises a step of: culturing a host cell comprising nucleic acids encoding chains of the molecule under conditions suitable for the expression of the chains to produce the antibody or the bispecific binding molecule or the fusion protein or a fragment thereof or the multispecific binding molecule of the present disclosure.

In another aspect, the present disclosure provides a vector comprising the nucleic acid described above. In one preferred embodiment, the vector is an expression vector. It will be fully appreciated by those skilled in the art that vectors commonly used in the art to which the present disclosure pertains can be applied to the present disclosure.

In one embodiment, the present disclosure provides a host cell comprising the nucleic acid or the vector.

The term "host cell" refers to a cell into which an exogenous polynucleotide has been introduced, including the progeny of such cells. Host cells include "transformants" and "transformed cells", which include primary transformed cells and progeny derived therefrom, regardless of the number of passages. Progeny may not be exactly the same as parental cells in terms of nucleic acid content, and may contain mutations. Mutant progeny having the same function or biological activity that are screened or selected from the initially transformed cells are included herein. Host cells are any type of cell system that can be used to produce the antibody molecules of the present disclosure, including eukaryotic cells, such as mammalian cells (e.g., CHO cells or HEK293 cells), insect cells, and yeast cells, and prokaryotic cells, such as Escherichia coli cells. Host cells include cultured cells, and also include cells within transgenic animals, transgenic plants, or cultured plant tissues or animal tissues.

### Composition

In another aspect, the present disclosure provides a composition, e.g., preferably a pharmaceutical composition, comprising one of or a combination of the HER3-binding protein, fusion protein, multispecific antigen-binding protein, or protein-drug conjugate of the present disclosure, which is formulated together with a pharmaceutically acceptable carrier.

As used herein, "pharmaceutically acceptable carrier" includes any and all physiologically compatible solvents, dispersion media, coatings, antibacterial and antifungal agents, buffers, stabilizers, isotonic agents, absorption delaying agents, etc. Preferably, the carrier is suitable for intravenous, intramuscular, subcutaneous, parenteral, spinal, or epidermal administration (e.g., by injection or infusion). According to the route of administration, the active compound, i.e., the antibody molecule, may be encapsulated in a material to protect the compound from acids and other natural conditions that may inactivate the compound.

The amount of the active ingredient that can be combined with a carrier material to prepare a single dosage form varies depending on the subject being treated and the particular mode of administration. The amount of the active ingredient that can be combined with a carrier material to prepare a single dosage form is generally an amount of the composition that produces a therapeutic effect. Generally, such amounts range from about 0.01% to about 99% of the active ingredient, for example, from about 0.1% to about 70% or from about 1% to about 30% of the active ingredient, based on 100%, combined with a pharmaceutically acceptable carrier.

Actual dosage levels of the active ingredient in the pharmaceutical composition of the present disclosure may be varied so as to obtain an amount of the active ingredient that is effective in achieving the desired therapeutic response for a particular patient, composition, and mode of administration, without being toxic to the patient. The selected dosage level depends upon a variety of pharmacokinetic factors, including the activity of the particular composition of the present disclosure employed or an ester, salt, or amide thereof, the route of administration, the time of administration, the rate of excretion of the particular compound employed, the duration of treatment, other drugs, compounds, and/or materials used in combination with the particular composition employed, the age, sex, body weight, condition, general health, and medical history of the patient being treated, and similar factors well-known in the medical arts.

In some embodiments, the protein-drug conjugate composition of the present disclosure has a DAR value of about 1.0 to 16.0, preferably about 2.0 to 12.0, more preferably about 3.0 to 6.0, and even more preferably about 3.5 to 4.5. For example, the protein-drug conjugate composition has a DAR value of about 2.0, about 2.1, about 2.2, about 2.3, about 2.4, about 2.5, about 2.6, about 2.7, about 2.8, about 2.9, about 3.0, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, or about 6.0.

The composition of the present disclosure may be administered via one or more routes of administration using one or more methods well-known in the art. It will be appreciated by those skilled in the art that the route and/or mode of administration varies depending on the desired result. Preferred routes of administration for the HER3-binding protein, multispecific antigen-binding protein, or protein-drug conjugate of the present disclosure include intravenous, intramuscular, intradermal, intraperitoneal, subcutaneous, spinal, or other parenteral routes of administration, such as injection or infusion. The phrase "parenteral administration" as used herein refers to a mode of administration other than enteral and topical administration, which is generally injection, including but not limited to intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion.

### Pharmaceutical Combination

The term "pharmaceutical combination" or "combination product" refers to a non-fixed combination product or a fixed combination product, including but not limited to a kit. The term "non-fixed combination" means that the active ingredients (e.g., (i) the HER3-binding molecule of the present disclosure, (ii) the multispecific antigen-binding protein of the present disclosure, and (iii) the protein-drug conjugate of the present disclosure) are administered, either simultaneously or sequentially (without a specific time limitation or at identical or different time intervals), to a patient as separate entities, wherein such administration provides prophylactically or therapeutically effective levels of two or more active agents in the patient. The term "fixed combination" means that two or more active agents are simultaneously administered in the form of a single entity to a patient. The dose and/or time interval of two or more active agents are/is preferably selected such that the combined use of the components can result in a therapeutic effect on the disease or condition greater than that achieved by the use of any one of the components alone. Each of the components may be in a separate formulation form, and their formulation forms may be identical or different.

Thus, in yet another aspect, the present application further provides a pharmaceutical combination or combination product comprising the binding protein, the single variable domain, the VHH, the fusion protein, the antibody, the multispecific antigen-binding protein, and the protein-drug conjugate of the present disclosure, wherein for the definitions regarding the binding protein, the single variable domain, the VHH, the fusion protein, the antibody, the multispecific antigen-binding protein, and the protein-drug conjugate in the combination, reference can be made to the technical features described in the first aspect. In some embodiments, the pharmaceutical combination or combination product may further comprise one or more additional therapeutic agents, e.g., a chemotherapeutic agent.

The present application further provides a kit of parts comprising the pharmaceutical combination. For example, the kit of parts comprises, within the same package:
- a first container containing the binding protein, the single variable domain, the VHH, the fusion protein, the antibody, the multispecific antigen-binding protein, the nucleic acid, the vector, and the host cell of the present disclosure or a pharmaceutical composition comprising the binding protein, the single variable domain, the VHH, the fusion protein, the antibody, the multispecific antigen-binding protein, the nucleic acid, the vector, and the host cell, and
- a second container containing the protein-drug conjugate of the present disclosure or a pharmaceutical composition comprising the protein-drug conjugate,
wherein for the definitions regarding the binding protein, the single variable domain, the VHH, the fusion protein, the antibody, the multispecific antigen-binding protein, the nucleic acid, the vector, the host cell, and the protein-drug conjugate, reference can be made to the technical features described in the aforementioned aspects of the present disclosure.

In some embodiments, the kit of parts further comprises, within the same package, an additional container containing an additional therapeutic agent or comprising the additional therapeutic agent. In some embodiments, the additional therapeutic agent is a chemotherapeutic agent.

### Treatment Method and Use

In yet another aspect, the present application further provides a method for treating a tumor or cancer, e.g., a solid tumor or a non-solid tumor, e.g., a hematological system tumor, comprising administering to a patient in need thereof one of or a combination of more than one of the binding protein, the single variable domain, the VHH, the fusion protein, the antibody, the multispecific antigen-binding protein, the nucleic acid, the vector, the host cell, and the protein-drug conjugate of the present disclosure. The present application further provides a method for treating a tumor or cancer, e.g., an advanced or metastatic solid malignancy, comprising administering to a patient in need thereof the pharmaceutical composition of the present application.

The description of "administering...a pharmaceutical combination" or "...in combination with..." in the present application includes both the case where a plurality of drugs are administered simultaneously and the case where a plurality of drugs are administered sequentially. When administered sequentially, the plurality of drugs are administered at intervals of no more than 24 h, e.g., no more than 18 h, no more than 15 h, no more than 12 h, no more than 10 h, no more than 8 h, no more than 5 h, no more than 3 h, no more than 2 h, no more than 1 h, or no more than 0.5 h.

In another aspect, the present application further relates to use of the HER3-binding protein, the multispecific antigen-binding protein, the nucleic acid, the vector, the host cell, and/or the protein-drug conjugate in the preparation of a medicament for treating and/or preventing a tumor.

In some embodiments, the tumor includes a solid tumor and/or a non-solid tumor, e.g., a hematological system tumor. In some embodiments, the tumor is HER3-positive and/or TROP2-positive. In some embodiments, the tumor is one whose treatment would benefit from inhibiting HER3 and/or TROP2.

### Examples

### Instruments and equipment

| **Name** | **Manufacturer** | **Model** | **No.** |
|---|---|---|---|
| Flow cytometer | Beckman | CytoFlex | 10000396 |
| Cell counter | Count-star | ICI1000 | 10000395 |
| Biosafety cabinet | Airtech | BSC-1804IIA2 | 10000388 |
| Cell incubator | Thermo | Model311 | 10000385 |
| Multimode microplate reader | Molecular Devices | SpectraMax I3x | 10000397 |

### Reagents and consumables

| **Name** | **Manufacturer** | **Catalog No.** |
|---|---|---|
| RPMI1640 | Gibco | 11875-093 |
| FBS | Hyclone | 10091-148 |
| Trypsin-EDTA | Hyclone | 25200056 |
| Penicillin-Streptomycin (PS) | Gibco | 15140-122 |
| PBS | Hyclone | SH30028.02 |
| Matrigel | Corning | 356230 |
| proA biosensors | Sartorius | 18-5012 |
| Human HER3 Protein, His Tag | ACRO | ER3-H5223 |
| SA-HRP | Abcam | ab7403 |
| APC anti-human IgG Fc Antibody | Biolegend | 366906/B343083 |
| CellTrace^{™} Violet cell proliferation Kit, for flow cytometry | Thermo Fisher | C34557/2535896 |
| CCK8 | Dojindo | CK04/SW602 |
| Nunc microplate | Nunc | 442404 |
| 96-well polypropylene microplate, F-bottom, black | Sartorius | 655209 |

### Example 1: Screening, Preparation, and Characterization of HER3 Single-Domain Antibodies

### 1.1 Construction of HER3 single-domain antibody immune library

A healthy camel was selected and immunized with a human HER3-Fc fusion protein as the antigen via multi-point intramuscular injection in the neck once every two weeks, and a total of six immunizations were performed. At the end of the last immunization, 50 mL of peripheral blood was collected from the camel into a vacuum blood collection tube, and the supernatant was collected as post-immunization serum.

Lymphocytes were isolated using density gradient centrifugation, and total RNA was extracted using an RNA extraction kit provided by QIAGEN. The extracted RNA was all reverse-transcribed into cDNA using a Super-Script III FIRST STRANDSUPERMIX kit (ThermoFisher) according to the instructions. Nested PCR was performed to amplify nucleic acid fragments encoding the variable regions of heavy-chain antibodies.

A nucleic acid fragment encoding the target heavy-chain single-domain antibody was recovered and cloned into the phage display vector pComb3XSS using the restriction endonuclease SfiI. The product was subsequently electroporated into Escherichia coli electrocompetent cells TG1 to construct an anti-HER3 immune single-domain antibody phage display library, and the library was characterized. Through gradient dilution plating, the library size was calculated to be about 1.0 × 10⁸.

### 1.2. Panning for HER3 single-domain antibody

The previously obtained phage library was subjected to panning. In the first round of panning, HER3-muFc (human HER3-mouse Fc fusion protein) was used as the screening antigen, and PD-L1-Fc was used as the negative screening antigen. In the second and third rounds of panning, HER3-muFc was used as the screening antigen, and SP-Fc (signal peptide-Fc fusion protein) was used as the negative screening antigen. After panning, clones binding to HER3-muFc while not binding to Fc were obtained.

The binding-positive phages obtained after panning were used to infect blank Escherichia coli cells, and the cells were plated. Subsequently, colonies were picked, inoculated separately into 2TY-AG (containing 10% glycerol), and left to stand overnight at room temperature. The next day, each culture was inoculated into 200 µL of 2TY-AG, with an inoculation amount of 1%, and cultured with shaking at 250 rpm at 37 °C until OD600 was about 0.5. Helper phage M13KO7 was added for infection (at a multiplicity of infection of 1:20). The cultures were left to stand at 37 °C for 15 min and then incubated at 220 rpm for 45 min. 800 µL of 2TY-AG was added to each well, and the plate was incubated overnight at 30 °C at 220 rpm and centrifuged the next day. The supernatants were collected for ELISA analysis. Plates were coated overnight at 4 °C with HER3-Fc and SP-Fc, respectively, and the obtained supernatants were added. The plates were incubated at room temperature for 2 hours. After washing, the secondary antibody goat anti-HA tag HRP (purchased from Abcam) or goat anti-mouse IgG-HRP (purchased from Thermo) was added, and the plates were incubated at room temperature for 2 hours. After washing, the TMB chromogenic solution was added, and the absorbance values at wavelengths of 450 nm and 650 nm were read. The final absorbance values were obtained by subtracting the absorbance values at the wavelength of 650 nm from the absorbance values at the wavelength of 450 nm. The results are shown in Table 1.

**Table 1. OD values of positive clones**

| Sample | OD (HER3-Fc) | OD (SP-Fc) | Sample | OD (HER3-Fc) | OD (SP-Fc) |
|---|---|---|---|---|---|
| iBT42 | 2.992 | 0.062 | iBT19 | 2.423 | 0.06 |
| iBT47 | 2.928 | 0.079 | iBT27 | 2.414 | 0.046 |
| iBT44 | 2.843 | 0.055 | iBT17 | 2.382 | 0.055 |
| iBT16 | 2.764 | 0.051 | iBT34 | 2.318 | 0.044 |
| iBT6 | 2.754 | 0.059 | iBT28 | 1.925 | 0.052 |
| iBT11 | 2.741 | 0.052 | iBT40 | 1.915 | 0.057 |
| iBT21 | 2.739 | 0.059 | iBT14 | 1.799 | 0.056 |
| iBT4 | 2.677 | 0.081 | iBT7 | 1.717 | 0.195 |
| iBT9 | 2.64 | 0.061 | iBT22 | 1.695 | 0.053 |
| iBT24 | 2.56 | 0.051 | iBT3 | 1.683 | 0.042 |
| iBT10 | 2.522 | 0.062 | iBT33 | 1.593 | 0.047 |
| iBT13 | 2.515 | 0.051 | iBT46 | 1.502 | 0.053 |
| iBT41 | 2.505 | 0.064 | iBT25 | 1.448 | 0.068 |
| iBT23 | 2.501 | 0.05 | iBT20 | 1.359 | 0.049 |
| iBT12 | 2.486 | 0.048 | iBT26 | 1.337 | 0.051 |
| iBT39 | 2.474 | 0.049 | iBT45 | 0.859 | 0.046 |
| iBT32 | 2.472 | 0.099 | iBT35 | 0.512 | 0.052 |
| iBT1 | 2.47 | 0.051 | iBT15 | 0.484 | 0.047 |
| iBT38 | 2.468 | 0.047 | iBT43 | 0.324 | 0.053 |
| iBT18 | 2.462 | 0.052 | iBT31 | 0.3 | 0.115 |
| iBT5 | 2.459 | 0.05 | iBT8 | 0.108 | 0.054 |
| iBT2 | 2.439 | 0.046 | iBT36 | 0.101 | 0.045 |
| iBT29 | 2.432 | 0.048 | iBT30 | 0.096 | 0.04 |
| iBT37 | 2.431 | 0.055 | | | |

The positive clones specifically binding to HER3 obtained by screening were sequenced. The amino acid sequence of iBT 11 is shown below:

QVQLQESGGGSVQSGGSLRLSCAASGYTTSSVCMAWFRQAPGNEREGVAHITRDGRTMYADSVRGRFTIS
QDNAKNTLFLQMNSLKPEDTGMYYCAARVCEWRSTVQAPRSEAYQLWGRGTQVTVSS (SEQ ID NO: 1)

### 1.3. Preparation of HER3 single-domain antibody and its Fc fusion protein

A sequence fragment encoding the HER3 single-domain antibody VHH was amplified by PCR and fused with a DNA fragment encoding human IgG1-Fc, or a His tag (Chis) was fused to its C-terminus. The resulting sequence was cloned into a conventional mammalian expression vector to obtain a recombinant plasmid for expressing an HER3 single-domain antibody-Fc/Chis fusion protein in mammals. The universal primers used for PCR amplification are shown below:
Upstream primer cccACCGGTCAGGTGCAGCTGCAGGAGTC (SEQ ID NO: 57)
Downstream primer cccGGATCCTGAGGAGACGGTGACCTGG (SEQ ID NO: 58)

The constructed plasmid vectors were transfected into HEK293 cells for transient antibody expression. The recombinant expression plasmids were diluted with Freestyle293 culture medium, and a PEI (polyethylenimine) solution required for transformation was added. Each plasmid/PEI mixture was added to a suspension of HEK293 cells, and suspension cell culture was performed at 37 °C with 5% CO₂. After 5-6 days of culture, the transient expression culture supernatant was collected and purified by Protein A affinity chromatography or using a Ni column to obtain the target HER3 single-domain antibody-Fc/Chis fusion protein.

### 1.4. Determination of affinity for HER3

The affinity of the HER3 single-domain antibody was determined by ELISA: Coating was performed overnight at 4 °C with 5 µg/mL Her3-muFc. The HER3 single-domain antibody-Chis to be tested was serially diluted 3-fold with 1% BSA + 0.05% PBST20 from a starting concentration of 5 µg/mL to obtain a total of 10 gradients, and incubation was performed at room temperature for 2 h. After plate washing with 1% BSA + 0.05% PBST20, the secondary antibody anti-6*his tag HRP (purchased from abcam, diluted in a 1:10,000 ratio) was added. After plate washing with 1% BSA + 0.05% PBST20, the TMB chromogenic solution was added, and color development was performed at room temperature for 5 min. The absorbance values at wavelengths of 450 nm and 650 nm were read. The final absorbance values were obtained by subtracting the absorbance values at the wavelength of 650 nm from the absorbance values at the wavelength of 450 nm. The results are shown in FIG. 1. The EC50 value of iBT11-Chis was 5.39 ng/mL.

The binding kinetics of the HER3 single-domain antibody-Fc fusion protein to HER3-chis was studied using biolayer interferometry (BLI). The iBT11-Fc fusion protein was diluted to 10 µg/mL and then immobilized on an AHC biosensor, and then HER3-chis was diluted to 5 gradients: 100 nM, 50 nM, 25 nM, 12.5 nM, and 6.25 nM, and allowed to bind to the immobilized iBT11-Fc fusion protein. The equilibrium dissociation constant (KD) was calculated using Octet K2 data analysis software 9.0. The results are shown in Table 2.

**Table 2. The affinity of iBT11-Fc (BLI)**

| Sample | KD (M) | kon (1/Ms) | kdis (1/s) |
|---|---|---|---|
| iBT11-Fc | 2.27E-09 | 1.88E+05 | 4.26E-04 |

### 1.5. Humanization of HER3 single-domain antibody

The humanization was accomplished using protein surface amino acid humanization (resurfacing) and VHH humanization universal framework grafting (CDR grafting to a universal framework).

First, the universal humanization VHH framework hNbBcII10FGLA (PDB No. 3EAK), designed by Cécile Vincke et al. based on sequence homology, was obtained. The framework design is based on the nanobody NbBcII10 (PDB No. 3DWT). Modeling was performed using Modeller 9, and the relative solvent accessibility of amino acids on the framework was calculated based on the three-dimensional structure of the protein.

The specific procedure of VHH humanization universal framework grafting is as follows: A highly homologous human antibody sequence is obtained through IMGT, and the target sequence is humanized with reference to the universal humanization VHH framework hNbBcII10FGLA (PDB No. 3EAK). A highly homologous sequence framework is used as the framework template, and the CDRs are replaced with the CDR regions of the target antibody. Non-surface amino acids on the framework are then back-mutated based on the model to complete the humanization of the target antibody.

The antibody iBT11 was humanized to obtain the following humanized variants: huiBT11v1 (SEQ ID NO: 2), huiBT11v3 (SEQ ID NO: 3), huiBT11v4 (SEQ ID NO: 4), huiBT11v5 (SEQ ID NO: 5), huiBT11v6 (SEQ ID NO: 6), and huiBT11v7 (SEQ ID NO: 7).

Genes encoding the humanized sequences described above were synthesized and fused with a DNA fragment encoding human IgG1-Fc, and the resulting sequences were cloned into conventional mammalian expression vectors to obtain recombinant plasmids for expressing HER3 single-domain antibody-Fc fusion proteins in mammals. The constructed vectors were transfected into HEK293 cells for transient antibody expression. The recombinant expression plasmids were diluted with Freestyle293 culture medium, and a PEI (polyethylenimine) solution required for transformation was added. Each plasmid/PEI mixture was added to a suspension of HEK293 cells, and the cells were cultured at 37 °C at 130 rpm with 5% CO₂. After four hours, EXCELL293 culture medium and 2 mM glutamine were added, and the cells were cultured at 130 rpm. After 24 hours, 3.8 mM VPA was added. After 72 hours, 4 g/L glucose was added. After 5-6 days of culture, the transient expression culture supernatant was collected and purified by Protein A affinity chromatography to obtain the target huiBT11 single-domain antibody-Fc fusion proteins.

### 1.6. Determination of affinity of humanized antibodies

The affinity of humanized antibodies for HER3 was determined by ELISA: Coating was performed overnight at 4 °C with 5 µg/mL Her3-muFc. The antibodies were serially diluted 4-fold with 1% BSA + 0.05% PBST20 from a starting concentration of 100 µg/mL to obtain a total of 10 gradients, and incubation was performed at room temperature for 2 h. After plate washing with 1% BSA + 0.05% PBST20, the detection secondary antibody goat anti-human IgG (Fc specific)-HRP (purchased from Sigma, diluted in a 1:8000 ratio) was added, and incubation was performed at room temperature for 2 h. After plate washing with 1% BSA + 0.05% PBST20, the TMB chromogenic solution was added, and color development was performed at room temperature for 5 min. The absorbance values at wavelengths of 450 nm and 650 nm were read. The final absorbance values were obtained by subtracting the absorbance values at the wavelength of 650 nm from the absorbance values at the wavelength of 450 nm. The results are shown in Table 3.

For the sequences of the positive control antibody AV203, reference was made to Patent WO2011136911: Genes encoding AV203HC (SEQ ID NO: 55) and AV203LC (SEQ ID NO: 56) were synthesized and cloned into conventional mammalian expression vectors, and the constructed vectors were transfected into HEK293 cells for transient antibody expression.

**Table 3. The affinity of humanized antibodies (ELISA method)**

| Conc(ng/mL) | huiBT11v1-Ld-Fc | huiBT11v4-Ld-Fc | huiBT11v3-Ld-Fc | huiBT11v5-Ld-Fc | huiBT11v6-Ld-Fc | huiBT11v7-Ld-Fc | Av203 |
|---|---|---|---|---|---|---|---|
| 100000 | 2.311 | 2.3 | 2.346 | 2.313 | 2.322 | 2.381 | 2.345 |
| 25000 | 2.243 | 2.259 | 2.28 | 2.267 | 2.28 | 2.283 | 2.263 |
| 6250 | 2.347 | 2.257 | 2.262 | 2.315 | 2.272 | 2.29 | 2.24 |
| 1562.5 | 2.251 | 2.254 | 2.292 | 2.264 | 2.279 | 2.275 | 2.231 |
| 390.625 | 2.232 | 2.291 | 2.257 | 2.261 | 2.301 | 2.274 | 2.226 |
| 97.656 | 1.777 | 1.763 | 1.759 | 1.758 | 1.79 | 1.782 | 1.481 |
| 24.414 | 0.763 | 0.741 | 0.765 | 0.738 | 0.772 | 0.794 | 0.332 |
| 6.104 | 0.243 | 0.244 | 0.245 | 0.239 | 0.253 | 0.268 | 0.096 |
| 1.526 | 0.089 | 0.089 | 0.092 | 0.086 | 0.092 | 0.092 | 0.052 |
| 0.381 | 0.051 | 0.05 | 0.053 | 0.051 | 0.053 | 0.051 | 0.044 |
| 0.095 | 0.042 | 0.04 | 0.042 | 0.042 | 0.042 | 0.043 | 0.041 |
| EC50(ng/mL) | 41.7 | 42.4 | 42.4 | 43.3 | 41.2 | 41 | 70.7 |

### 1.7. Determination of blocking activity of humanized antibodies

The blocking activity of humanized antibodies against the binding of HER3-muFc to NRG1b1 was determined by ELISA. Coating was performed overnight at 4 °C with 1 µg/mL NRG1 beta 1 (purchased from Sino) and SP-NRG1b-Chis. The antibodies to be tested were serially diluted 4-fold with 1% BSA + 0.05% PBST20 + 200 ng/mL Her3-muFc from a starting concentration of 20 µg/mL to obtain a total of 6 gradients, and incubation was performed at room temperature for 2 h. After plate washing with 1% BSA + 0.05% PBST20, the detection secondary antibody goat anti-mouse IgG1-HRP (purchased from Thermo, diluted in a 1:3000 ratio) was added, and incubation was performed at room temperature for 2 h. After plate washing with 1% BSA + 0.05% PBST20, the TMB chromogenic solution was added, and color development was performed at room temperature for 7.5 min. The absorbance values at wavelengths of 450 nm and 650 nm were read. The final absorbance values were obtained by subtracting the absorbance values at the wavelength of 650 nm from the absorbance values at the wavelength of 450 nm. The results are shown in Table 4.

**Table 4. The blocking activity of humanized antibodies (ELISA method)**

| Conc (ng/mL) | huiBT11v 1-Ld-Fc(Her3-muFc)-NRG1b1 | huiBT11v 4-Ld-Fc(Her3-muFc)-NRG1b1 | huiBT11v 3-Ld-Fc(Her3-muFc)-NRG1b1 | huiBT11v 5-Ld-Fc(Her3-muFc)-NRG1b1 | huiBT11v 6-Ld-Fc(Her3-muFc)-NRG1b1 | huiBT11v 7-Ld-Fc(Her3-muFc)-NRG1b1 | Av203(Her 3-muFc)-NRG1b1 | Av203(Her 3-muFc)-SP-NRG1b-Chis |
|---|---|---|---|---|---|---|---|---|
| 20000 | 0.251 | 0.232 | 0.249 | 0.255 | 0.245 | 0.254 | 0.354 | 0.083 |
| 5000 | 0.234 | 0.214 | 0.218 | 0.229 | 0.23 | 0.23 | 0.388 | 0.076 |
| 1250 | 0.24 | 0.224 | 0.233 | 0.235 | 0.228 | 0.241 | 0.419 | 0.076 |
| 312.5 | 0.269 | 0.256 | 0.27 | 0.269 | 0.265 | 0.274 | 0.538 | 0.102 |
| 78.125 | 1.791 | 1.817 | 1.837 | 1.864 | 1.823 | 1.8 | 2.14 | 1.981 |
| 19.531 | 2.254 | 2.23 | 2.256 | 2.265 | 2.246 | 2.23 | 2.252 | 2.205 |
| 4.883 | 2.333 | 2.289 | 2.281 | 2.305 | 2.303 | 2.26 | 2.292 | 2.208 |
| IC50 (ng/mL) | 105 | 109 | 110 | 110 | 108 | 108 | 160 | 122 |

### Example 2: Preparation of Antibody-Drug Conjugate

### 2.1. Preparation of antibody A

Genes encoding the sequences of the first polypeptide (SEQ ID NO: 51) and the second polypeptide (SEQ ID NO: 50) of the target bispecific antibody (antibody A) were synthesized, amplified by PCR using primers, and cloned into conventional pcDNA3.4 mammalian expression vectors to obtain pcDNA3.4-A recombinant plasmids.

HEK293 cells were cultured in 293M culture medium at 37 °C at 130 + 10 rpm with 5% CO₂. Before transient transfection, the HEK293 cell density was adjusted to 6 × 10⁶ cells/mL. The pcDNA3.4-A plasmids and the transfection reagent PEI were well mixed in a 1:6 ratio, and the well-mixed transfection reagent and plasmid complex was added to about 2 mL of expression culture medium OPM-293-CD05. The mixture was left to stand for 5 min. The above solution was added to 100 mL of expression culture medium, and the mixture was incubated at 37 °C at 130 + 10 rpm with 5% CO₂. On day 7, the mixture was centrifuged at 1500 rpm for 10 min, and the supernatant was collected. The collected supernatant was subjected to Protein A affinity purification to obtain the target protein. The size of the target protein was confirmed to be correct by SDS PAGE.

### 2.2. Preparation of linker-toxin

The following linker-toxin (LP1) was prepared according to the process described in Patent Application CN113264983A (the content of which is incorporated herein by reference):

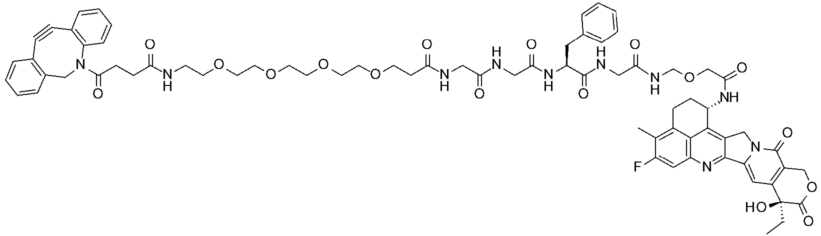

Analysis showed LP1 MS m/z (ESI): 1375.77.

### 2.3. Preparation of antibody A-ADC

Step 1: A stock solution of antibody A (mainly G0F) was desalted into an HEPES buffer, and the resulting solution was set aside for later use. Ultrapure water (210 µL), MnCl2 (1 M, 5 µL), UDP-GalNAz (100 mM, 25 µL), Tris-HCl (1 M, 5 µL), antibody A (25.5 mg/mL, 196 µL), and GalT1 (5.2 mg/mL, 12 µL, SEQ ID NO: 52) were sequentially added to a 1.5 mL centrifuge tube. The mixture was left to react at 30 °C at 400 rpm for 12 h. After the reaction was complete, the reaction mixture was purified by Protein A and concentrated by ultrafiltration into DPBS to give antibody A-(N₃)₄ for later use.

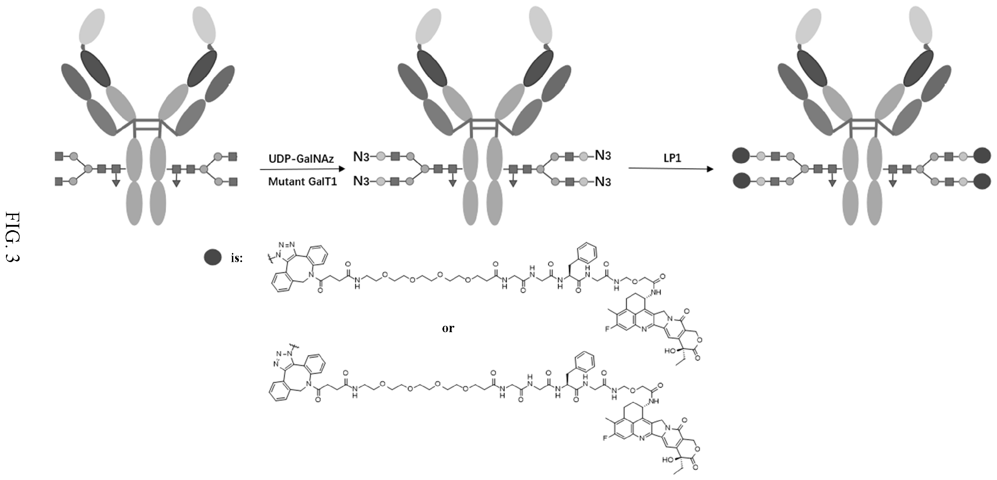

Step 2: LP1 (16 µL, 50 mM), DMSO (25 µL), and the antibody A-(N₃)₄ obtained in step 1 (10 mg/mL, 800 µL) were sequentially added to a 1.5 mL centrifuge tube. The mixture was left to react at 30 °C at 400 rpm for 12 h. After the reaction was complete, the reaction mixture was desalted into DPBS to obtain antibody A-ADC. The molecular weight was measured by mass spectrometry. The results are shown in FIG. 2. Comparison with the antibody A before conjugation confirmed that the target product had been obtained. RP-HPLC analysis confirmed that the product had a DAR value of about 3.8. The overall reaction scheme is shown in FIG. 3.

### Example 3: Characterization of Biological Activity of Antibody A-ADC

### 3.1. Determination of TROP2-binding activity of antibody A-ADC (BLI method)

The affinity of antibody A-ADC for human and monkey TROP2 was determined based on bio-layer interferometry (BLI). First, antibody A-ADC was diluted to 10 µg/mL and immobilized on a proA biosensor. Then, the TROP2 protein was diluted to obtain 7 concentrations and allowed to bind to antibody A-ADC. Binding signals with different intensities could be detected. Data were analyzed using Data Analysis HT 12.0 software, fitting was performed using a 1:1 model, and the equilibrium dissociation constant (KD) values of the samples were calculated. The results are shown in Table 5. The ability of antibody A-ADC to bind to the human TROP2 protein and the monkey TROP2 protein was comparable to that of the naked antibody.

**Table 5. The affinity of samples for the human and monkey TROP2 proteins**

| **Sample ID** | **Loading Sample ID** | **KD (M)** | **ka (1/Ms)** | **kdis (1/s)** |
|---|---|---|---|---|
| hTROP2-His | Antibody A-ADC | 2.854E-09 | 7.19E+04 | 2.052E-04 |
| | hRS7-IgG 1 | 3.008E-09 | 1.446E+05 | 4.351E-04 |
| | Antibody A | 3.376E-09 | 7.479E+04 | 2.525E-04 |
| | Antibody A-ADC | 4.443E-09 | 9.969E+04 | 4.429E-04 |
| cynoTROP2-His | hRS7-IgG 1 | 3.801E-09 | 1.795E+05 | 6.9824E-04 |
| | Antibody A | 5.099E-09 | 9.059E+04 | 4.619E-04 |

### 3.2. Determination of HER3-binding activity of antibody A-ADC (BLI method)

The affinity of antibody A-ADC for the human and monkey HER3 proteins was determined using a method similar to that described above. The results are shown in Table 6. The ability of antibody A-ADC to bind to the human HER3 protein and the monkey HER3 protein was comparable to that of the naked antibody.

**Table 6. The affinity of samples for the human and monkey HER3 proteins**

| **Sample ID** | **Loading Sample ID** | **KD (M)** | **ka (1/Ms)** | **kdis (1/s)** |
|---|---|---|---|---|
| | Antibody A-ADC | 1.233E-09 | 5.944E+04 | 7.331E-05 |
| hHER3-His | iBT11V7-Fc | 1.442E-09 | 1.052E+05 | 1.516E-04 |
| | Antibody A | 3.438E-09 | 5.933E+04 | 2.04E-04 |
| | Antibody A-ADC | 5.035E-09 | 6.058E+04 | 3.05E-04 |
| cynoHER3-His | iBT11V7-Fc | 3.292E-09 | 1.078E+05 | 3.547E-04 |
| | Antibody A | 4.46E-09 | 6.183E+04 | 2.758E-04 |

### 3.3. Determination of simultaneous binding activity of antibody A-ADC to HER3 and TROP2

The ability of antibody A-ADC to simultaneously bind to the HER3 and TROP2 proteins was assessed by ELISA. The test sample (multiple concentration gradients) was added to a microplate pre-coated with antigen 1 (HER3-His), and the plate was incubated. Then, biotinylated antigen 2 (TROP2-Biotin, prepared in-house) was added, and the plate was incubated. Finally, a horseradish peroxidase-labeled detection antibody (SA-HRP) was added to form a solid-phase antigen 1-antibody A-ADC sample-antigen 2-enzyme-labeled detection antibody complex. Absorbance values were read at 450 nm using a microplate reader, and data were analyzed by four-parameter fit curve analysis. The results are shown in Table 7. Both antibody A-ADC and antibody A could bind to the human HER3 and TROP2 proteins simultaneously, with the binding EC₅₀ values being 31.42 nM and 29.95 nM, respectively, indicating that the two were comparable in binding ability. iBT11V7-Fc and hRS7-IgG1 could not bind to the two proteins simultaneously.

**Table 7. The binding activity of antibody A-ADC and antibody A to the human HER3 and TROP2 proteins**

| **Sample** | **EC₅₀ (nM)** |
|---|---|
| Antibody A-ADC | 31.42 |
| Antibody A | 29.95 |

### 3.4. Determination of binding activity of antibody A-ADC to tumor cells

The triple-negative breast cancer cell line MDA-MB-468, the human gastric cancer cell line NCI-N87, and the human pancreatic cancer cell line BxPC-3 (all purchased from Beina Chuanglian Biotech Co., Ltd.) (1.0 × 10⁵/well) were separately co-incubated with antibody A-ADC and antibody A (the highest final concentration: 200 nM, 3-fold serial dilution, 10 concentrations in total) on ice for 30 min. After 2 washes, the APC anti-human IgG Fc fluorescent antibody was added, and incubation was performed on ice in a dark place for 30 min. After 2 washes, the fluorescence intensity on the cell surface was measured using a flow cytometer. The flow cytometry results were analyzed using CytExpert software, the mean fluorescence intensity (MFI) of each test sample was calculated, and four-parameter fitting was performed for the experimental results using GraphPad Prism 7.0.

The results are shown in Table 8. Antibody A-ADC and the naked antibody, antibody A, exhibited similar binding activity to tumor cells.

**Table 8. The binding activity of antibody A-ADC to tumor cells**

| **Sample** | | **EC₅₀ (nM)** | |
|---|---|---|---|
| | **MDA-MB-468** | **NCI-N87** | **BxPC-3** |
| Antibody A-ADC | 4.959 | 2.93 | 4.18 |
| Antibody A | 4.732 | 2.927 | 3.538 |

### 3.5. Determination of bridging effect of antibody A-ADC on 293T-TROP2 and 293T-HER3 cells

293T-TROP2-GFP cells (1.0 × 10⁵/well, HEK293T cells expressing TROP2 on the cell surface and expressing green fluorescent protein intracellularly, prepared in-house) and 293T-HER3-CellTrace Violet cells (1.0 × 10⁵/well, expressing HER3 on the cell surface, labeled with CellTrace^{™} Violet fluorescent dye, purchased from Kyinno Biotechnology Co., Ltd.) were mixed in a 1:1 ratio. After the cell mixture was well mixed with antibody A-ADC, antibody A, and a mixture of iBT11V7-FC and hRS7-IgG1 (concentration range: 0.016-50 nM), the resulting mixtures were incubated on ice in a dark place for 30 min. After 2 washes, fluorescence signals of 293T-HER3-CellTrace Violet cells and fluorescence signals of 293T-TROP2-GFP cells were collected by the PB450/FITC channels of a CytoFelx flow cytometer, respectively. The dual fluorescence signal events in the two-dimensional scatter plots of the PB450 channel and the FITC channel reflected the bridging effect of antibody A-ADC on the two types of target cells.

The results are shown in Table 9. As the concentration of antibody A-ADC increased, the percentage of dual fluorescence signal events among the total events in the test sample gradually increased from 1.82% to 22.46%, showing significant concentration dependence; however, as the concentration of the drug continued to increase, the percentage of dual fluorescence signal events among the total events gradually decreased, and the bridging effect of antibody A-ADC on the two types of cells decreased, showing a "Hook-effect". The ability of antibody A to bridge the two types of cells was comparable to that of antibody A-ADC. The results show that antibody A, as an anti-HER3 and TROP2 bispecific antibody-drug conjugate, could bind to TROP2- and HER3-positive cells simultaneously and had the ability to bridge the two types of cells. In contrast, the mixture of the anti-TROP2 monoclonal antibody and the anti-HER3 single-domain antibody-Fc fusion protein showed no significant bridging effect.

**Table 9. The bridging effect of antibody A-ADC on TROP2- and HER3-positive cells**

| **Double positive events %** | **Conc. nM** | | | | | |
|---|---|---|---|---|---|---|
| | 50 | 10 | 2 | 0.4 | 0.08 | 0.016 |
| **Antibody A-ADC** | 1.34% | 14.65% | 22.46% | 17.47% | 10.41% | 1.82% |
| **Antibody A** | 1.93% | 18.85% | 21.99% | 17.65% | 7.00% | 1.39% |
| **iBT11V7-FC+hRS7-IgG1** | 0.75% | 0.49% | 0.77% | 0.81% | 0.72% | 0.69% |

### 3.6. Determination of killing effect of antibody A-ADC on tumor cells

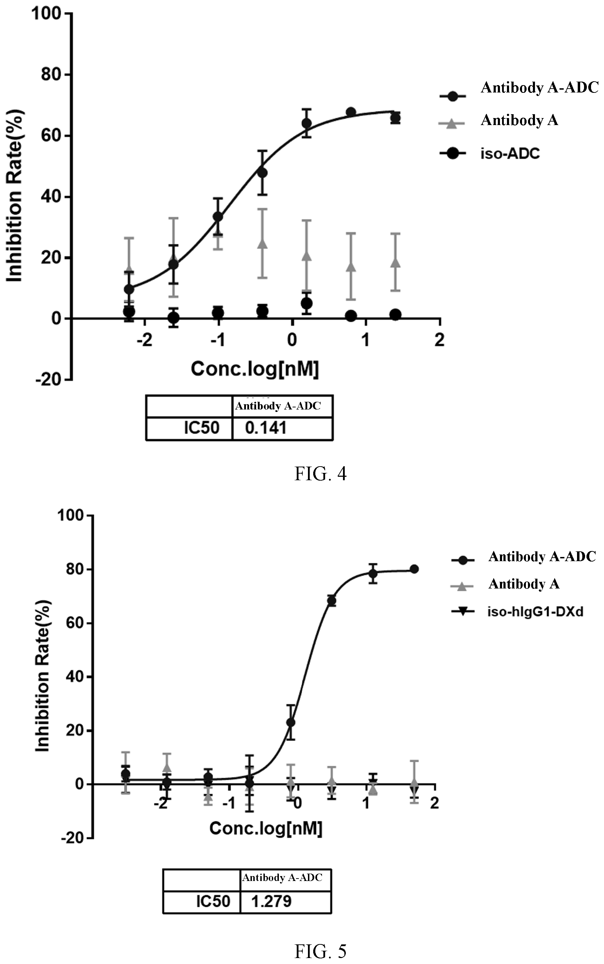

Human pancreatic cancer BxPC-3 cells were plated on 96-well plates at 5 × 10³/well and cultured overnight, and then antibody A-ADC, antibody A, and iso-ADC (an isotype control ADC drug, the antibody of which was a human IgG1 isotype control, and the toxin-linker of which was the same as that of antibody A-ADC, obtained by the same preparation process as antibody A-ADC) were separately added, with the final concentrations being 0.006-25 nM. The cells were cultured at 37 °C for 120 h. The OD values at 450/650 nm were determined using the CCK-8 method and a microplate reader, the viability of the experimental cells was calculated, and thus, the inhibition rate of each experimental group was calculated. Then, the logarithmic values of the standard curve sample concentration (x) and the calculated inhibition rates (%) (y) were fitted using the 4-parameter logistic fit in GraphPad Prism 7.0 software to calculate the IC₅₀ of the test samples. The experimental results are shown in FIG. 4. Antibody A-ADC exhibited a significant killing effect on BxPC-3 cells, with an IC₅₀ of 0.141 nM, while antibody A and iso-ADC exhibited no significant killing effects on BxPC-3 cells.

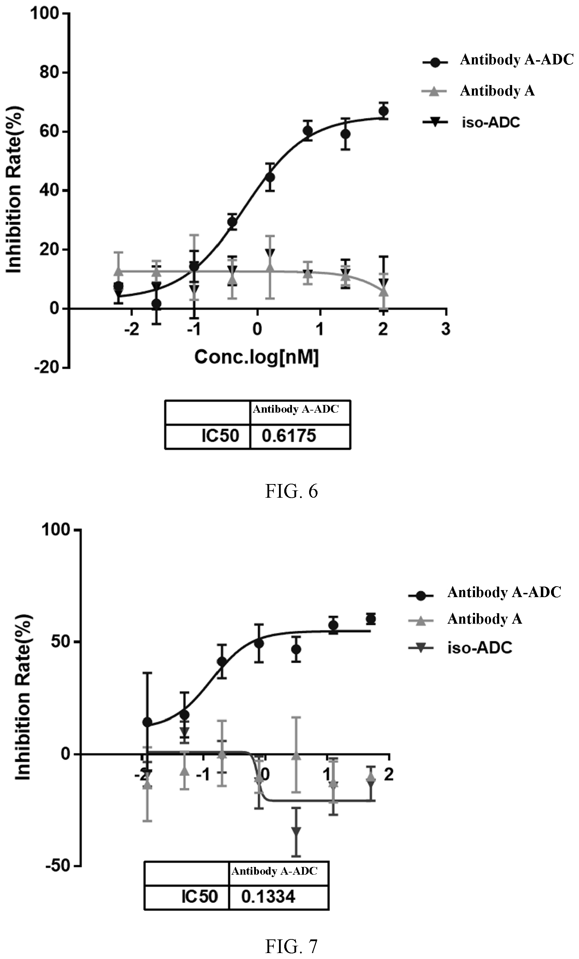

The killing effects of antibody A-ADC on the human epidermal carcinoma cell line A431 (purchased from Beina Chuanglian Biotech Co., Ltd., the final concentrations of the sample: 0.003-50 nM), the human pancreatic cancer cell line CAPAN-2 (purchased from Beina Chuanglian Biotech Co., Ltd., the final concentrations of the sample: 0.006-100 nM), and the human lung adenocarcinoma cell line HCC827 (purchased from Nanjing Cobioer Biosciences Co., Ltd., the final concentrations of the sample: 0.012-50 nM) were tested using a similar method. The results are shown in FIGs. 5-7. Antibody A-ADC exhibited significant killing effects on all these tumor cells, while antibody A and iso-ADC exhibited no significant killing effect.

### 3.7. Determination of bystander killing effect of antibody A-ADC on TROP2- and HER3-negative cells

To investigate the bystander effect of antibody A-ADC, a BxPC-3 (TROP2- and HER3-positive) mono-culture system, a 293T-GFP (TROP2- and HER3-negative) mono-culture system, and a BxPC-3 and 293T-GFP (1:1) co-culture system were treated with antibody A-ADC and iso-ADC. After 96 h, the cells in all sample wells were counted and assayed using a flow cytometer:
Mono-culture plating: The density of both cell types was adjusted to 3 × 10⁴/mL using a culture medium (RPMI1640 + 10% FBS), and each type of cells was plated on a 96-well plate at 100 µL/well (3 × 10³ cells/well). The cells were cultured overnight in an incubator at 37 °C with 5% CO₂.

Co-culture plating: The two types of cells were mixed in a culture medium (RPMI-1640 + 10% FBS), with the density of BxPC-3 cells being 3.0 × 10⁴/mL and the density of 293T-GFP cells being 3.0 × 10⁴/mL. The cells were plated on a 96-well plate at 100 µL/well and cultured overnight at 37 °C with 5% CO₂.

Antibody A-ADC and iso-ADC (working concentration: 10 nM) were added at 100 µL/well, and a drug-free control group (negative control) was set. Three replicates were set for each sample. Then, the cells were cultured at 37 °C with 5% CO₂ for 96 h. Subsequently, 20 µL of CCK-8 was added to the mono-culture sample wells, and after 3 h of standing at 37 °C, the OD values at 450/650 nm were determined using a microplate reader. The cells in the co-culture sample wells were digested with trypsin and counted, and the remaining cells were washed once with DPBS and then assayed using a flow cytometer. The number of cells N was determined using a cell counter, and the proportion of TROP2- and HER3-positive cells (V1L% parental group) and the proportion of TROP2- and HER3-negative cells (V1R% parental group) in each concentration group of the co-culture group were determined using a flow cytometer. $\begin{matrix}For the mono - culture system : The inhibition rate \left(%\right) of the test sample at each concentration = 1 - \\ \frac{Experimental group OD value}{Control group OD value} ∗ 100 %\end{matrix}$ $\begin{matrix}For the co - culture system : The inhibition rate \left(%\right) of the test sample at each concentration against BxPC - 3 = \\ 1 - \frac{Number of cells in experimental group N ∗ Number of cells in experiemental group V 1 L % parental group}{Number of cells in control group N ∗ Control cell group V 1 L % parental group} ∗ 100 %\end{matrix}$ $\begin{matrix}The inhibition rate \left(%\right) of the test sample at each concentration against 293 T - GFP = \\ 1 - \frac{Number of cells in experimental group N ∗ Number of cells in experimental group V 1 R % parental group}{Number of cells in control group N ∗ Control cell group V 1 R % parental group} ∗ 100 %\end{matrix}$

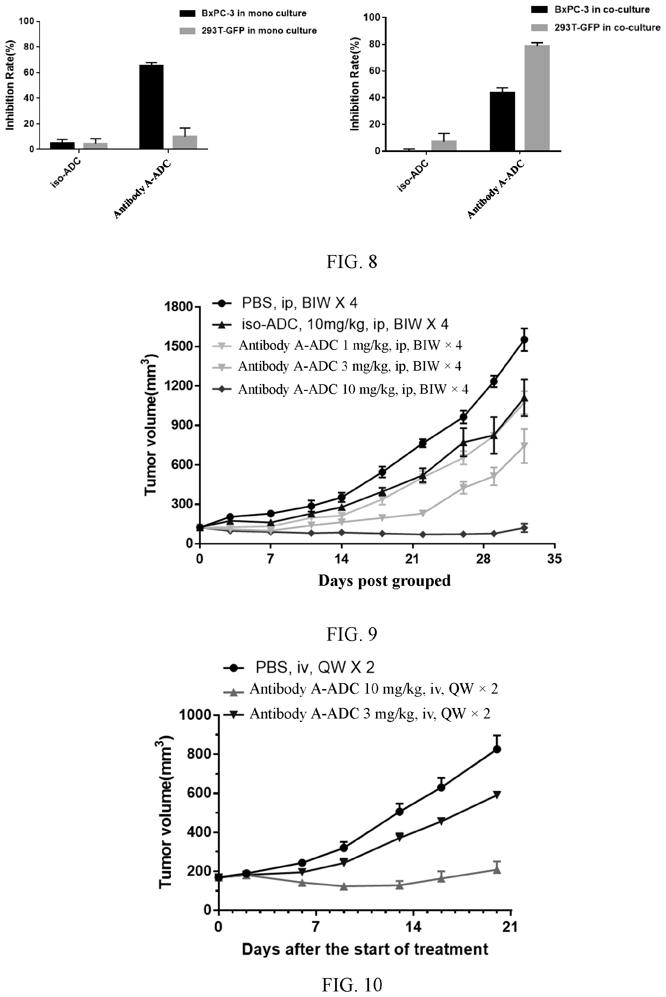

The results are shown in FIG. 8. In the mono-culture system, the average inhibition rate of 10 nM antibody A-ADC against BxPC-3 cells was 65.31%, and its killing effect on 293T-GFP cells was not significant; in the co-culture system, the average inhibition rate of 10 nM antibody A-ADC against BxPC-3 cells was 43.86%, and its average inhibition rate against 293T-GFP cells was 78.8%. It can be seen that antibody A-ADC exhibited a significant bystander killing effect on TROP2- and HER3-negative cells compared to iso-ADC.

### 3.8. Evaluation of anti-tumor effect of antibody A-ADC in human lung cancer CDX model HCC827 subcutaneous xenograft mouse animal model

BALB/c Nude mice (purchased from Vital River (Beijing) Laboratory Animal Technology Co., Ltd.) were subcutaneously injected with human HCC827 cells to establish an animal model: 0.2 mL (10 × 10⁶ cells) of HCC827 cells (mixed with Matrigel in a 1:1 volume ratio) was subcutaneously inoculated into the right dorsal side of each mouse. When the mean tumor volume reached about 123 mm³, animals were randomly divided into 5 groups of 6 according to body weight and tumor volume: a vehicle control group (PBS), an iso-ADC group (10 mg/kg), an antibody A-ADC low-dose group (1 mg/kg), an antibody A-ADC medium-dose group (3 mg/kg), and an antibody A-ADC high-dose group (10 mg/kg). The day of grouping was defined as day 0. The test articles were intraperitoneally injected on days 1, 5, 8, and 12 (4 injections in total). During the experiment, tumor volume and mouse body weight were measured twice weekly.

The results showed that during the experiment, there was no significant difference in animal body weight between the PBS group and the antibody A-ADC treatment groups, and there were no significant abnormalities in the status of the mice. As shown in FIG. 9, 32 days after grouping and administration, the mean tumor volume of the vehicle control group was 1552 mm³, and the tumor volumes of the antibody A-ADC (1 mg/kg, 3 mg/kg, and 10 mg/kg) treatment groups were 1073 mm³, 743 mm³, and 120 mm³, respectively, with TGI% (relative tumor growth inhibition rate, in percentage terms) of 33.4%, 56.5%, and 100.2%, respectively (with p values of 0.0103, 0.0001, and 0.0001, respectively).

It can be seen that in the CDX model of human lung cancer HCC827, antibody A-ADC, administered 4 times at doses ranging from 1 to 10 mg/kg, exhibited a significant dose-dependent tumor-inhibiting effect. In addition, the body weight change results showed that antibody A-ADC was well tolerated at each dose.

### 3.9. Evaluation of anti-tumor effect of antibody A-ADC in human gastric cancer CDX model NCI-N87 subcutaneous xenograft mouse animal model

0.2 mL (10 × 10⁶ cells) of NCI-N87 cells (mixed with Matrigel in a 1:1 volume ratio) was subcutaneously inoculated into the right dorsal side of each BALB/c Nude mouse. When the mean tumor volume reached about 169 mm³, animals were randomly divided into 3 groups of 6 according to body weight and tumor volume: a vehicle control group (PBS), an antibody A-ADC low-dose group (3 mg/kg), and an antibody A-ADC high-dose group (10 mg/kg). Administration was performed once weekly for a total of 2 weeks.

The results showed that the single-drug test group of each test article showed no significant influence on the body weight of the mice, and there were no significant abnormalities in the status of the mice. As shown in FIG. 10, 20 days after the start of administration, the mean tumor volume of the tumor-bearing mice in the solvent control group reached 826 mm³. In the treatment groups of the test article antibody A-ADC (10 mg/kg and 3 mg/kg), the tumor volumes were 209 mm³ and 591 mm³ (T/C% = 27.22% and 72.64%; TGI% = 94.07% and 35.65%; p < 0.0001 and p = 0.0807), respectively. The treatment group of the test article antibody A-ADC (10 mg/kg) showed a significant anti-tumor effect compared to the control group.

### 3.10. Evaluation of anti-tumor effect and tolerability of antibody A-ADC in osimertinib-resistant human lung cancer PDX model LU-01-1377 subcutaneous xenograft NOD/SCID mouse animal model

The establishment of human-derived tumor PDX models initially comes from clinical samples obtained through surgical resection, which are defined as the P0 generation after being implanted into experimental mice. After implantation of tumor tissue from the P0 generation into the next generation, the P1 generation is obtained. Similarly, this process of implanting tumor tissue into experimental mice can continue. FP3 tumors are obtained by re-thawing the P2 generation. The next generation resulting from the FP3 generation is defined as FP4, and so on. Tumor tissue from the FP8 generation was used in this pharmacodynamic experiment with the LU-01-1377 model.

A 20-30 mm³ LU-01-1377 tumor tissue block was inoculated into the right dorsal side of each NOD/SCID mouse (purchased from Vital River (Beijing) Laboratory Animal Technology Co., Ltd.), and tumors were allowed to grow. The *in vivo* pharmacodynamic experiment was conducted on day 33 after the inoculation of the LU-01-1377 tumor blocks. When the mean tumor volume reached 141 mm³, random grouping and administration were started. The day of grouping was defined as day 1, and administration was started immediately after grouping. The experiment included antibody A-ADC treatment groups (1 mg/kg, 3 mg/kg, and 10 mg/kg) and a vehicle PBS negative control group, with 6 animals in each group. After grouping, administration was performed by tail vein injection twice weekly.

The efficacy and tolerability were evaluated based on tumor growth inhibition (TGI) rates and body weight changes.

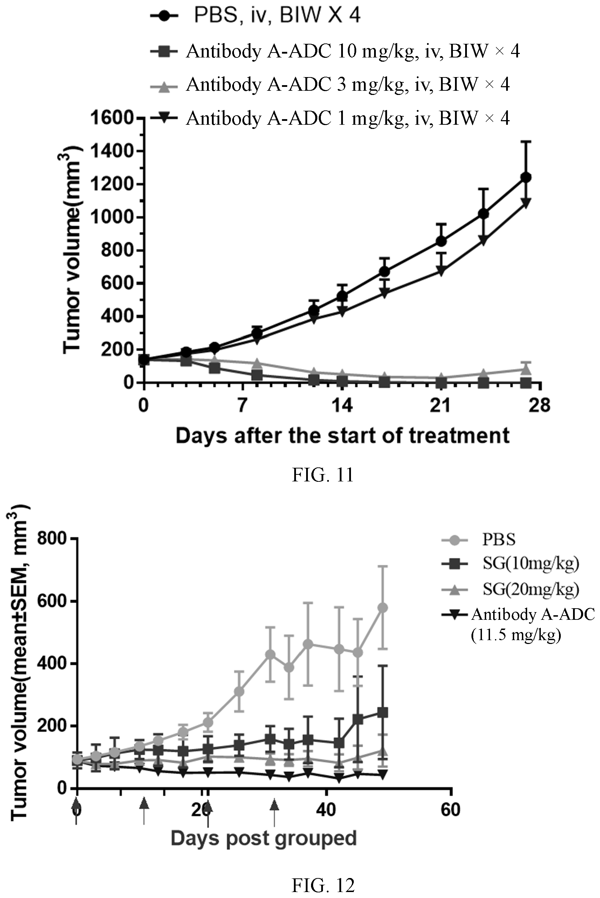

The body weight change results showed that on day 27 after the first dose, all the animals in the control group and the test drug groups survived without significant body weight loss. The anti-tumor efficacy results are shown in FIG. 11. The mean tumor volume of the mice in the PBS control group on day 27 after the first dose following grouping was 1244 + 216 mm³. The mean tumor volume of the antibody A-ADC high-dose treatment group (10 mg/kg) on day 27 after the first dose following grouping was 0 + 0 mm³ (10 mg/kg), with a relative tumor growth inhibition (TGI) rate (%) of 112.78%; there was a statistically significant difference (p < 0.0001) between this treatment group and the PBS control group. The mean tumor volume of the antibody A-ADC medium-dose treatment group (3 mg/kg) on day 27 after the first dose following grouping was 81 + 43 mm³, with a relative tumor growth inhibition (TGI) rate (%) of 105.43%; there was a statistically significant difference (p < 0.0001) between this treatment group and the PBS control group. The mean tumor volume of the antibody A-ADC low-dose treatment group (1 mg/kg) on day 27 after the first dose following grouping was 1085 + 171 mm³, with a relative tumor growth inhibition (TGI) rate (%) of 14.35%; there was no statistically significant difference (p = 0.7601) between this treatment group and the PBS control group.

### 3.11. Evaluation of anti-tumor effect of antibody A-ADC in HCC827 cell strain subcutaneous graft BALB/c nude female mouse model

100 µL (1 × 10⁶ cells) of a suspension of HCC827 cells in a mixture of PBS and Matrigel (1:1) was subcutaneously inoculated into the right dorsal side of each BALB/c nude mouse (purchased from Zhejiang Vital River Laboratory Animal Technology Co., Ltd.). After inoculation, the growth of tumors was observed regularly. When the tumors grew to a mean volume of about 93 mm³, animals were randomly grouped according to tumor size and dosed. The day of grouping was defined as day 0. The experiment included an antibody A-ADC treatment group (11.5 mg/kg), sacituzumab govitecan positive control groups (10 mg/kg and 20 mg/kg, purchased from Gilead Sciences, hereinafter referred to as SG), and a PBS blank control group, with 6 mice in each group. Administration was performed by tail vein injection once weekly, and a total of four doses were administered. The experiment was ended on day 49 after the first dose. The efficacy was evaluated based on tumor growth inhibition rates, and the safety was evaluated based on animal body weight changes and mortality.

At the end of the experiment, no animal deaths were observed in any group except for the SG 10 mg/kg group, in which one mouse died, and no abnormal weight loss was observed in the mice. The anti-tumor efficacy results are shown in FIG. 12. The mean tumor volume of the mice in the PBS group at the end of the experiment was 580 mm³. The tumor volume of the antibody A-ADC treatment group (11.5 mg/kg) was 43 mm³, with TGI (%) being 92.6% (p < 0.001) compared to the PBS group. The tumor volumes of the positive control SG 10 mg/kg group and 20 mg/kg group were 244 mm³ and 121 mm³, respectively, with TGI (%) being 58.0% (p < 0.05) and 79.2% (p < 0.001) compared to the PBS group.

### 3.12. Study of stability of antibody A-ADC in serum of different species

Antibody A-ADC was diluted with human, cynomolgus monkey, rat, and mouse serum to prepare 10 µg/mL and 100 µg/mL solutions, and then the solutions were aliquoted at 100 µL per tube, with one aliquot prepared for each time point. The aliquots were incubated at 37 °C for 0, 3, 7, 14, and 21 days, respectively, and then taken out and cryopreserved. The DXd content in serum was determined by LC-MS/MS: DXd in the test samples was extracted by protein precipitation, and the treated samples were analyzed by liquid chromatography-tandem mass spectrometry (LC-MS/MS).

The results showed that by day 21, the DXd release rates of antibody A-ADC (100 µg/mL) in human, cynomolgus monkey, rat, and mouse serum were 0.637%, 0.698%, 0.355%, and 0.439%, respectively. The DXd release rates of antibody A-ADC (10 µg/mL) in human, cynomolgus monkey, rat, and mouse serum were 0.696%, 0.702%, 0.368%, and 0.492%, respectively. This indicates that antibody A-ADC could remain stable in the serum of various species, with relatively low payload toxin (DXd) release rates.

### SEQUENCE LISTING

SEQ ID NO: 1 (iBT11)

QVQLQESGGGSVQSGGSLRLSCAASGYTTSSVCMAWFRQAPGNEREGVAHITRDGRTMYADSVRGRFTIS
QDNAKNTLFLQMNSLKPEDTGMYYCAARVCEWRSTVQAPRSEAYQLWGRGTQVTVSS

SEQ ID NO: 2 (huiBT11v1)

QVQLVESGGGSVQSGGSLRLSCAASGYTTSSVCMAWFRQAPGNEREGVAHITRDGRTMYADSVRGRFTIS
RDNSKNTLYLQMNSLRAEDTAVYYCAARVCEWRSTVQAPRSEAYQLWGQGTLVTVSS

SEQ ID NO: 3 (huiBT11v3)

QVQLVESGGGSVQSGGSLRLSCAASGYTTSSVCMAWFRQAPGNGLEGVAHITRDGRTMYADSVRGRFTIS
RDNSKNTLYLQMNSLRAEDTAVYYCAARVCEWRSTVQAPRSEAYQLWGQGTLVTVSS

SEQ ID NO: 4 (huiBT11v4)

QVQLVESGGGLVQPGGSLRLSCAASGYTTSSVCMAWFRQAPGKGLEGVAHITRDGRTMYADSVRGRFTIS
RDNSKNTLYLQMNSLRAEDTAVYYCAARVCEWRSTVQAPRSEAYQLWGQGTLVTVSS

SEQ ID NO: 5 (huiBT11v5)

QVQLVESGGGSVQSGGSLRLSCAASGYTTSSVCMAWFRQAPGKGLEGVAHITRDGRTMYADSVRGRFTIS
RDNSKNTLYLQMNSLRAEDTAVYYCAARVCEWRSTVQAPRSEAYQLWGQGTLVTVSS

SEQ ID NO: 6 (huiBT11v6)

QVQLVESGGGLVQPGGSLRLSCAASGYTTSSVCMAWFRQAPGNGLEGVAHITRDGRTMYADSVRGRFTIS
RDNSKNTLYLQMNSLRAEDTAVYYCAARVCEWRSTVQAPRSEAYQLWGQGTLVTVSS

SEQ ID NO: 7 (huiBT11v7)

QVQLVESGGGLVQPGGSLRLSCAASGYTTSSVCMAWFRQAPGNEREGVAHITRDGRTMYADSVRGRFTIS
RDNSKNTLYLQMNSLRAEDTAVYYCAARVCEWRSTVQAPRSEAYQLWGQGTLVTVSS

SEQ ID NO: 8 (CDR1 Kabat)
SVCMA

SEQ ID NO: 9 (CDR2 Kabat)
HITRDGRTMYADSVRG

SEQ ID NO: 10 (CDR3 Kabat)
RVCEWRSTVQAPRSEAYQL

SEQ ID NO: 11 (CDR1 AbM)
GYTTSSVCMA

SEQ ID NO: 12 (CDR2 AbM)
HITRDGRTM

SEQ ID NO: 13 (CDR3 AbM)
RVCEWRSTVQAPRSEAYQL

SEQ ID NO: 14 (CDR1 Chothia)
GYTTSSV

SEQ ID NO: 15 (CDR2 Chothia)
TRDGR

SEQ ID NO: 16 (CDR3 Chothia)
RVCEWRSTVQAPRSEAYQL

SEQ ID NO: 17 (CDR1 IMGT)
GYTTSSVC

SEQ ID NO: 18 (CDR2 IMGT)

ITRDGRT

SEQ ID NO: 19 (CDR3 IMGT)

AARVCEWRSTVQAPRSEAYQL

SEQ ID NO: 20 (IgG1 Fc C220S)

EPKSSDKTHTCPPCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLTVDKSRWQQGNVFSCSV
MHEALHNHYTQKSLSLSPGK

SEQ ID NO: 21 (hRS7 VH)

QVQLQQSGSELKKPGASVKVSCKASGYTFTNYGMNWVKQAPGQGLKWMGWINTYTGEPTYTDDFKGR
FAFSLDTSVSTAYLQISSLKADDTAVYFCARGGFGSSYWYFDVWGQGSLVTVSS

SEQ ID NO: 22 (hRS7 VH CDR1 Kabat)

NYGMN

SEQ ID NO: 23 (hRS7 VH CDR2 Kabat)

WINTYTGEPTYTDDFKG

SEQ ID NO: 24 (hRS7 VH CDR3 Kabat)

GGFGSSYWYFDV

SEQ ID NO: 25 (hRS7 VH CDR1 AbM)

GYTFTNYGMN

SEQ ID NO: 26 (hRS7 VH CDR2 AbM)

WINTYTGEPT

SEQ ID NO: 27 (hRS7 VH CDR3 AbM)

GGFGSSYWYFDV

SEQ ID NO: 28 (hRS7 VH CDR1 Chothia)

GYTFTNY

SEQ ID NO: 29 (hRS7 VH CDR2 Chothia)

NTYTGE

SEQ ID NO: 30 (hRS7 VH CDR3 Chothia)

GGFGSSYWYFDV

SEQ ID NO: 31 (hRS7 VH CDR1 IMGT)

GYTFTNYG

SEQ ID NO: 32 (hRS7 VH CDR2 IMGT)

INTYTGEP

SEQ ID NO: 33 (hRS7 VH CDR3 IMGT)

ARGGFGSSYWYFDV

SEQ ID NO: 34 (hRS7 VL)

DIQLTQSPSSLSASVGDRVSITCKASQDVSIAVAWYQQKPGKAPKLLIYSASYRYTGVPDRFSGSGSGTDFT
LTISSLQPEDFAVYYCQQHYITPLTFGAGTKVEIK

SEQ ID NO: 35 (hRS7 VL CDR1 Kabat)

KASQDVSIAVA

SEQ ID NO: 36 (hRS7 VL CDR2 Kabat)

SASYRYT

SEQ ID NO: 37 (hRS7 VL CDR3 Kabat)

QQHYITPLT

SEQ ID NO: 38 (hRS7 VL CDR1 AbM)

KASQDVSIAVA

SEQ ID NO: 39 (hRS7 VL CDR2 AbM)

SASYRYT

SEQ ID NO: 40 (hRS7 VL CDR3 AbM)

QQHYITPLT

SEQ ID NO: 41 (hRS7 VL CDR1 Chothia)

KASQDVSIAVA

SEQ ID NO: 42 (hRS7 VL CDR2 Chothia)

SASYRYT

SEQ ID NO: 43 (hRS7 VL CDR3 Chothia)

QQHYITPLT

SEQ ID NO: 44 (hRS7 VL CDR1 IMGT)

SAS

SEQ ID NO: 45 (hRS7 VL CDR2 IMGT)

QDVSIA

SEQ ID NO: 46 (hRS7 VL CDR3 IMGT)

QQHYITPLT

SEQ ID NO: 47 (IgG1 constant region)








SEQ ID NO: 48 (Igκ constant region)




SEQ ID NO: 49 (hRS7 HC)

QVQLQQSGSELKKPGASVKVSCKASGYTFTNYGMNWVKQAPGQGLKWMGWINTYTGEPTYTDDFKGR
FAFSLDTSVSTAYLQISSLKADDTAVYFCARGGFGSSYWYFDVWGQGSLVTVSSASTKGPSVFPLAPSSKST
SGGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSDGSFFLYSKLTVDKSRWQ
QGNVFSCSVMHEALHNHYTQKSLSLSPGK

SEQ ID NO: 50 (hRS7 LC)

DIQLTQSPSSLSASVGDRVSITCKASQDVSIAVAWYQQKPGKAPKLLIYSASYRYTGVPDRFSGSGSGTDFT
LTISSLQPEDFAVYYCQQHYITPLTFGAGTKVEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

SEQ ID NO: 51 (antibody A HC)

QVQLVESGGGLVQPGGSLRLSCAASGYTTSSVCMAWFRQAPGNEREGVAHITRDGRTMYADSVRGRFTIS
RDNSKNTLYLQMNSLRAEDTAVYYCAARVCEWRSTVQAPRSEAYQLWGQGTLVTVSSGAPGGGGGSQV
QLQQSGSELKKPGASVKVSCKASGYTFTNYGMNWVKQAPGQGLKWMGWINTYTGEPTYTDDFKGRFA
FSLDTSVSTAYLQISSLKADDTAVYFCARGGFGSSYWYFDVWGQGSLVTVSSASTKGPSVFPLAPSSKSTSG
GTAALGCLVKDYFPEPVTVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSKLTVDKSRWQQG
NVFSCSVMHEALHNHYTQKSLSLSPGK

SEQ ID NO: 52 (Mutant GalT1)

GSNSAAAIGQSSGELRTGGARPPPPLGASSQPRPGGDSSPVVDSGPGPASNLTSVPVPHTTALSLPACPEESP
LLVGPMLIEFNMPVDLELVAKQNPNVKMGGRYAPRDCVSPHKVAIIIPFRNRQEHLKYWLYYLHPVLQRQ
QLDYGIYVINQAGDTIFNRAKLLNVGFQEALKDYDYTCFVFSDVDLIPMNDHNAYRCFSQPRHISVAMDK

FGFSLPYVQLFGGVSALSKQQFLTINGFPNNYWGWGGEDDDIFNRLVFRGMSISRPNAVVGRCRMIRHSR
DKKNEPN PQRFDRIAHTKETMLSDGLNSLTYQVLDVQRYPLYTQITVDIGTPS

SEQ ID NO: 53 (human GalT1 Y285L)

MRLREPLLSGSAAMPGASLQRACRLLVAVCALHLGVTLVYYLAGRDLSRLPQLVGVSTPLQGGSNSAAAI
GQSSGELRTGGARPPPPLGASSQPRPGGDSSPVVDSGPGPASNLTSVPVPHTTALSLPACPEESPLLVGPMLI
EFNMPVDLELVAKQNPNVKMGGRYAPRDCVSPHKVAIIIPFRNRQEHLKYWLYYLHPVLQRQQLDYGIYV
INQAGDTIFNRAKLLNVGFQEALKDYDYTCFVFSDVDLIPMNDHNAYRCFSQPRHISVAMDKFGFSLPYV
QLFGGVSALSKQQFLTINGFPNNYWGWGGEDDDIFNRLVFRGMSISRPNAVVGRCRMIRHSRDKKNEPNP
QRFDRIAHTKETMLSDGLNSLTYQVLDVQRYPLYTQITVDIGTPS

SEQ ID NO: 54 (bovine GalT1 Y289L)

MKFREPLLGGSAAMPGASLQRACRLLVAVCALHLGVTLVYYLAGRDLRRLPQLVGVHPPLQGSSHGAAAI
GQPSGELRLRGVAPPPPLQNSSKPRSRAPSNLDAYSHPGPGPGPGSNLTSAPVPSTTTRSLTACPEESPLLVGP
MLIEFNIPVDLKLVEQQNPKVKLGGRYTPMDCISPHKVAIIIPFRNRQEHLKYWLYYLHPILQRQQLDYGIY
VINQAGESMFNRAKLLNVGFKEALKDYDYNCFVFSDVDLIPMNDHNTYRCFSQPRHISVAMDKFGFSLPY
VQLFGGVSALSKQQFLSINGFPNNYWGWGGEDDDIYNRLAFRGMSVSRPNAVIGKCRMIRHSRDKKNEP
NPQRFDRIAHTKETMLSDGLNSLTYMVLEVQRYPLYTKITVDIGTPS

SEQ ID NO: 55 (AV203HC)

MGWSLILLFLVAVATRVLSQVQLVESGGGLVKPGGSLRLSCAASGFTFSDYAMSWIRQAPGKGLEWVSTIS
DGGTYTYYPDSVKGRFTISRDNAKNSLYLQMNSLRAEDTAVYYCAREWGDYDGFDYWGQGTLVTVSSA
STKGPSVFPLAPSSKSTSGGTAALGCLVKDYFPEPVTVSWNSGALTSGVHTFPAVLQSSGLYSLSSVVTVPS
SSLGTQTYICNVNHKPSNTKVDKKVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEYKCKVSNKALP
APIEKTISKAKGQPREPQVYTLPPSREEMTKNQVSLTCLVKGFYPSDIAVEWESNGQPENNYKTTPPVLDSD
GSFFLYSKLTVDKSRWQQGNVFSCSVMHEALHNHYTQKSLSLSPGK

SEQ ID NO: 56 (AV203LC)

MDFQVQIISFLLISASVIMSRGDIQMTQSPSSLSASVGDRVTITCRASQEISGYLSWYQQKPGKAPKRLIYAA
STLDSGVPSRFSGSGSGTEFTLTISSLQPEDFATYYCLQYDSYPYTFGQGTKLEIKRTVAAPSVFIFPPSDEQL
KSGTASVVCLLNNFYPREAKVQWKVDNALQSGNSQESVTEQDSKDSTYSLSSTLTLSKADYEKHKVYAC
EVTHQGLSSPVTKSFNRGEC

SEQ ID NO: 57 (common upstream primer for sequence encoding single-domain antibody VHH)

cccACCGGTCAGGTGCAGCTGCAGGAGTC

SEQ ID NO: 58 (common downstream primer for sequence encoding single-domain antibody VHH)
cccGGATCCTGAGGAGACGGTGACCTGG

## Claims

1. A human epidermal growth factor receptor 3 (HER3)-binding protein, comprising at least one immunoglobulin single variable domain, wherein the immunoglobulin single variable domain comprises a CDR1, a CDR2, and a CDR3 from the VHH set forth in SEQ ID NO: 1.

2. The HER3-binding protein according to claim 1, wherein the CDR1, the CDR2, and the CDR3 are defined according to the following definition system: Kabat, AbM, Chothia, or IMGT.

3. The HER3-binding protein according to claim 1 or 2, wherein the immunoglobulin single variable domain is of the family Camelidae, humanized, or chimeric.

4. The HER3-binding protein according to any one of claims 1-3, wherein the CDR1, the CDR2, and the CDR3 from the VHH set forth in SEQ ID NO: 1 are selected from any one of the following groups: SEQ ID NOs: 8-10, SEQ ID NOs: 11-13, SEQ ID NOs: 14-16, and SEQ ID NOs: 17-19.

5. The HER3-binding protein according to any one of claims 1-4, wherein the at least one immunoglobulin single variable domain comprises one or more of the amino acid sequences set forth in SEQ ID NOs: 1-7.

6. The HER3-binding protein according to claim 5, wherein the at least one immunoglobulin single variable domain comprises the amino acid sequence set forth in one of SEQ ID NOs: 1-7.

7. The HER3-binding protein according to any one of claims 1-6, wherein the HER3-binding protein comprises one said immunoglobulin single variable domain.

8. The HER3-binding protein according to any one of claims 1-6, wherein the HER3-binding protein comprises 2, 3, 4, 5, or more said immunoglobulin single variable domains; optionally, the 2, 3, 4, 5, or more immunoglobulin single variable domains have or do not have identical sequences.

9. The HER3-binding protein according to any one of claims 1-8, further comprising an immunoglobulin Fc region, preferably a human immunoglobulin Fc region, and more preferably a human IgG1, IgG2, IgG3, or IgG4 Fc region.

10. The HER3-binding protein according to claim 9, wherein the amino acid sequence of the immunoglobulin Fc region is set forth in SEQ ID NO: 20.

11. The HER3-binding protein according to claim 9 or 10, wherein the immunoglobulin Fc region is linked directly or linked indirectly by a linker to the at least one immunoglobulin single variable domain.

12. A multispecific antigen-binding protein, comprising the HER3-binding protein according to claims 1-11 or an antigen-binding fragment thereof, which provides a first binding specificity.

13. The multispecific antigen-binding protein according to claim 12, further comprising a second binding specificity provided by an antibody or an antigen-binding fragment thereof that binds to trophoblast cell surface antigen 2 (TROP2), and optionally more binding specificities.

14. A multispecific antigen-binding protein, comprising an antibody or an antigen-binding fragment thereof that binds to trophoblast cell surface antigen 2 (TROP2), and at least one immunoglobulin single variable domain that binds to human epidermal growth factor receptor 3 (HER3), wherein:
preferably,
the antibody or the antigen-binding fragment thereof that binds to TROP2 comprises a heavy chain variable region and a light chain variable region, wherein the heavy chain variable region comprises a VH CDR1, a VH CDR2, and a VH CDR3 selected from any one of the following groups: SEQ ID NOs: 22-24, SEQ ID NOs: 25-27, SEQ ID NOs: 28-30, and SEQ ID NOs: 31-33, and
the light chain variable region comprises a VL CDR1, a VL CDR2, and a VL CDR3 selected from any one of the following groups: SEQ ID NOs: 35-37, SEQ ID NOs: 38-40, SEQ ID NOs: 41-43, and SEQ ID NOs: 44-46;
and/or
preferably, the immunoglobulin single variable domain that binds to HER3 comprises a VHH CDR1, a VHH CDR2, and a VHH CDR3 selected from any one of the following groups: SEQ ID NOs: 8-10, SEQ ID NOs: 11-13, SEQ ID NOs: 14-16, and SEQ ID NOs: 17-19.

15. The multispecific antigen-binding protein according to claim 14, wherein the heavy chain variable region of the antibody or the antigen-binding fragment thereof that binds to TROP2 comprises the amino acid sequence set forth in SEQ ID NO: 21.

16. The multispecific antigen-binding protein according to claim 14, wherein the light chain variable region of the antibody or the antigen-binding fragment thereof that binds to TROP2 comprises the amino acid sequence set forth in SEQ ID NO: 34.

17. The multispecific antigen-binding protein according to any one of claims 14-16, wherein the antibody or the antigen-binding fragment thereof that binds to TROP2 comprises a heavy chain that further comprises a human IgG1 constant region or a variant thereof; for example, the heavy chain comprises the amino acid sequence set forth in SEQ ID NO: 47.

18. The multispecific antigen-binding protein according to any one of claims 14-17, wherein the antibody or the antigen-binding fragment thereof that binds to TROP2 comprises a light chain that further comprises a human Igκ constant region or a variant thereof; for example, the light chain comprises the amino acid sequence set forth in SEQ ID NO: 48.

19. The multispecific antigen-binding protein according to any one of claims 14-18, wherein the antibody that binds to TROP2 comprises a heavy chain that comprises the amino acid sequence set forth in SEQ ID NO: 49.

20. The multispecific antigen-binding protein according to any one of claims 14-19, wherein the antibody that binds to TROP2 comprises a light chain that comprises the amino acid sequence set forth in SEQ ID NO: 50.

21. The multispecific antigen-binding protein according to any one of claims 14-20, wherein the at least one immunoglobulin single variable domain that binds to HER3 comprises the amino acid sequence set forth in one of SEQ ID NOs: 1-7.

22. The multispecific antigen-binding protein according to claim 21, comprising one immunoglobulin single variable domain.

23. The multispecific antigen-binding protein according to any one of claims 14-22, wherein the at least one immunoglobulin single variable domain that binds to HER3 is linked directly or linked indirectly by a linker to the antibody or the antigen-binding fragment that binds to TROP2.

24. The multispecific antigen-binding protein according to any one of claims 14-23, wherein the C-terminus of the at least one immunoglobulin single variable domain that binds to HER3 is linked to the N-terminus of a heavy chain of the antibody or the antigen-binding fragment that binds to TROP2.

25. The multispecific antigen-binding protein according to any one of claims 14-23, wherein the N-terminus of the at least one immunoglobulin single variable domain that binds to HER3 is linked to the C-terminus of a heavy chain of the antibody or the antigen-binding fragment that binds to TROP2.

26. The multispecific antigen-binding protein according to any one of claims 14-23, wherein the C-terminus of the at least one immunoglobulin single variable domain that binds to HER3 is linked to the N-terminus of a light chain of the antibody or the antigen-binding fragment that binds to TROP2.

27. The multispecific antigen-binding protein according to any one of claims 14-23, wherein the N-terminus of the at least one immunoglobulin single variable domain that binds to HER3 is linked to the C-terminus of a light chain of the antibody or the antigen-binding fragment that binds to TROP2.

28. The multispecific antigen-binding protein according to any one of claims 14-27, comprising a first polypeptide set forth in SEQ ID NO: 51 and a second polypeptide set forth in SEQ ID NO: 50.

29. A protein-drug conjugate, having the structure of formula I:
P-(L1-sp1-L2-sp2-D)n (I),
wherein protein P comprises the multispecific antigen-binding protein according to any one of claims 12-28, D is a substance having biological activity, L1 is a linker for linking to P, sp1 is a first spacing unit, L2 is a cleavable linker, sp2 is a second spacing unit and is linked to D, and n = 1-20.

30. The protein-drug conjugate according to claim 29, wherein L1 is selected from: 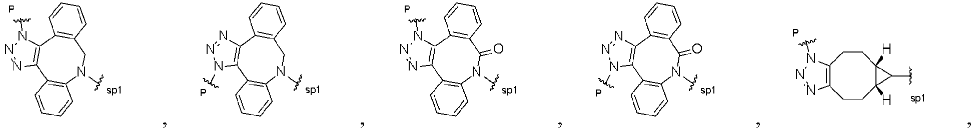 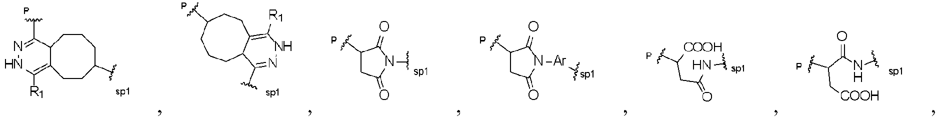 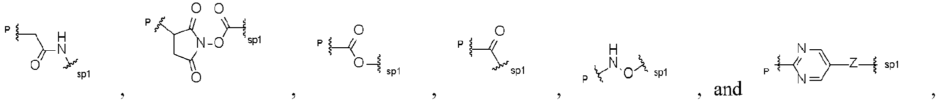 wherein Ar represents C₆₋₁₀ arylene optionally substituted with halogen or C₁₋₆ alkyl; R₁ is selected from hydrogen, halogen, and C₁₋₆ alkyl; Z is selected from a direct bond, C2-6 alkynylene, C₂₋₆ alkenylene, C₆₋₁₀ arylene, 5-10 membered heteroarylene, amido, sulfonamido, imino, and CF₂.

31. The protein-drug conjugate according to claim 29 or 30, wherein sp1 has the structure represented by formula II: 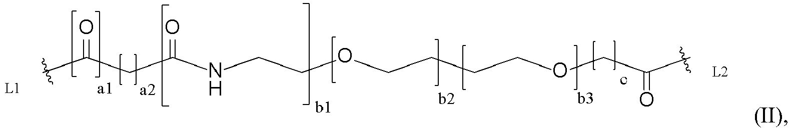 wherein a1 = 0 or 1, a2 = an integer from 0 to 6, b1 = 0 or 1, b2 = an integer from 0 to 16, b3 = an integer from 0 to 16, and c = an integer from 0 to 6, and at least one of b2 and b3 is 0.

32. The protein-drug conjugate according to claim 31, wherein:
(1) a1 = 0, a2 = 2, 3, 4, 5, or 6, b1 = 0, b2 = 0, b3 = 0, and c = 0;
(2) a1 = 0, a2 = 0, b1 = 0, b2 = 0, b3 = 0, and c = 2, 3, 4, 5, or 6;
(3) a1 = 1, a2 = 2, 3, 4, 5, or 6, b1 = 1, b2 = 2, 3, 4, 5, 6, 7, or 8, b3 = 0, and c = 0;
(4) a1 = 0, a2 = 2, 3, 4, 5, or 6, b1 = 1, b2 = 2, 3, 4, 5, 6, 7, or 8, b3 = 0, and c = 0; or
(5) a1 = 1, a2 = 0, b1 = 0, b2 = 0, b3 = 2, 3, 4, 5, 6, 7, or 8, and c = 2, 3, 4, 5, or 6.

33. The protein-drug conjugate according to any one of claims 29-32, wherein L2 is a dipeptide, tripeptide, or tetrapeptide amino acid residue.

34. The protein-drug conjugate according to claim 33, wherein L2 is selected from the following dipeptide amino acid residues: -Phe-Lys-, -Val-Ala-, -Val-Lys-, -Val-Cit-, -Ala-Lys-, -Phe-Cit-, -Leu-Cit-, -Ile-Cit-, -Phe-Arg-, -Trp-Cit-, -Gly-Gly-, -Ala-Ala-, -Gly-Val-, and -Gly-Glu-; the left side of the dipeptide amino acid residue is linked to sp1, and the right side thereof is linked to sp2.

35. The protein-drug conjugate according to claim 33, wherein L2 is selected from the following tripeptide amino acid residues: -Glu-Val-Ala-, -Glu-Val-Cit-, -αGlu-Val-Ala-, -αGlu-Val-Cit-, -Val-Lys-Gly, and -Val-Cit-Gly-; the left side of the tripeptide amino acid residue is linked to sp1, and the right side thereof is linked to sp2.

36. The protein-drug conjugate according to claim 33, wherein L2 is selected from the following tetrapeptide amino acid residues: -Gly-Gly-Phe-Gly- and -Gly-Phe-Gly-Gly-; the left side of the tetrapeptide amino acid residue is linked to sp1, and the right side thereof is linked to sp2.

37. The protein-drug conjugate according to any one of claims 29-36, wherein sp2 is absent, or sp2 is selected from: 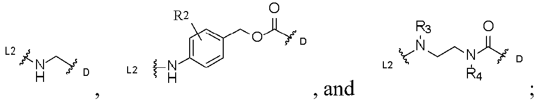
R₂ is independently selected from hydrogen, C₁₋₆ alkyl, hydroxy, amino, halogen, nitro, cyano, 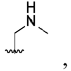 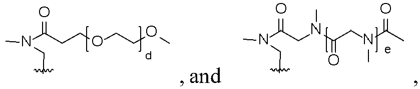 d is an integer from 1 to 20, and e is an integer from 1 to 20; R₃ and
R₄ are each independently selected from hydrogen and C₁₋₆ alkyl;
the alkyl may be optionally substituted with hydroxy, amino, halogen, nitro, and cyano.

38. The protein-drug conjugate according to any one of claims 29-37, wherein -L1-sp1-L2-sp2- is selected from the following structures: 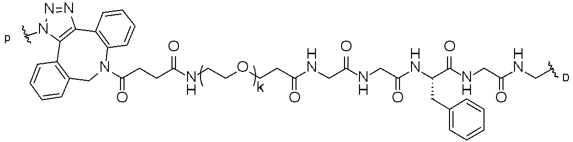 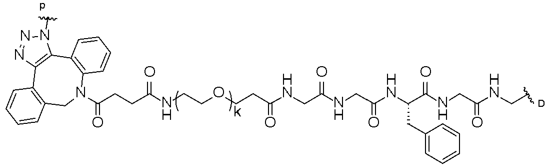 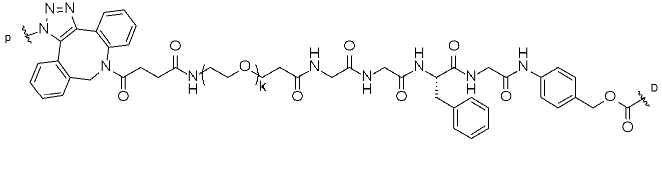 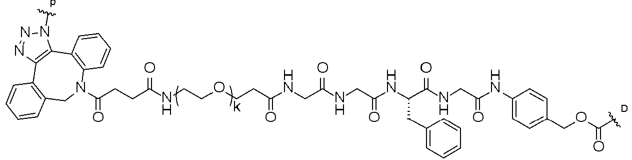 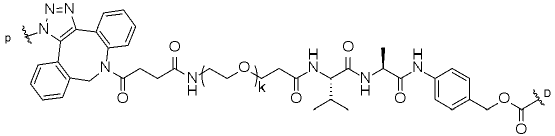 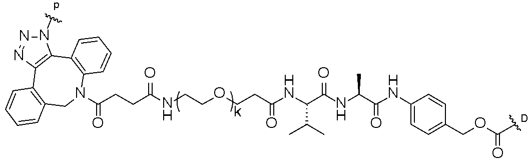 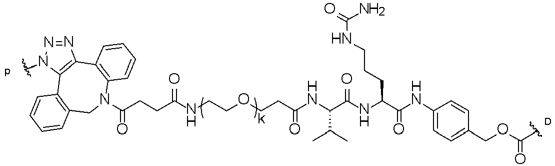 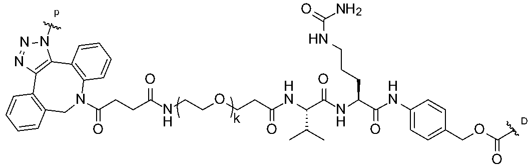 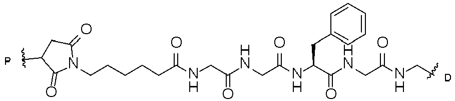 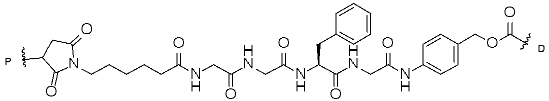 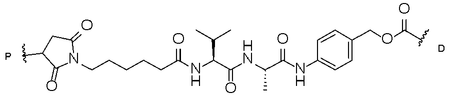 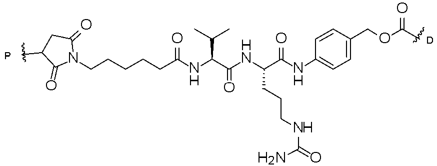 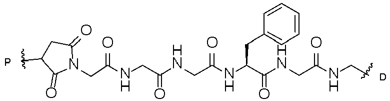 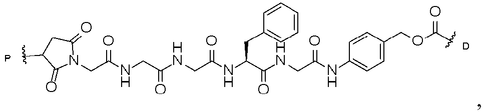 and 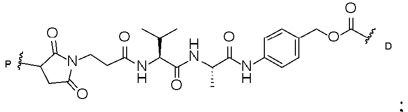 k is an integer from 1 to 20, e.g., 2, 3, 4, 5, 6, 7, 8, 9, or 10.

39. The protein-drug conjugate according to any one of claims 29-38, wherein D is selected from a cytotoxin, a protein kinase inhibitor, an immune agonist, a glucocorticoid, an oligonucleotide, a radioisotope, a polypeptide, and any combination thereof.

40. The protein-drug conjugate according to claim 39, wherein D is a cytotoxin selected from a DNA alkylating agent, a DNA deconstructing agent, a topoisomerase I inhibitor, a topoisomerase II inhibitor, a microtubule inhibitor, a ribosome inhibitor, and any combination thereof.

41. The protein-drug conjugate according to claim 40, wherein D is selected from an auristatin derivative, a maitansine derivative, an eribulin derivative, a tubulysin derivative, a pyrrolobenzodiazepine (PDB) derivative, a duocarmycin derivative, a calicheamicin derivative, PNU-159682 and a derivative thereof, a camptothecin derivative, an amatoxin derivative, and any combination thereof.

42. The protein-drug conjugate according to claim 40, wherein D has the structure represented by formula III: 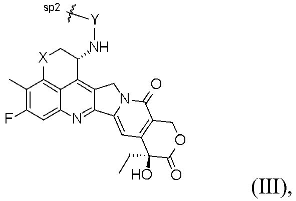 wherein X is selected from CH₂, NH, O, S, and SO₂;
Y is absent, or Y has the structure represented by 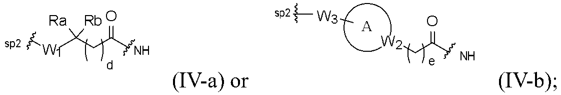
W₁ and W₃ are each independently selected from O, S, and NH, and W₂ is selected from CH and N;
Rₐ and R_{b} are each independently selected from hydrogen, deuterium, halogen, C₁₋₆ alkyl, C₁₋₆ alkoxy, C₃₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
ring A is selected from the following groups: 5-10 membered cycloalkylene, 5-10 membered heterocycloalkylene, 6-10 membered arylene, and 5-10 membered heteroarylene;
the alkyl, alkoxy, cycloalkyl, heterocycloalkyl, cycloalkylene, heterocycloalkylene, arylene, and heteroarylene are each independently, optionally, and further substituted with a group selected from deuterium, halogen, hydroxy, amino, cyano, carbonyl, and nitro;
d and e are each independently selected from integers from 0 to 5.

43. The protein-drug conjugate according to claim 42, wherein D has the structure represented by formula III-a: 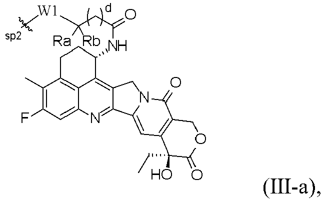 wherein W₁ is O or NH;
Rₐ and R_{b} are each independently selected from hydrogen, deuterium, halogen, C₁₋₆ alkyl, C₁₋₆ alkoxy, C₃₋₆ cycloalkyl, 3-6 membered heterocycloalkyl, hydroxy, amino, cyano, and nitro; or, Rₐ and R_{b}, together with the carbon atom to which they are attached, form 3-6 membered cycloalkyl, 3-6 membered heterocycloalkyl, or carbonyl; or, Rₐ is linked to the N atom of the amide moiety to form 3-6 membered heterocycloalkyl, and R_{b} is hydrogen;
the alkyl, alkoxy, cycloalkyl, and heterocycloalkyl are each independently, optionally, and further substituted with a group selected from deuterium, halogen, hydroxy, amino, cyano, and nitro;
d is an integer from 0 to 5.

44. The protein-drug conjugate according to claim 43, wherein W₁ is O.

45. The protein-drug conjugate according to claim 43 or 44, wherein d is 0, 1, or 2.

46. The protein-drug conjugate according to any one of claims 43-45, wherein Rₐ is selected from hydrogen, deuterium, halogen, C₁₋₆ alkyl, C₁₋₆ alkoxy, C₃₋₆ cycloalkyl, 3-6 membered heterocycloalkyl, hydroxy, and amino, and R_{b} is hydrogen.

47. The protein-drug conjugate according to any one of claims 43-45, wherein Rₐ and R_{b}, together with the carbon atom to which they are attached, form 3-6 membered cycloalkyl or 3-6 membered heterocycloalkyl.

48. The protein-drug conjugate according to claim 43, wherein D is selected from the following groups:

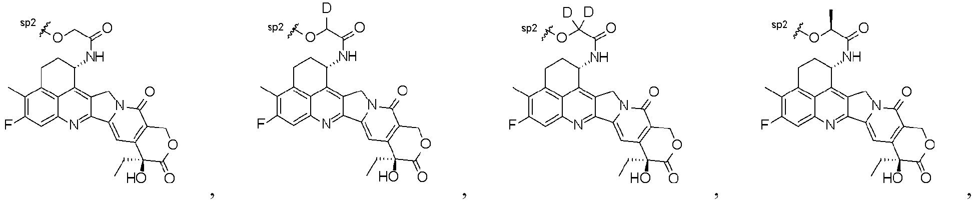

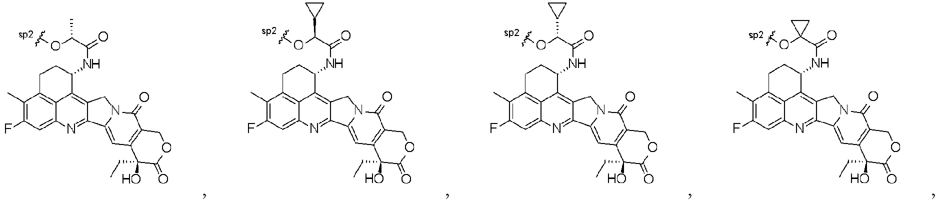

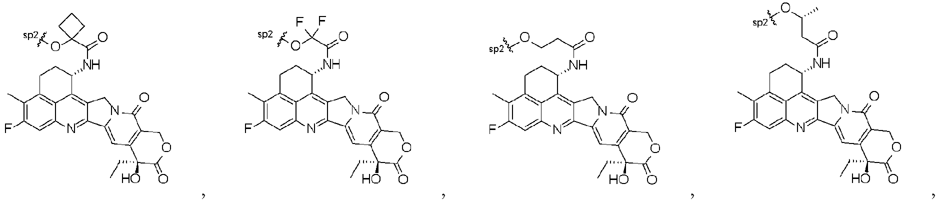

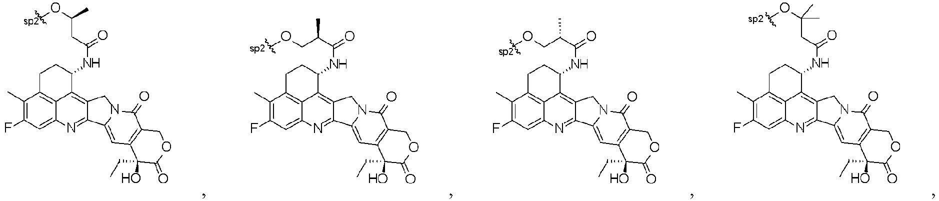

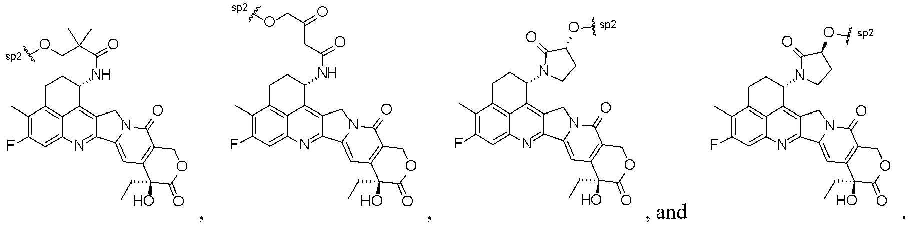

49. The protein-drug conjugate according to claim 42, wherein D has the structure represented by formula III-b: 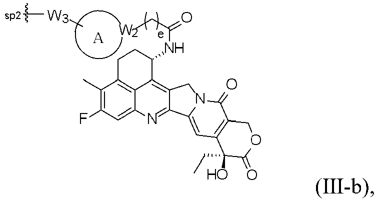
wherein W₃ is O or NH, and W₂ is CH and N;
ring A is selected from the following groups: 5-10 membered cycloalkylene, 5-10 membered heterocycloalkylene, 6-10 membered arylene, and 5-10 membered heteroarylene;
the cycloalkylene, heterocycloalkylene, arylene, and heteroarylene are each independently, optionally, and further substituted with a group selected from deuterium, halogen, hydroxy, amino, cyano, carbonyl, and nitro;
e is an integer from 0 to 5.

50. The protein-drug conjugate according to claim 49, wherein W₃ is O.

51. The protein-drug conjugate according to claim 49 or 50, wherein W₂ is CH.

52. The protein-drug conjugate according to any one of claims 49-51, wherein e is 0, 1, or 2.

53. The protein-drug conjugate according to any one of claims 49-52, wherein ring A is 5-10 membered cycloalkylene.

54. The protein-drug conjugate according to claim 49, wherein D is selected from the following groups:

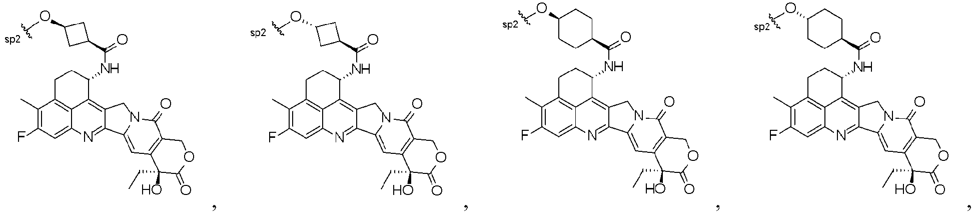

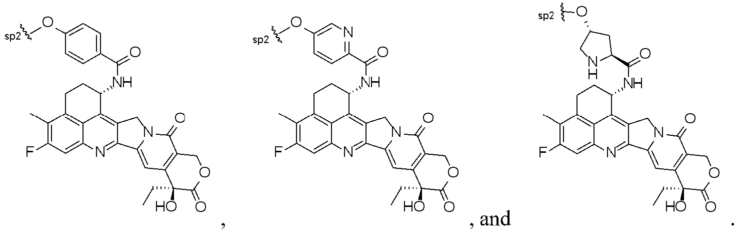

55. The protein-drug conjugate according to claim 41, wherein D is a camptothecin derivative and is preferably selected from the following structures:

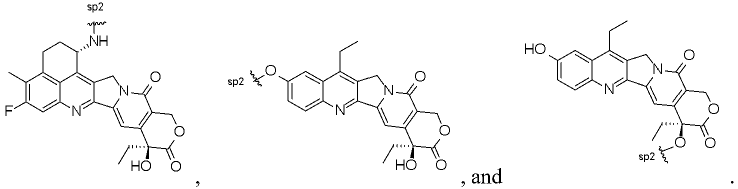

56. The protein-drug conjugate according to claim 29, wherein -L1-sp1-L2-sp2-D is selected from the following structures: 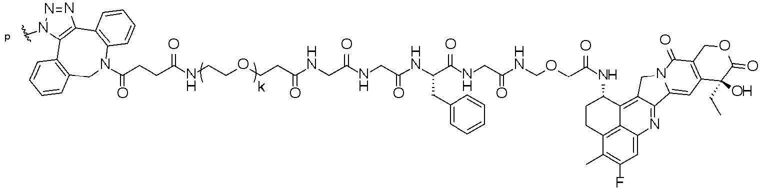 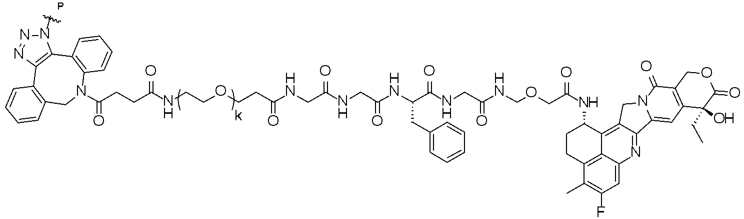 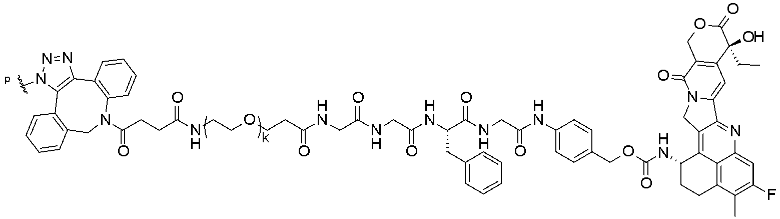 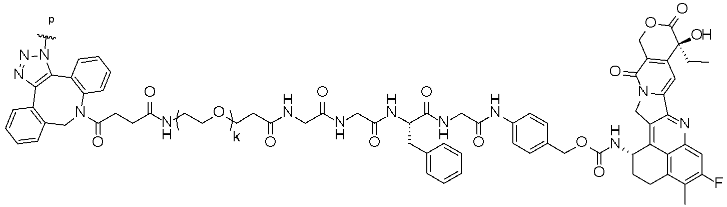 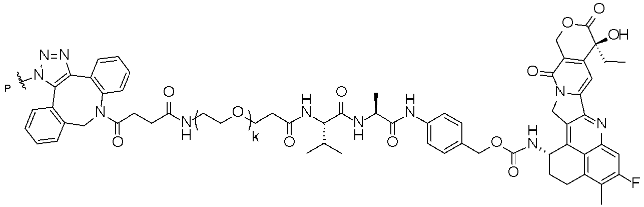 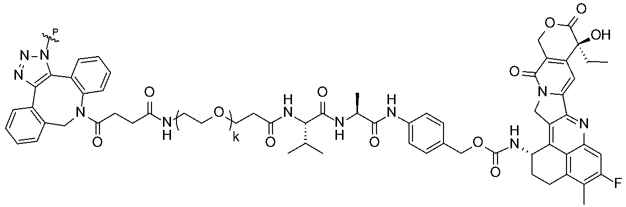 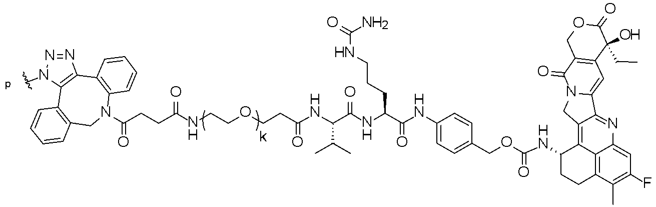 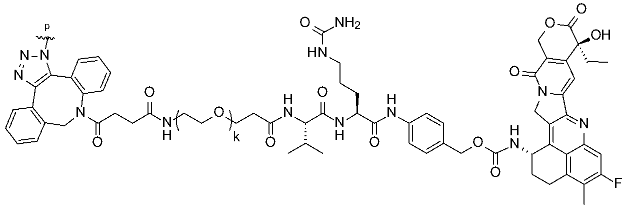 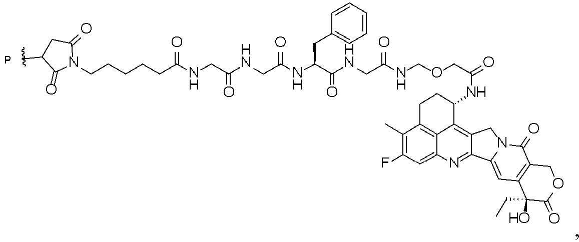 and 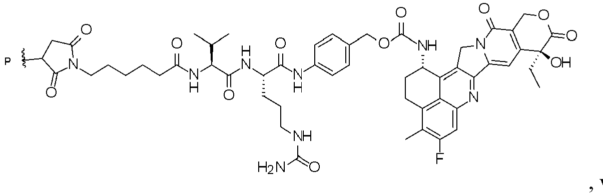 wherein k is an integer from 1 to 20.

57. The protein-drug conjugate according to any one of claims 29-56, wherein L1 is linked to the multispecific antigen-binding protein P by sulfhydryl, and the sulfhydryl is obtained by reducing a disulfide bond between heavy chains and/or a disulfide bond between a heavy chain and a light chain.

58. The protein-drug conjugate according to any one of claims 29-56, wherein L1 is linked to the multispecific antigen-binding protein P by an oligosaccharide.

59. The protein-drug conjugate according to claim 58, wherein the oligosaccharide has the structure represented by formula V-a or formula V-b: 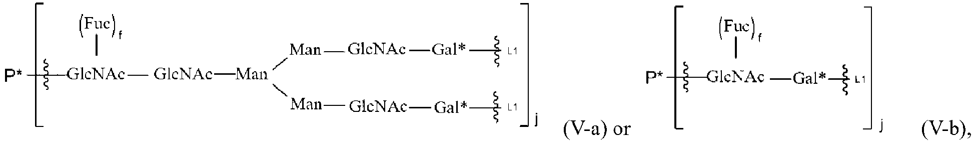
wherein P* binds to HER3 and TROP2 and comprises the antigen-binding protein according to any one of claims 12-28, GlcNAc is N-acetylglucosamine, Fuc is fucose, Man is mannose, f is 0 or 1, and j is 1 to 20;
Gal* is a modified galactose selected from the following structures: 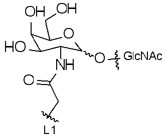 and 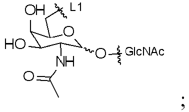
the oligosaccharide is linked to P* by the core GlcNAc.

60. The protein-drug conjugate according to claim 59, wherein the modified galactose is linked to GlcNAc by a β-1,4-glycosidic bond.

61. The protein-drug conjugate according to any one of claims 58-60, wherein the oligosaccharide is linked to the Fc fragment of P*, preferably to the CH2 domain of the Fc fragment, and more preferably to Asn297 (numbered according to the EU index of Kabat) of the Fc fragment.

62. The protein-drug conjugate according to any one of claims 29-61, having the structure represented by formula VI: 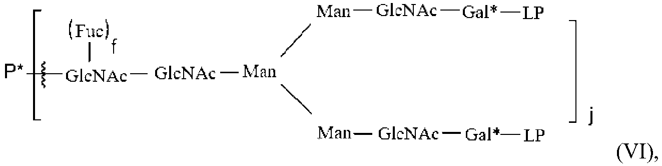
wherein P* binds to HER3 and TROP2; for example, P* comprises the antigen-binding protein according to any one of claims 12-28; GlcNAc is N-acetylglucosamine, Fuc is fucose, Man is mannose, f is 0 or 1, and j is 1 to 20;
Gal* is a modified galactose selected from the following structures: 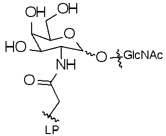 and 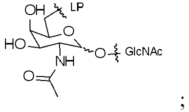
the oligosaccharide is linked to P* by the core GlcNAc;
LP is selected from the following structures: 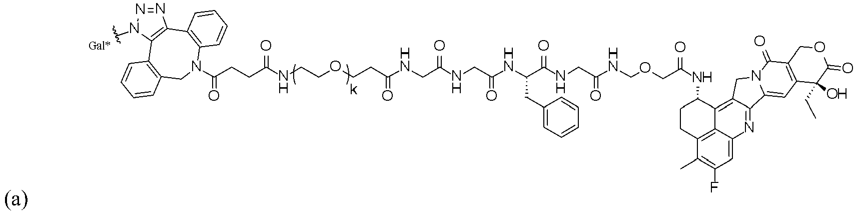 and/or 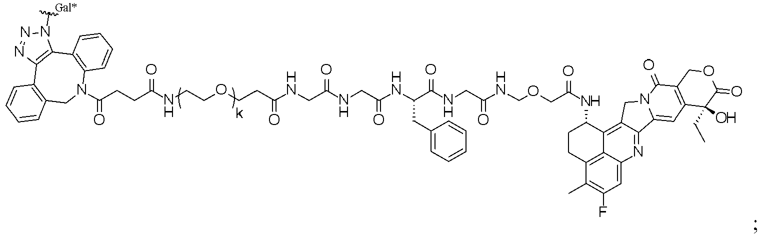 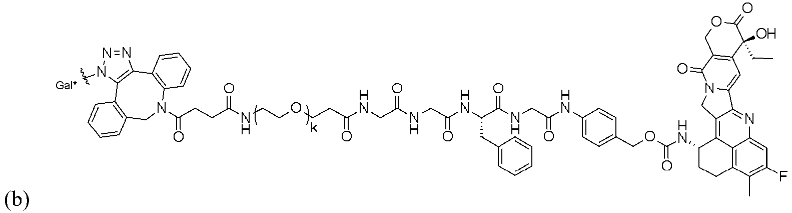 and/or 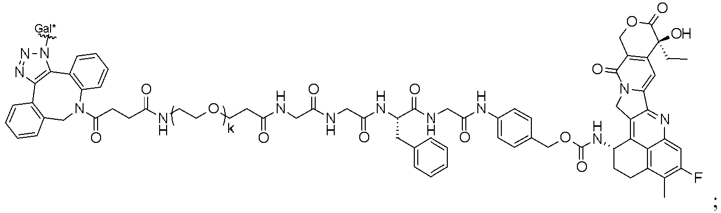 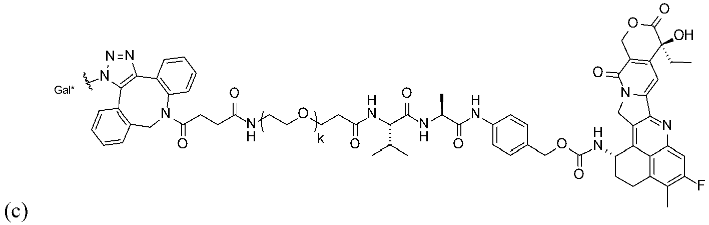 and/or 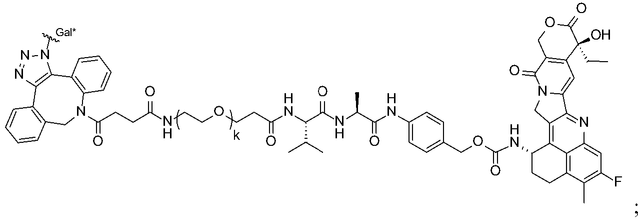 and 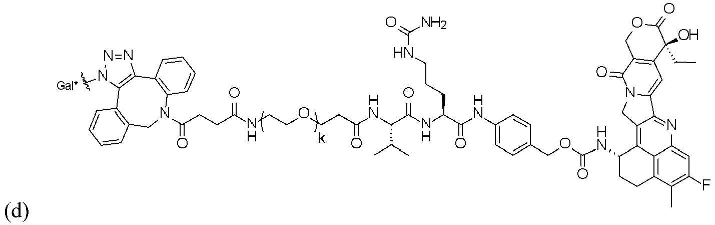 and/or 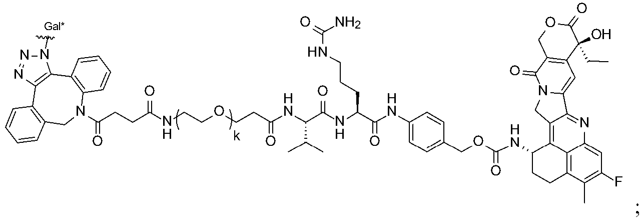
k is an integer from 1 to 20, e.g., 2, 3, 4, 5, 6, 7, 8, 9, or 10;
the core GlcNAc is directly linked to P*.

63. The protein-drug conjugate according to any one of claims 59-62, wherein the antigen-binding protein comprises a first polypeptide set forth in SEQ ID NO: 51 and a second polypeptide set forth in SEQ ID NO: 50.

64. The protein-drug conjugate according to claim 62 or 63, wherein the oligosaccharide is linked to the Fc fragment of P*, preferably to the CH2 domain of the Fc fragment, and more preferably to Asn297 (numbered according to the EU index of Kabat) of the Fc fragment.

65. A protein derivative, having the structure represented by formula VII: 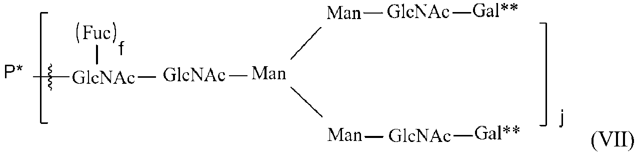
wherein P* binds to HER3 and TROP2 and comprises the antigen-binding protein according to any one of claims 12-28, GlcNAc is N-acetylglucosamine, Fuc is fucose, Man is mannose, f is 0 or 1, and j is 1 to 20;
Gal** is a modified galactose selected from the following structures: 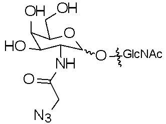 and 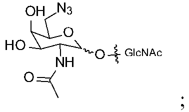
the oligosaccharide is linked to P* by the core GlcNAc.

66. The protein derivative according to claim 65, wherein the core GlcNAc is linked to the Fc fragment of P*, preferably to the CH2 domain of the Fc fragment, and more preferably to Asn297 (numbered according to the EU index of Kabat) of the Fc fragment.

67. An isolated polynucleotide, encoding the HER3-binding protein according to any one of claims 1-11 or an antigen-binding fragment thereof or the multispecific antigen-binding protein according to any one of claims 12-28.

68. A vector, comprising the polynucleotide according to claim 67, wherein preferably, the vector is an expression vector.

69. A host cell, comprising the polynucleotide according to claim 67 or the vector according to claim 68, wherein preferably, the host cell is prokaryotic or eukaryotic; more preferably, the host cell is selected from yeast cells, mammalian cells (for example, the host cell is a CHO cell, such as a CHO-K1 cell or an expiCHO cell, or the host cell is a 293 cell, such as an HEK293 cell), and other cells suitable for preparing an antibody or an antigen-binding fragment thereof.

70. A method for preparing a multispecific antigen-binding protein that binds to an HER3-binding protein or an antigen-binding fragment thereof or comprises the HER3-binding protein or the antigen-binding fragment thereof, comprising culturing a host cell comprising a nucleic acid encoding the HER3-binding protein according to any one of claims 1-11 or an antigen-binding fragment thereof or the multispecific antigen-binding protein according to any one of claims 12-28 under conditions suitable for the expression of the protein, wherein optionally, the method further comprises recovering the binding protein or the multispecific antigen-binding protein or an antigen-binding fragment thereof or a fusion protein thereof from the host cell.

71. Use of the HER3-binding protein according to any one of claims 1-11, the multispecific antigen-binding protein according to any one of claims 12-28, or the protein-drug conjugate according to any one of claims 29-64 in the preparation of a medicament for treating and/or preventing a tumor.

72. The use according to claim 71, wherein the tumor comprises a solid tumor and/or a non-solid tumor.
